# EUROPEAN PATENT SPECIFICATION

(11) **EP 3 289 089 B1**
(45) Date of publication and mention of the grant of the patent: **01.03.2023**
(21) Application number: 16787063.3
(22) Date of filing: 27.04.2016
(51) Int. Cl.: A01N 63/60, C12N 15/82, C12N 9/22

(54) **CONTROLLING FUNGAL PATHOGENS BY DISABLING THEIR SMALL RNA PATHWAYS USING RNAi-BASED STRATEGY**
BEKÄMPFUNG FUNGALER PATHOGENE DURCH DEAKTIVIERUNG VON KLEIN-RNA-WEGEN UNTER VERWENDUNG VON RNAI-BASIERTER STRATEGIE
RÉGULATION DE PATHOGÈNES FONGIQUES PAR DÉSACTIVATION DE LEURS VOIES DE PETITS ARN EN UTILISANT UNE STRATÉGIE À BASE D'ARNI

(30) Priority: 27.04.2015 US 201562153440 P; 24.07.2015 US 201514809063
(43) Date of publication of application: 07.03.2018
(73) Proprietor: The Regents of the University of California, Oakland, CA 94607 (US)
(72) Inventor: JIN, Hailing, Riverside, California 92521 (US)
(74) Representative: J A Kemp LLP
(86) International application number: PCT/US2016/029560
(87) International publication number: WO 2016/176324

(56) References cited:
- EP-A1- 2 698 379
- WO-A1-2012/155109
- WO-A1-2014/033723
- US-A1- 2011 119 788
- US-A1- 2014 283 211
- US-A1- 2015 089 688
- US-B1- 6 495 133
- Yun Chen ET AL: "Characterization of RNA silencing components in the plant pathogenic fungus Fusarium graminearum", Scientific reports, 27 July 2015 (2015-07-27), page 12500, XP055502585, England DOI: 10.1038/srep12500 Retrieved from the Internet: URL:https://www.ncbi.nlm.nih.gov/pmc/artic les/PMC4515635/pdf/srep12500.pdf [retrieved on 2018-08-27]
- Cornelia Stärkel: "Host Induced Gene Silencing", , 1 January 2011 (2011-01-01), XP055502637, Retrieved from the Internet: URL:https://d-nb.info/1020929731/34 [retrieved on 2018-08-28]
- DELERIS, A ET AL.: 'Hierarchical Action and Inhibition of Plant Dicer-Like Proteins in Antiviral Defense.' SCIENCE vol. 313, no. 5783, 01 June 2006, pages 68 - 71, XP055327573

## Description

### CROSS-REFERENCE TO RELATED APPLICATIONS

The present application claims priority to U.S. Provisional Application No. 62/153,440, filed April 27, 2015, and to U.S. Patent Application No. 14/809,063, filed July 24, 2015.

### STATEMENT AS TO RIGHTS TO INVENTIONS MADE UNDER FEDERALLY SPONSORED RESEARCH AND DEVELOPMENT

This invention was made with Government support under Grant No. MCB-0642843, IOS-1257576 awarded by the National Science Foundation, a NIH grant (R01 GM093008). The government has certain rights in this invention.

### BACKGROUND OF THE INVENTION

In plants, pathogen attacks invoke multiple layers of host immune responses. Many pathogens of plants and animals deliver effectors into host cells to suppress host immunity, and many plants have evolved resistance proteins to recognize effectors and trigger robust resistance.

*Botrytis cinerea* is a fungal pathogen that infects flowers and almost all vegetable and fruit crops and annually causes $10-100 billion losses worldwide. With its broad host range, *B. cinerea* is a useful model for studying the pathogenicity of aggressive fungal pathogens.

### BRIEF SUMMARY OF THE INVENTION

The invention provides a method of increasing pathogen resistance in a plant or a part of a plant, the method comprising:
contacting the plant or the part of the plant with a first double-stranded RNA, small RNAs, or small RNA duplexes that target a first fungal pathogen dicer-like (DCL) gene or promoter, and contacting the plants or part of the plant with a second double-stranded RNA, small RNAs, or small RNA duplexes that target a second fungal pathogen DCL gene or promoter, wherein
   (a) the first double-stranded RNA, small RNAs, or small RNA duplexes target *Botrytis* DCL1 and the second double-stranded RNA, small RNAs, or small RNA duplexes target *Botrytis* DCL2; wherein the first and second DCL gene or promoter comprise a sequence of SEQ ID NO:1, SEQ ID NO:3, SEQ ID NO:9, SEQ ID NO:10, SEQ ID NO:28, or SEQ ID NO:29, or a fragment thereof comprising at least 50 contiguous nucleotides, or
   (b) the first double-stranded RNA, small RNAs, or small RNA duplexes target *Verticillium* DCL1 and the second double-stranded RNA, small RNAs, or small RNA duplexes target *Verticillium* DCL2; wherein the first and second DCL gene or promoter comprise a sequence of SEQ ID NO:5, SEQ ID NO:7, SEQ ID NO: 11, SEQ ID NO: 12, SEQ ID NO:30, or SEQ ID NO:31 or a fragment thereof comprising at least 50 contiguous nucleotides,
wherein the plant is a species of the genera *Asparagus, Atropa, Avena, Brassica, Citrus, Citrullus, Capsicum, Cucumis, Cucurbita, Daucus, Fragaria, Glycine, Gossypium, Helianthus, Heterocallis, Hordeum, Hyoscyamus, Lactuca, Linum, Lolium, Lycopersicon, Malus, Manihot, Majorana, Medicago, Nicotiana, Oryza, Panieum, Pannesetum, Persea, Pisum, Pyrus, Prunus, Raphanus, Rosa, Secale, Senecio, Sinapis, Solanum, Sorghum, Trigonella, Triticum, Vitis, Vigna,* or *Zea,* and wherein the plant or the part of the plant has increased resistance to the fungal pathogen compared to a control plant or control plant part that has not been contacted with the double-stranded RNA, small RNAs, or small RNA duplexes.

The invention further provides a method of increasing pathogen resistance in a fruit, a vegetable, or a flower, the method comprising:
contacting the fruit, vegetable, or flower with a first double-stranded RNA, small RNAs, or small RNA duplexes that target a first fungal pathogen dicer-like (DCL) gene or promoter, and contacting the fruit, vegetable, or flower with a second double-stranded RNA, small RNAs, or small RNA duplexes that target a second fungal pathogen DCL gene or promoter, wherein
   (a) the first double-stranded RNA, small RNAs, or small RNA duplexes target *Botrytis* DCL1 and the second double-stranded RNA, small RNAs, or small RNA duplexes target *Botrytis* DCL2; wherein the first and second DCL gene or promoter comprise the sequence of SEQ ID NO:1, SEQ ID NO:3, SEQ ID NO:9, SEQ ID NO:10, SEQ ID NO:28, or SEQ ID NO:29, or a fragment thereof comprising at least 50 contiguous nucleotides; or
   (b) the first double-stranded RNA, small RNAs or small RNA duplexes target *Verticillium* DCL1 and the second double-stranded RNA, small RNAs, or small RNA duplexes target *Verticillium* DCL2; wherein the first and second DCL gene or promoter comprises the sequence of SEQ ID NO:5, SEQ ID NO:7, SEQ ID NO:11, SEQ ID NO:12, SEQ ID NO:30, or SEQ ID NO:31 or a fragment thereof comprising at least 50 contiguous nucleotides, and
wherein the fruit, vegetable, or flower has increased resistance to the fungal pathogen compared to a control fruit, vegetable, or flower that has not been contacted with the double-stranded RNA, small RNAs, or small RNA duplexes.

The invention further provides a method of making a plant having increased pathogen resistance to a first species and a second species of pathogens, wherein the first species of pathogen is a species of *Botrytis* and the second species of pathogen is a species of *Verticillium,* the method comprising:
introducing into the plant by transformation
   (1) a heterologous expression cassette comprising a promoter operably linked to a first polynucleotide that inhibits expression of a *Botrytis* DCL1 gene or promoter and a heterologous expression cassette comprising a promoter operably linked to a second polynucleotide that inhibits expression of *Botrytis* DCL2 gene or promoter; wherein the DCL1 and DCL2 gene or promoter comprises the sequence of SEQ ID NO:1, SEQ ID NO:3, SEQ ID NO:9, SEQ ID NO: 10, SEQ ID NO:28, or SEQ ID NO:29, or a fragment thereof comprising at least 50 contiguous nucleotides;
   (2) a heterologous expression cassette comprising a promoter operably linked to a first polynucleotide that inhibits expression of a *Verticillium* DCL1 gene or promoter and a heterologous expression cassette comprising a promoter operably linked to a second polynucleotide that inhibits expression of *Verticillium* DCL2 gene or promoter; wherein the DCL1 and DCL2 gene or promoter comprises the sequence of SEQ ID NO:5, SEQ ID NO:7, SEQ ID NO:11, SEQ ID NO:12, SEQ ID NO:30, or SEQ ID NO:31 or a fragment thereof comprising at least 50 contiguous nucleotides; and
selecting a plant comprising the expression cassettes.

In one aspect, the present disclosure provides for plants (or a plant cell, seed, flower, leaf, fruit, or other plant part from such plants or processed food or food ingredient from such plants) having increased resistance to a pathogen (e.g., a fungal or oomycete pathogen). In another aspect of the disclosure, methods of making a pathogen-resistant plant are provided. In some aspects of the disclosure, the method comprises:
contacting the plant or the part of the plant with a double-stranded RNA, a small RNA (sRNA), or a small RNA duplex that targets a fungal or oomycete dicer-like (DCL) gene or long terminal repeat (LTR) region, wherein the plant is a species of the genera *Asparagus, Atropa, Avena, Brassica, Citrus, Citrullus, Capsicum, Cucumis, Cucurbita, Daucus, Fragaria, Glycine, Gossypium, Helianthus, Heterocallis, Hordeum, Hyoscyamus, Lactuca, Linum, Lolium, Lycopersicon, Malus, Manihot, Majorana, Medicago, Nicotiana, Oryza, Panieum, Pannesetum, Persea, Pisum, Pyrus, Prunus, Raphanus, Rosa, Secale, Senecio, Sinapis, Solanum, Sorghum, Trigonella, Triticum, Vitis, Vigna,* or *Zea,* and wherein the plant or the part of the plant has increased resistance to a fungal or oomycete pathogen compared to a control plant or control plant part that has not been contacted with the double-stranded RNA, sRNA, or small RNA duplex.

In some aspects of the disclosure, the method comprises:
contacting a fruit, vegetable, or flower with a double-stranded RNA or a small RNA duplex that targets a fungal or oomycete dicer-like (DCL) gene or long terminal repeat (LTR) region, wherein the fruit, vegetable, or flower has increased resistance to a fungal or oomycete pathogen compared to a control fruit, vegetable, or flower that has not been contacted with the double-stranded RNA, sRNA, or small RNA duplex.

In some aspects of the disclosure, the pathogen is a fungal pathogen. In some embodiments, the pathogen is *Botrytis* or *Verticillium.*

In some aspects of the disclosure, the double-stranded RNA, sRNA, or small RNA duplex targets a fungal or oomycete DCL gene. In some aspects of the disclosure, the double-stranded RNA, sRNA, or small RNA duplex targets any of SEQ ID NO:1, SEQ ID NO:3, SEQ ID NO:5, SEQ ID NO:7, SEQ ID NO:9, SEQ ID NO: 10, SEQ ID NO:11, SEQ ID NO:12, SEQ ID NO:28, SEQ ID NO:29, SEQ ID NO:30, or SEQ ID NO:31 or a fragment thereof (e.g., at least 20, 30, 40, 50, 60, 70, 80, 90, 100, 150, 200, 250, 300, 350, 400, 450, 500 or more contiguous nucleotides thereof). In some aspects of the disclosure, the double-stranded RNA, sRNA, or small RNA duplex comprises an inverted repeat of a sequence that is identical or substantially identical (*e.g.,* at least 60%, 65%, 70%, 75%, 80%, 85%, 90%, 91%, 92%, 93%, 94%, 95%, 96%, 97%, 98%, or 99% identical) to any of SEQ ID NO:1, SEQ ID NO:3, SEQ ID NO:5, SEQ ID NO:7, SEQ ID NO:9, SEQ ID NO:10, SEQ ID NO:11, or SEQ ID NO:12, or a fragment thereof, or a complement thereof. In some aspects of the disclosure, the double-stranded RNA, sRNA, or small RNA duplex comprises a spacer in between the inverted repeat sequences.

In some aspects of the disclosure, the double-stranded RNA is siRNA. In some embodiments, the double-stranded RNA, sRNA, or small RNA duplex is sprayed or brushed onto the plant or the part of the plant (*e.g*., a leaf, fruit, vegetable, or flower).

In some aspects of the disclosure, the method further comprises contacting the plant or the part of the plant with a second double-stranded RNA or a second small RNA duplex or a second sRNA that targets a second fungal pathogen DCL gene. In some aspects of the disclosure, the method comprises contacting the plant or the part of the plant with one or more double-stranded RNAs or small RNA duplexes or sRNAs that target a DCL gene from a first species of fungal pathogen (e.g., for targeting DCL genes in *Botrytis,* e.g., a double-stranded RNA, sRNA, or small RNA duplex that targets DCL1 in *B. cinerea* and a double-stranded RNA, sRNA, or small RNA duplex that targets DCL2 in *B. cinerea*) and further comprises contacting the plant or the part of the plant with one or more double-stranded RNAs or small RNA duplexes or sRNAs that target a DCL gene from a second species of fungal pathogen (e.g., for targeting DCL genes in *Verticillium,* e.g., a double-stranded RNA, sRNA, or small RNA duplex that targets DCL1 in *V. dahilae* and a double-stranded RNA, sRNA, or small RNA duplex that targets DCL2 in *V. dahilae*)*.*

In another aspect of the disclosure, methods of increasing pathogen resistance to multiple pathogens (e.g., fungal or oomycete pathogens) are provided. In some aspects of the disclosure, the method comprises:
contacting the plant or the part of the plant with (1) a first double-stranded RNA, sRNA, or small RNA duplex that targets a dicer-like (DCL) gene or a long terminal repeat (LTR) region from a first species of fungal or oomycete pathogen, and (2) a second double-stranded RNA, sRNA, or small RNA duplex that targets a DCL gene or a LTR region from a second species of fungal or oomycete pathogen, wherein the plant or the part of the plant has increased resistance to the first species of pathogen and the second species of pathogen compared to a control plant or control plant part that has not been contacted with the first and second double-stranded RNAs, sRNAs, or small RNA duplexes.

In some aspects of the disclosure, the method comprises contacting the plant with two or more double-stranded RNAs, sRNAs, or small RNA duplexes for targeting two or more DCL genes or LTR regions from the first species of pathogen (e.g., for targeting DCL genes in *Botrytis,* e.g., DCL1 and DCL2 in *B. cinerea*) and two or more double-stranded RNAs, sRNAs, or small RNA duplexes for targeting two or more DCL genes or LTR regions from the second species of pathogen (e.g., for targeting DCL genes in *Verticillium,* e.g., DCL1 and DCL2 in *V. dahilae*)*.*

In another aspect of the disclosure, a method of making a plant having increased pathogen resistance to multiple pathogens comprises:
introducing into the plant (1) a first heterologous expression cassette comprising a first promoter operably linked to a first polynucleotide that inhibits expression of a dicer-like (DCL) gene or a long terminal repeat (LTR) region from a first species of fungal or oomycete pathogen and (2) a second heterologous expression cassette comprising a second promoter operably linked to a second polynucleotide that inhibits expression of a DCL gene or a LTR region from a second species of fungal or oomycete pathogen; and
selecting a plant comprising the first expression cassette and the second expression cassette.

In some aspects of the disclosure, the method comprises introducing into the plant two or more heterologous expression cassettes for targeting two or more DCL genes or LTR regions from the first species of pathogen (e.g., for targeting DCL genes in *Botrytis,* e.g., DCL1 and DCL2 in *B. cinerea*) and two or more heterologous expression cassettes for targeting two or more DCL genes or LTR regions from the second species of pathogen (e.g., for targeting DCL genes in *Verticillium,* e.g., DCL1 and DCL2 in *V. dahilae*)*.* In some aspects of the disclosure, the polynucleotide comprises an antisense nucleic acid or inhibitory RNA (RNAi) that targets the DCL gene or LTR region or a fragment thereof.

In yet another aspect of the disclosure, methods of cultivating a plurality of pathogen-resistant plants are provided.

### BRIEF DESCRIPTION OF THE DRAWINGS

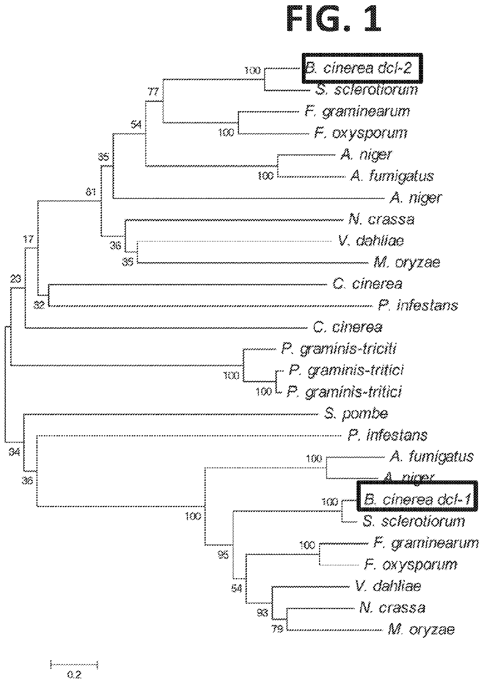

**FIG. 1****. The *B. cinerea* genome has two dicer-like (DCL) genes.** The phylogenetic tree of DCL proteins from different pathogenic fungal species, the DCL proteins from an oomycete pathogen *Phytophthora infestans* are also included. *Schizosaccharomyces pombe* and *Neurospora crassa* were used as references.

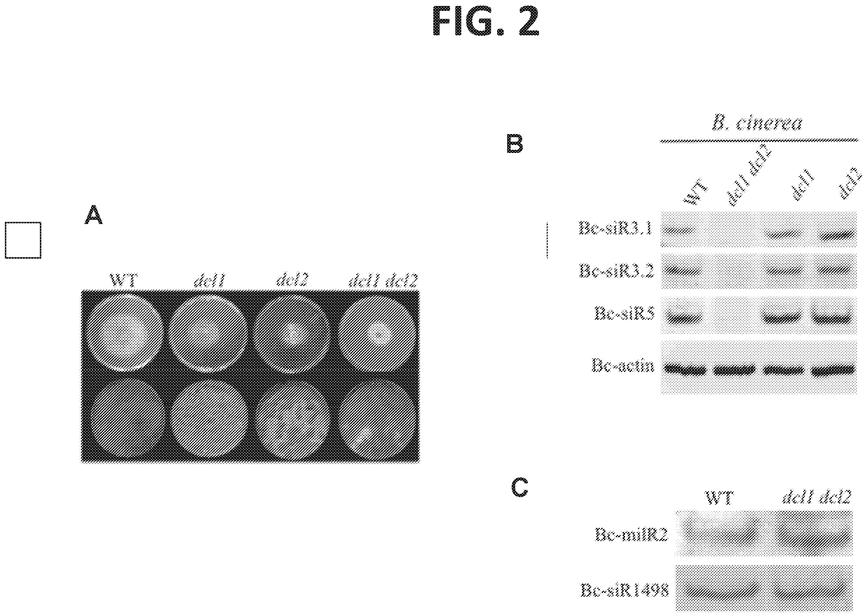

**FIG. 2A-C.** ***B. cinerea* (Bc)-sRNAs are dependent on both *B. cinerea* DCL proteins.** All of the *B. cinerea dcl1, dcl2,* and *dcl1 dcl2* mutant strains showed growth retardation and delayed development of conidiospores (A), but only the double mutant strain could not produce Bc-sRNA effectors (B). DCL-independent sRNAs were used as a control (C).

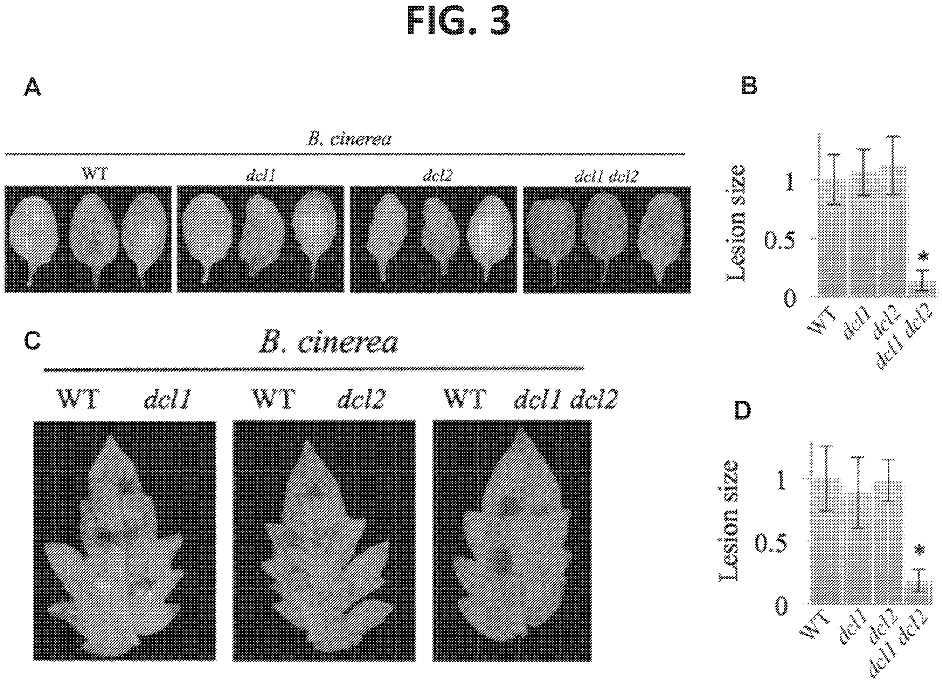

**FIG. 3A-D.** ***B. cinerea* DCLs are essential for its pathogenicity.** *B. cinerea dcl1 dcl2* double mutant, but not *dcl1* or *dcl2* single mutants, produced much weaker disease symptoms than did the wild type in both *Arabidopsis* (A-B) and *S. lycopersicum* (C-D).

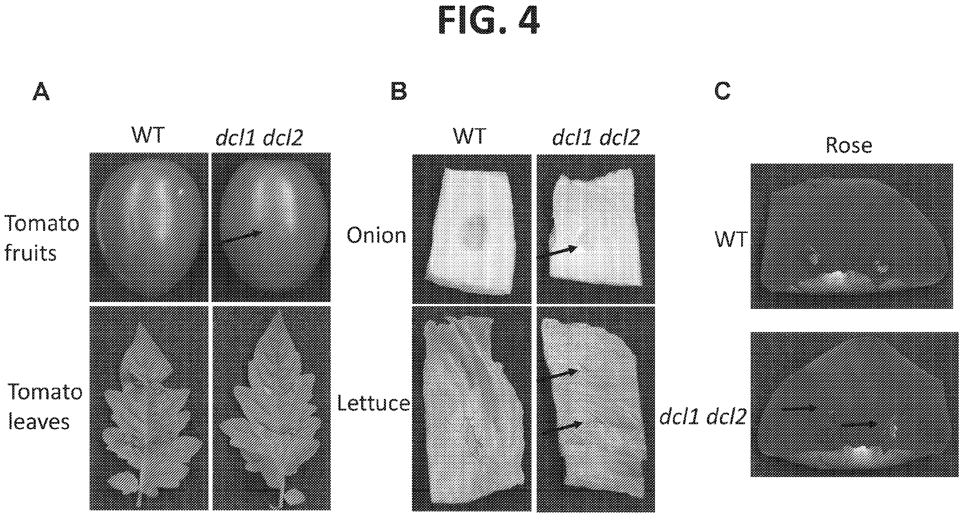

**FIG. 4A-C****. DCL-dependent small RNAs are important for fungal virulence.** *B*. *cinerea dell dcl2* double mutant is much less virulent on fruits, vegetables and flowers as compared to a wild-type *Botrytis* strain. Tomato fruits and leaves (A), onions and lettuces (B), and rose flowers (C) were infected by *B. cinerea* wild type strain B05 (WT) or *dell dcl2* double mutant strain. Photographs were taken after 3 days for tomato leaves and 4 days for tomato fruits, onion, lettuce and rose.

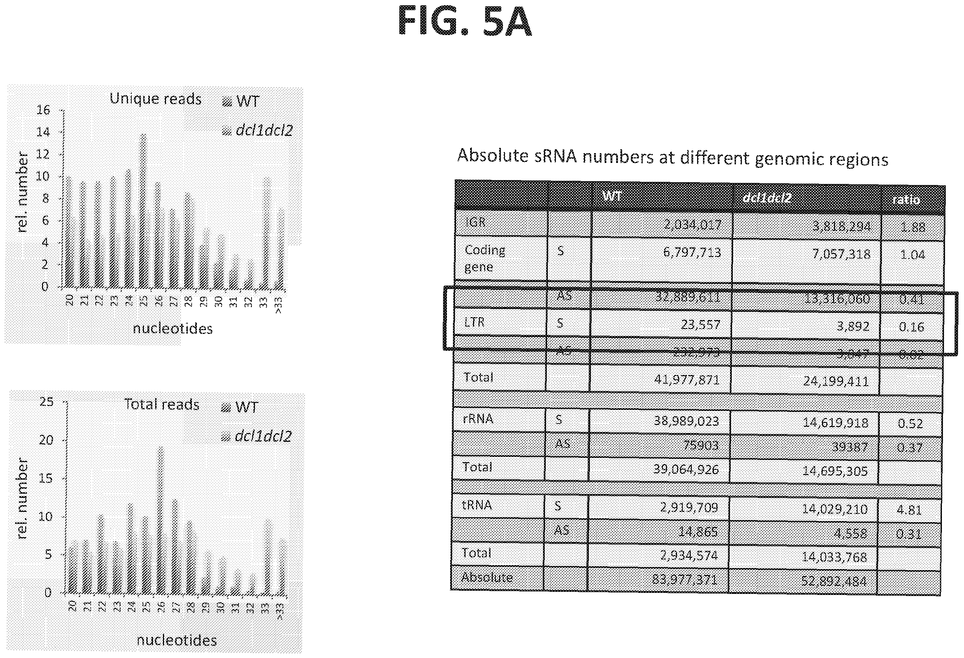

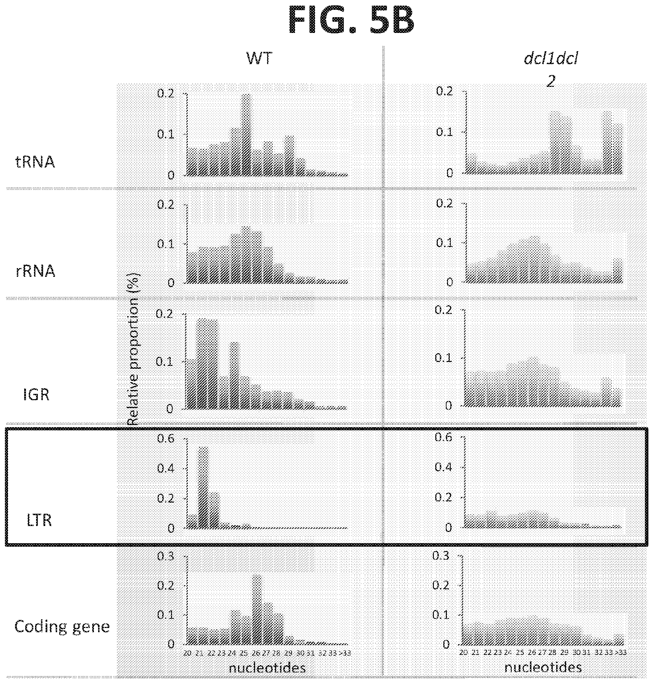

**FIG. 5A-B.** ***Botrytis* DCLs are responsible for generating long terminal repeat (LTR)-derived sRNAs**. Genome-wide comparative sRNA analysis on *dcl1 dcl2* and wild-type revealed that *Botrytis* DCLs are responsible for generating LTR-derived sRNAs, many of which are sRNA effectors.

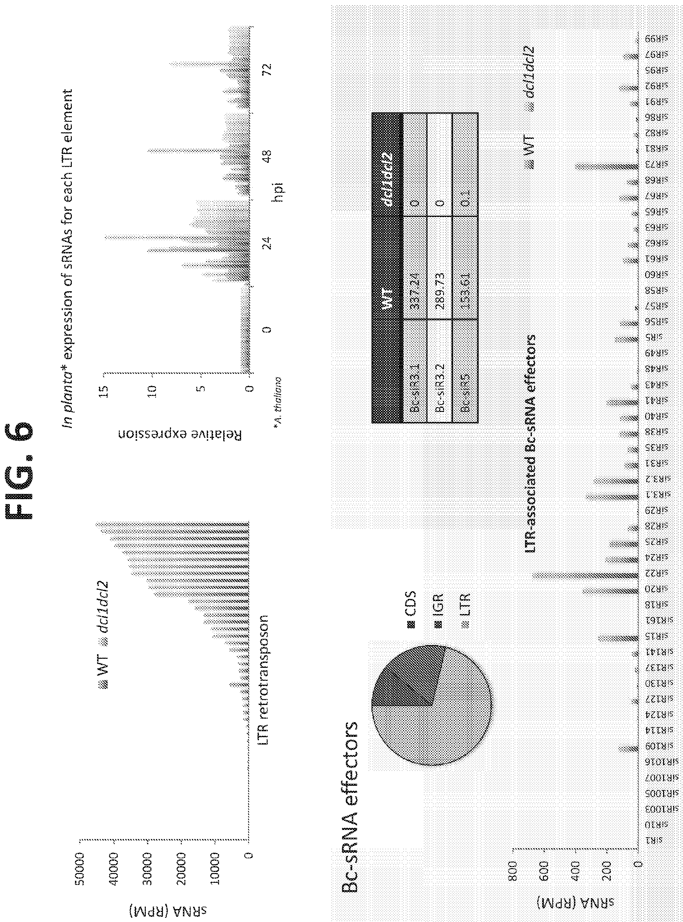

**FIG. 6****. LTR-derived Bc-sRNA effectors are dependent on DCL1 and DCL2.**

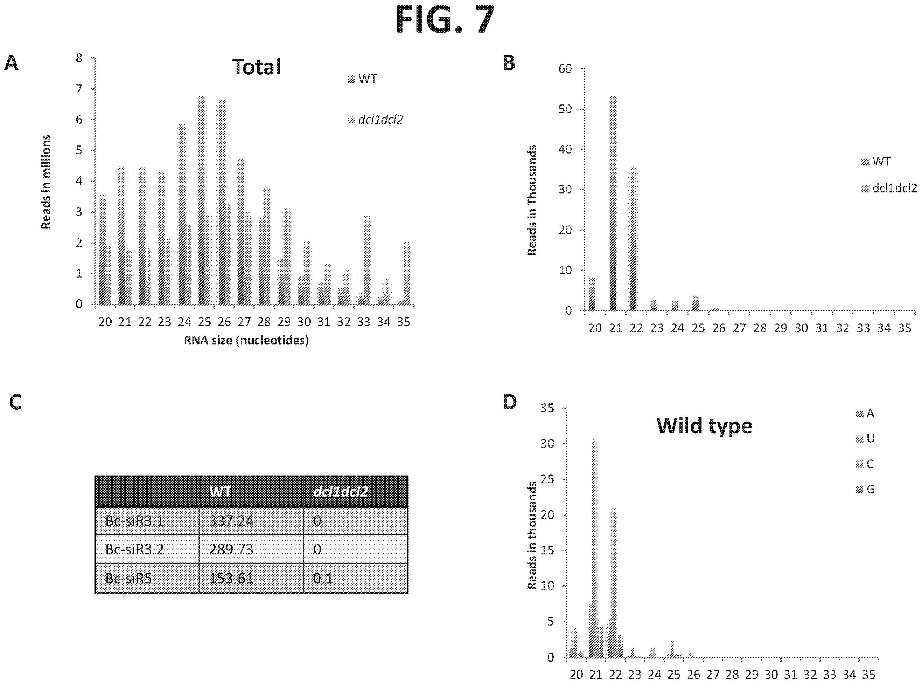

**FIG. 7A-D****. Retrotransposon-derived Bc-sRNAs are mostly BcDCL-dependent.** Two libraries were constructed from wild type *B. cinerea* and the *dcl1 dcl2* double mutant and sequenced using Illumina deep sequencing. (A) The read numbers of all Bc-sRNA reads from the two libraries according to sRNA size. (B) The read numbers of retrotransposon-derived Bc-sRNA from the two libraries according to sRNA size. (C) The normalized read numbers of Bc-siR3.1, Bc-siR3.2, and Bc-siR5 from the two libraries. (D) The read numbers of retrotransposon-derived Bc-sRNAs according to 5' nucleotide (A, U, C, or G) and sRNA size. The X-axis in A, B, and D indicates RNA size in nucleotides.

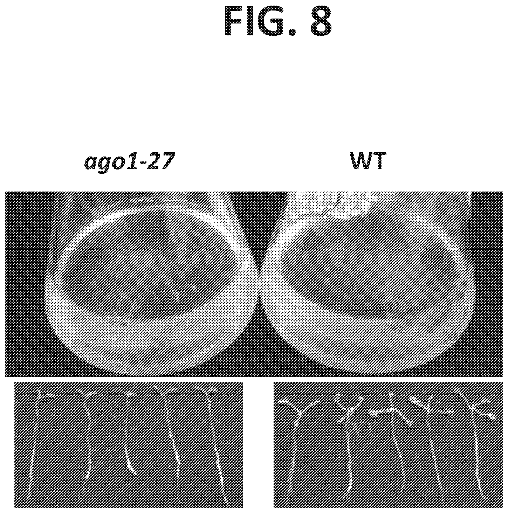

**FIG. 8****. Some *Verticillium* small RNAs were highly enriched in AGO1 pull-down fraction after infection.** Root culture was performed to obtain material for immunoprecipitation of AGO1-associated small RNA in wild-type (WT) and *ago1-27* mtuant Arabidopsis following infection. sRNAs that are associated with Arabidopsis AGO1 were pulled down and subjected to deep sequencing.

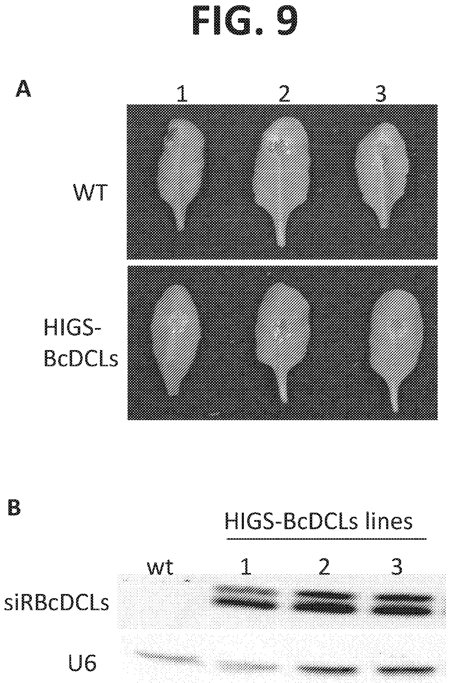

**FIG. 9A-B****. Knocking down BcDCLs by host induced gene silencing (HIGS) in transgenic Arabidopsis enhances plant resistance to *B. cinerea.*** (A) Three selected *B. cinerea dcl1dcl2* (HIGS-BcDCL) lines as well as wild type plants were infected with *B. cinerea.* Photographs were taken 4 days post infection (dpi). Three biological repeats indicated similar results. (B) The expression level of siRBcDCLs from wild type and three selected transgenic lines transformed with HIGS-BcDCLs as measured by Northern blot. U6 was used as loading control.

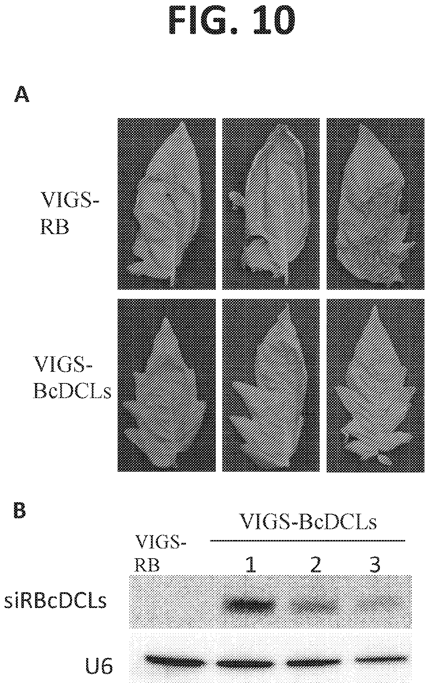

**FIG. 10A-B. Knocking down BcDCLs by virus induced gene silencing (VIGS)** **in tomato enhances plant resistance to *B. cinerea.*** (A) The fifth, sixth, and seventh leaves of tomato VIGS-RB and VIGS-BcDCLs plants were detached and infected with *B. cinerea* using spray inoculation. Photographs were taken 3 dpi. Three biological repeats indicated similar results. (B) The levels of siRBcDCLs from the corresponding infected leaves were measured by Northern blot. U6 was used as loading control.

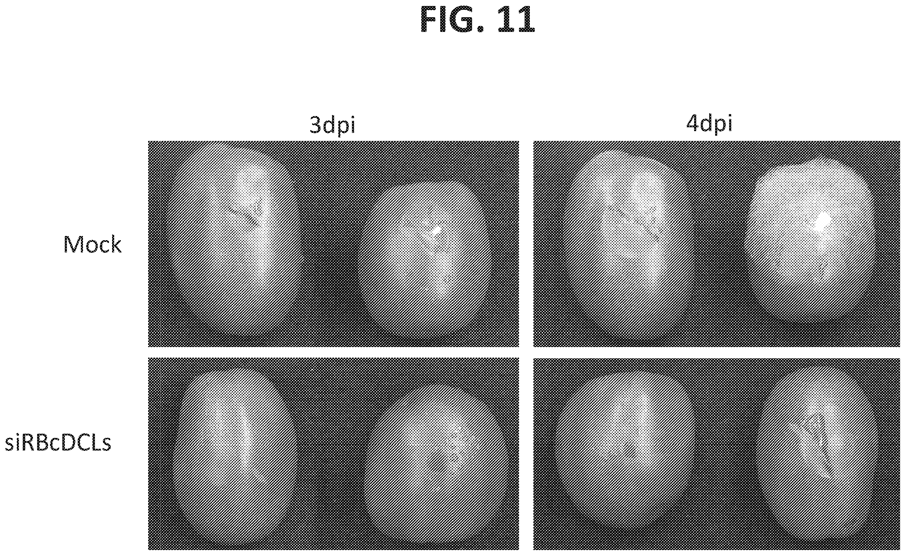

**FIG. 11****. Tomato was more resistant against *B. cinerea* when sprayed with RNA containing siRBcDCLs.** Mock RNA: Total RNA extracted from tobacco infiltrated by mock. siRBcDCLs RNA: Small RNA extracted from tobacco infiltrated by Agrobacteria carrying siRBcDCLs producing vector (pHellsgate8-B052DCLs).

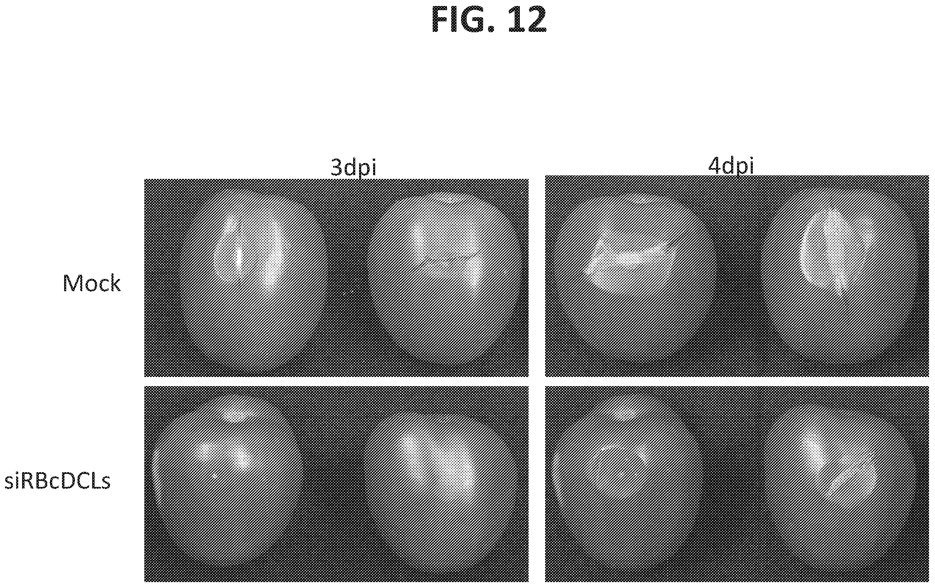

**FIG. 12****.** *B.* ***cinerea* was less virulent to tomatoes when mixed with *N. benthamiana* total RNA containing siRBcDCLs.** Mock RNA: Total RNA extracted from tobacco infiltrated by mock. siRBcDCLs RNA: Small RNA extracted from tobacco infiltrated by Agrobacteria carrying siRBcDCLs producing vector (pHellsgate8-B052DCLs).

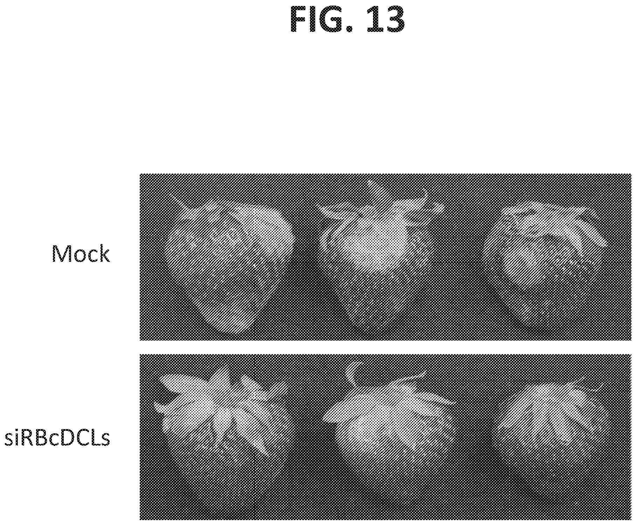

**FIG. 13****. *B. cinerea* was less virulent to strawberries when mixed with RNA containing siRBcDCLs.** Mock RNA: Total RNA extracted from tobacco infiltrated by mock. siRBcDCLs RNA: Small RNA extracted from tobacco infiltrated by Agrobacteria carrying siRBcDCLs producing vector (pHellsgate8-B052DCLs).

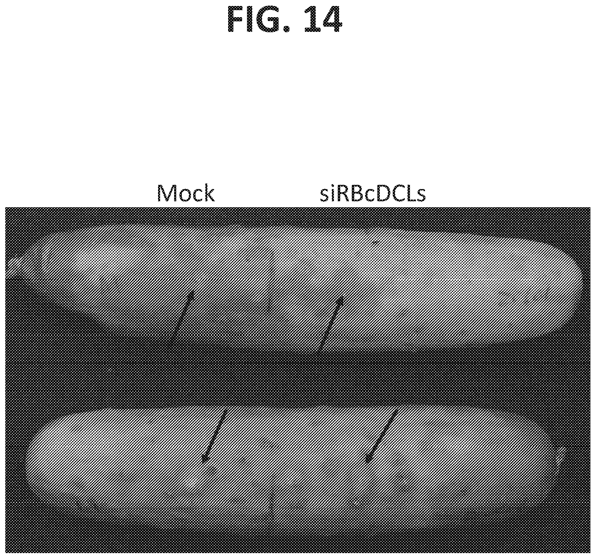

**FIG. 14****. *B. cinerea* was less virulent to cucumbers when mixed with RNA containing siRBcDCLs.** Mock RNA: Total RNA extracted from tobacco infiltrated by mock. siRBcDCLs RNA: Small RNA extracted from tobacco infiltrated by Agrobacteria carrying siRBcDCLs producing vector (pHellsgate8-B052DCLs).

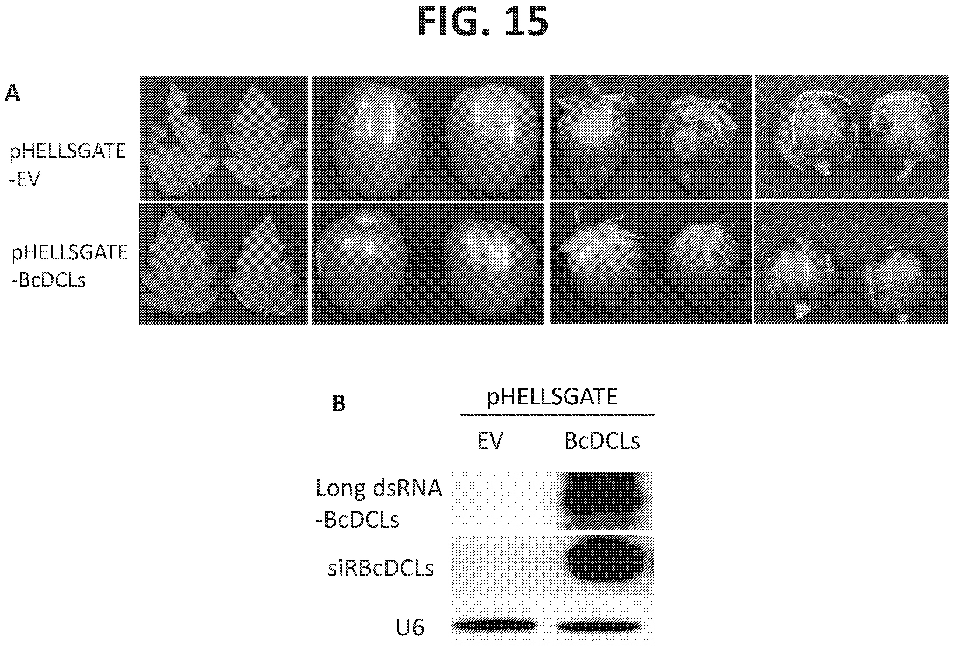

**FIG. 15A-B****. Spraying fruits with RNAs extracted from *N. Benthamiana* expressing Bc-DCL-targeting sRNAs exhibited reduced gray mold disease symptoms caused by *B. cinerea.*** (A) Tomato leaves and fruits, strawberry fruits, and grape fruits were pretreated with the total RNA from *N. Benthamiana* infiltrated with pHELLSGATE-BcDCLs and pHELLSGATE-EV by spray for 24 hours, followed by *B. cinerea* drop inoculation on the sprayed area of the fruits. The disease symptoms were recorded at 4 dpi for tomato and grape fruits and at 3 dpi for tomato leaves and strawberry fruits. Three biological repeats indicated similar results. (B) The expression levels of BcDCLs RNAi triggers, including long dsRNA-BcDCLs and siRBcDCLs, from total RNA extracted from *N. Benthamiana* infiltrated with with pHELLSGATE-BcDCLs or pHELLSGATE-EV. U6 was used as loading control.

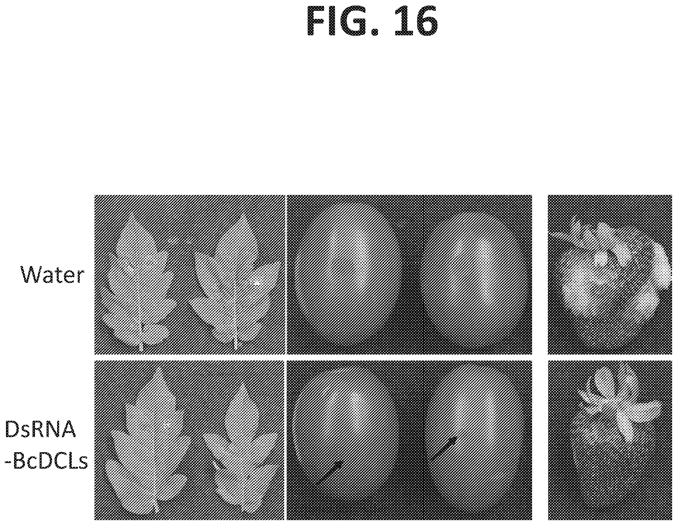

**FIG. 16****. Spraying tomato leaves and fruits with *in vitro* transcribed dsRNA against BcDCLs reduced gray mold disease caused by *B. cinerea* on tomato and strawberry.** Tomato leaves and fruits and strawberry fruits were pre-treated with water or *in vitro* transcribed long dsRNA against BcDCLs for 24 hours, followed by *B. cinerea* drop inoculation right on the pretreated area of the leaves or fruits. The disease symptoms were recorded at 4 dpi for tomato fruits and at 3 dpi for tomato leaves and strawberry fruits.

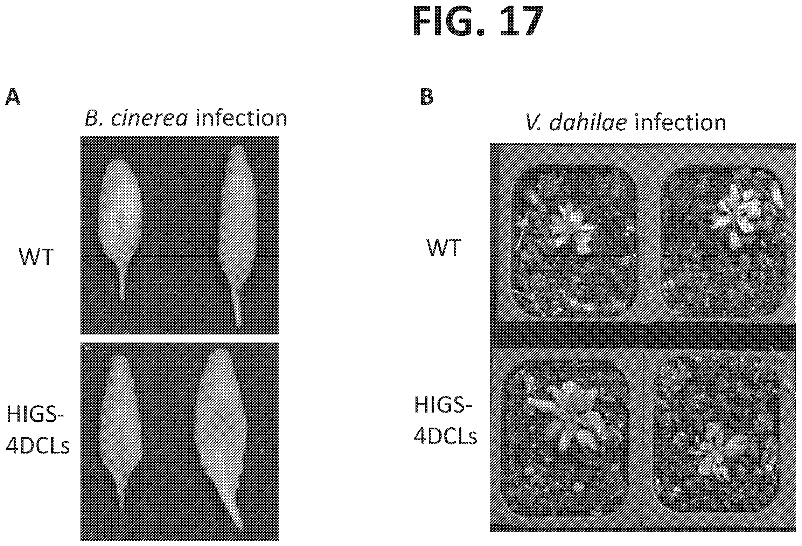

**FIG. 17A-B****. HIGS of DCL genes of both *B. cinerea* and *V. dahilae* increased plant tolerance to both pathogens.** (A) Two selected HIGS-4DCLs lines (4 weeks old) as well as wild type Arabidopsis plants were infected with *B. cinerea* by drop inoculation on the leaves. The pictures were taken after 4 dpi. Two biological repeats indicated similar results. (B). Two selected HIGS-4DCLs lines (2 weeks old) and wild type Arabidopsis plants were infected with *V. dahilae* by root inoculation. The pictures were taken after 3 weeks infection. Two biological repeats indicated similar results.

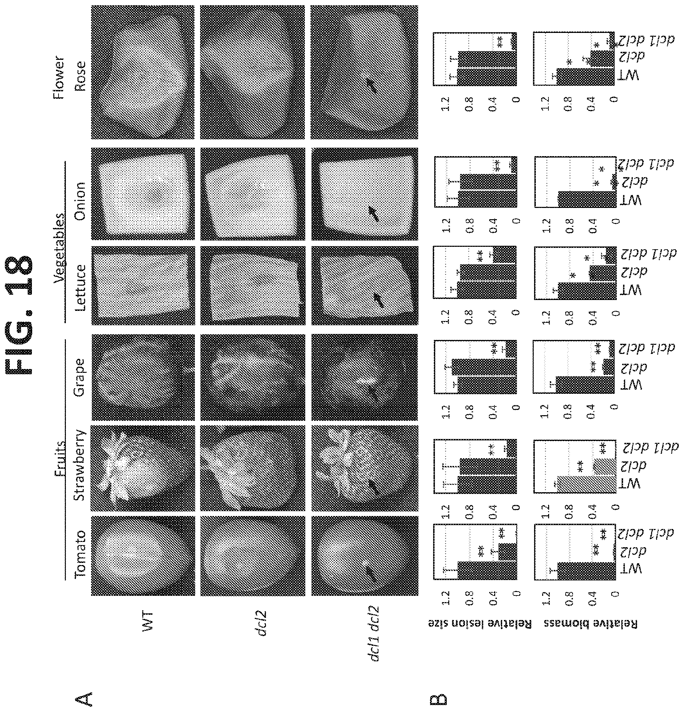

**FIG. 18A-B.** ***B. cinerea dcl1 dcl2* double mutant has reduced virulence on fruits, vegetables, and flower petals.** (A) *B. cinerea dcl1 dcl2* double mutant showed compromised virulence on fruits (tomato, strawberry, and grape), vegetables (lettuce and onion), and flower petals (rose), while *B. cinerea dcl2* single mutant showed similar virulence as or slightly reduced virulence than the WT strain. Similar results were obtained from at least three biological replicates. (B) Upper panel: Lesion sizes of the infected plant samples were measured 3 to 5 days post inoculation (dpi) using ImageJ, and error bars indicate the SD of at least 10 samples in each experiment. Lower panel: *B. cinerea* biomass was measured by quantitative PCR at 3 to 5 dpi. Error bars indicate the SD of three replicates. Asterisks indicate statistically significant differences (P < 0.01; Student's t test). The same experiments were performed three times for b and c, and yielded similar results.

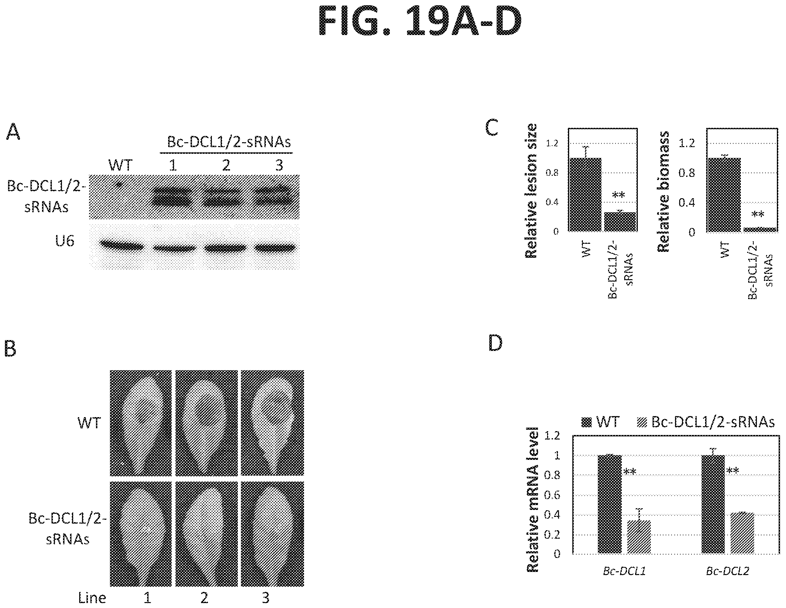

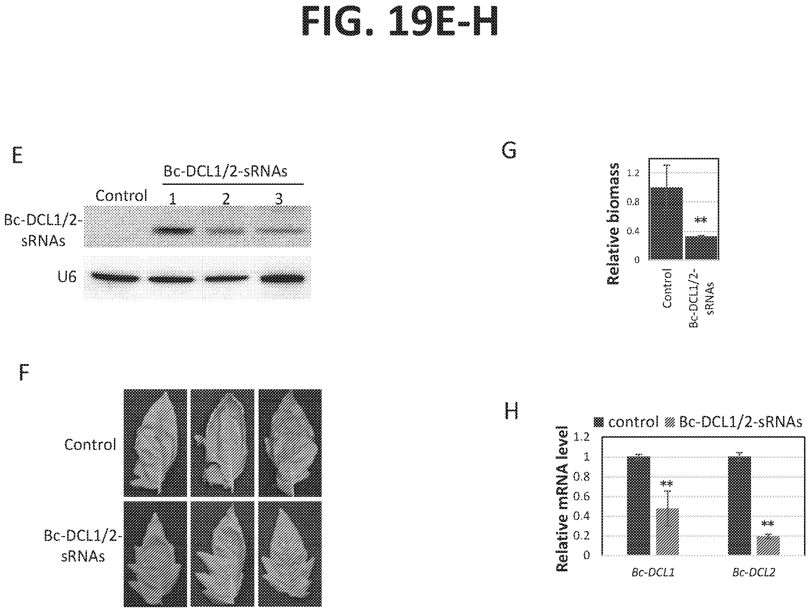

**FIG. 19A-H****. Expressing Bc-DCL1/2-sRNAs in Arabidopsis and tomato plants enhances plant resistance to** ***B. cinerea.*** (A) Bc-DCL1/2-sRNAs were highly expressed in the Arabidopsis transgenic plants, as detected by sRNA Northern blot. (B) Arabidopsis plants expressing Bc-DCL1/2-sRNAs showed enhanced disease resistance against *B. cinerea.* Similar results were obtained from three biological replicates. (C) Lesion sizes were measured 3 dpi using imageJ, and error bars indicate the SD of at least 10 samples. *B. cinerea* biomass was measured 3 dpi by quantitative PCR, and error bars indicate the SD of three replicates. Similar results were obtained from three biological replicates for (B) and (C). (D) The expression of *Bc-DCL1 and Bc-DCL2* were downregulated in infected Arabidopsis plants expressing Bc-DCL1/2-sRNAs, as measured by quantitative RT-PCR. (E) Northern blot analysis revealed the expression levels of Bc-DCL1/2-sRNAs in the tomato plants expressing Bc-DCL1/2-sRNAs through VIGS. (F) Tomato plants expressing Bc-DCL1/2-sRNAs were more resistant to *B. cinerea* compared to control plants. (G) *B. cinerea* biomass was measured 3 dpi, and error bars indicate the SD of three replicates. (H) *Bc-DCL1* and *Bc-DCL2* were silenced in infected tomato plants expressing Bc-DCL1/2-sRNAs, as measured by quantitative RT-PCR. Similar results were obtained from three biological replicates for (E)-(H). Asterisks indicate statistically significant differences (P < 0.01; Student's *t* test).

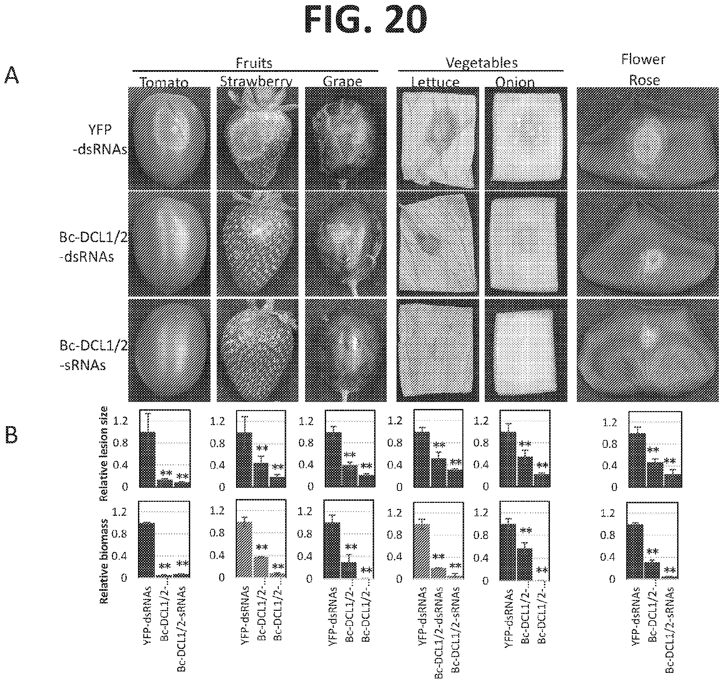

**FIG. 20A-B****. Externally applied Bc-DCL1/2-sRNAs and -dsRNAs inhibited pathogen virulence on fruits, vegetables, and flower petals.** (A) External application of Bc-DCL1/2-dsRNAs and -sRNAs inhibited the virulence of *B. cinerea* on fruits (tomato, strawberry, and grape), vegetables (lettuce and onion), and flower petals (rose), compared to control. Similar results were obtained from 4 biological repeats. (B) The lesion size and fungal biomass were measured 3 to 5 dpi using imageJ and quantitative PCR, respectively, and error bars indicate the SD of 10 samples and three repeats for the relative lesion size and relative biomass, respectively. Asterisks indicate statistically significant differences (P < 0.01; Student's *t* test). These experiments were repeated three times with similar results.

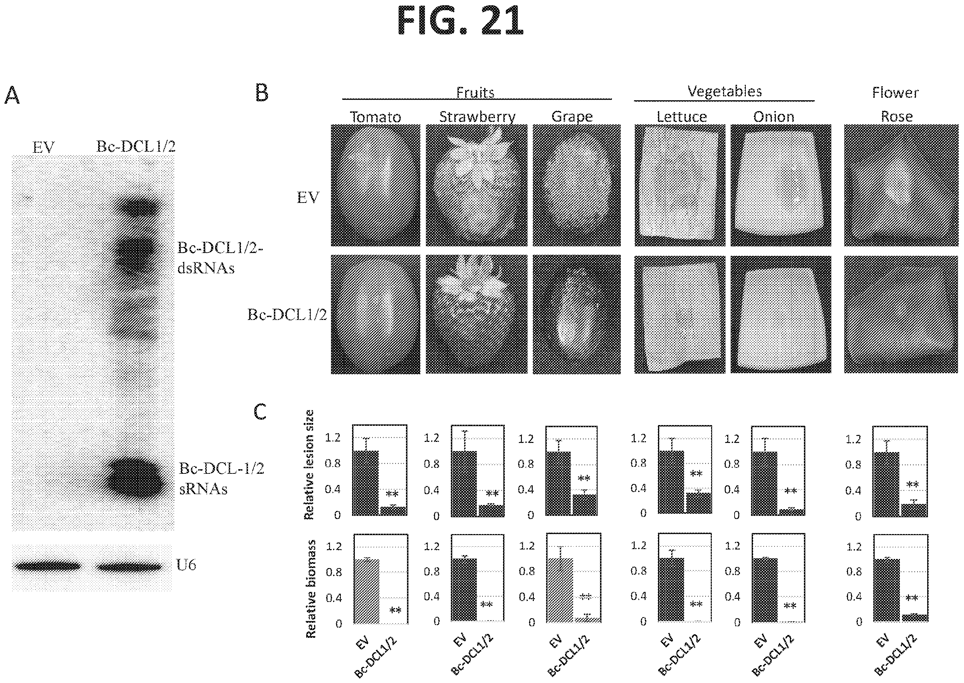

**FIG. 21A-C****. Treatment of plants with *N. Benthamiana* RNA extracts expressing Bc-DCL1/2-sRNAs and -dsRNAs reduced grey mold disease symptoms caused by** ***B. cinerea.*** (A) The *N. Benthamiana* total RNA extracts from plants expressing pHELLSGATE (EV) or expressing pHELLSGATE-Bc-DCL1/2 were used to measure the expression levels of Bc-DCL-sRNAs and -dsRNAs via Northern blot analysis. ³²P-labeled Bc-DCL-DNA was used as the probe. (B) The external application of *N. Benthamiana* total RNA extracts containing Bc-DCL1/2-sRNAs and -dsRNAs on fruits (tomato, strawberry, and grape), vegetables (lettuce and onion), and flower petals (rose) reduced grey mold disease symptoms, when compared with the external application of *N. Benthamiana* total RNA extracts without Bc-DCL1/2-sRNAs and -dsRNAs. (C) The lesion sizes were measured 3 to 5 dpi using imageJ, and error bars represent the SD of 10 plant samples. The biomass of *B. cinerea* was also calculated with quantitative PCR. Asterisks indicate statistically significant differences (P < 0.01; Student's *t* test). These experiments were repeated three times and yielded similar results.

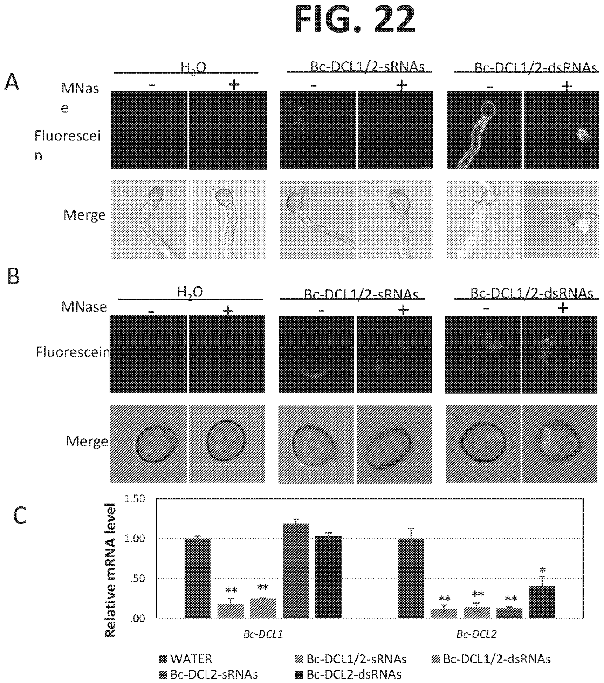

**FIG. 22A-C****. *B. cinerea* cells take up Bc-DCL1/2-sRNAs and -dsRNAs, which silenced *Bc-DCL1* and** ***Bc-DCL2.*** (A) Fluorescent Bc-DCL1/2-sRNAs and -dsRNAs were examined in the *B. cinerea* cells after 12 hours of co-culturing with *B. cinerea* spores on solid ME medium. Fluorescence-labeled DCL1/2-sRNAs and -dsRNAs were detected in the *B*. *cinerea* cells. They were still present in the *B. cinerea* cells after MNase treatment. (B) Fluorescent Bc-DCL1/2-sRNAs and -dsRNAs were observed in *B. cinerea* protoplasts after 16 hours of co-culturing with *B. cinerea* spores in liquid YEPD medium. MNase treatment of the protoplast did not eliminate the fluorescence. (C) *Bc-DCL1* and *Bc-DCL2* were examined at 40 hours after *B. cinerea* spores were co-cultured with Bc-DCL1/2-sRNAs and -dsRNAs in the liquid YEPD medium. *Bc-DCL2-targeting* sRNAs and dsRNAs only downregulated *Bc-DCL2* but not *Bc-DCL1.*

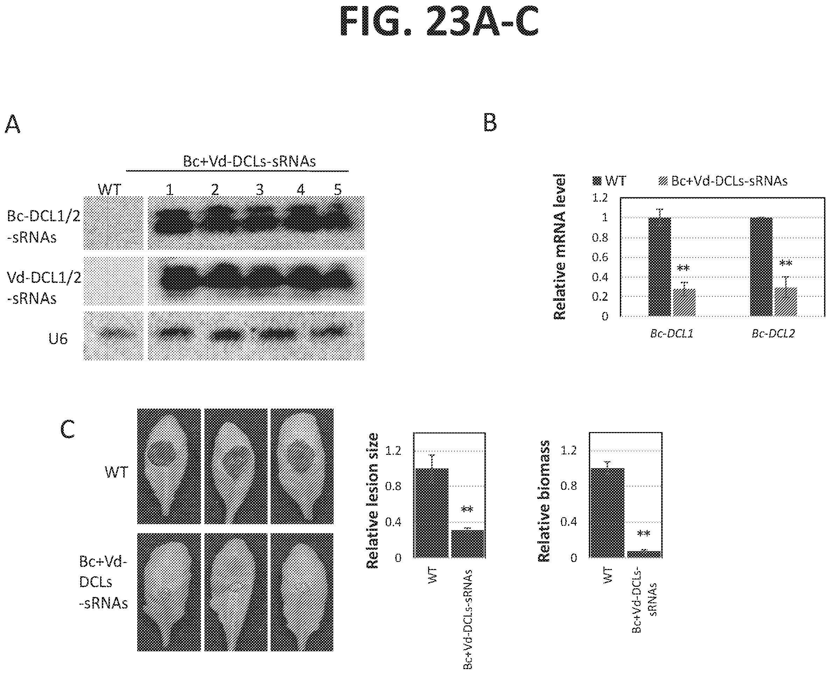

**FIG. 23A-E****. Arabidopsis plants simultaneously expressing sRNAs that target *DCL* genes *of B. cinerea* and *V. dahilae* show enhanced disease resistance to both pathogens.** (A) Northern blot analysis indicated the expression levels of Bc-DCL1/2-sRNAs and Vd-DCL1/2-sRNAs in the Arabidopsis transgenic plants expressing Bc+Vd-DCLs-sRNAs. (B) Quantitative RT-PCR showed that *Bc-DCL1* and *Bc-DCL2* were silenced in *B. cinerea-infected* Arabidopsis plants expressing Bc+Vd-DCL-sRNAs compared with WT plants. (C) Arabidopsis plants expressing Bc+Vd-DCLs-sRNAs inhibit the virulence *B*. *cinerea.* Lesion sizes were measured 3 dpi using ImageJ, and error bars indicate the SD of 10 leaves. *B. cinerea* biomass was measured 3 dpi by quantitative PCR. (D) The expression level of *Vd-DCL1* and *Vd-DCL2* were suppressed in *V. dahilae-infected* Arabidopsis plants expressing Bc+Vd-DCLs-sRNAs. (E) Arabidopsis plants expressing Bc+Vd-DCL-sRNAs were less susceptible to *V. dahilae* compared to WT plants. Biomass of *V. dahilae* was measured at 3 weeks post inoculation. Asterisks represented statistically significant differences (P < 0.01; Student's t test). Similar results were obtained from three replicates for (B)-(E).

### DEFINITIONS

The term "pathogen-resistant" or "pathogen resistance" refers to an increase in the ability of a plant to prevent or resist pathogen infection or pathogen-induced symptoms. Pathogen resistance can be increased resistance relative to a particular pathogen species or genus (*e.g., Botrytis*)*,* increased resistance to multiple pathogens, or increased resistance to all pathogens (*e.g*., systemic acquired resistance). In some embodiments, resistance of a plant to a pathogen is "increased" when one or more symptoms of pathogen infection are reduced relative to a control (e.g., a plant in which a polynucleotide that inhibits expression of a fungal pathogen DCL gene is not expressed).

"Pathogens" include, but are not limited to, viruses, bacteria, nematodes, fungi or insects (*see, e.g.,* Agrios, *Plant Pathology* (Academic Press, San Diego, CA (1988)). In some aspects of the disclosure, the pathogen is a fungal pathogen. In some embodiments, the pathogen is *Botrytis.*

The term "nucleic acid" or "polynucleotide" refers to a single or double-stranded polymer of deoxyribonucleotide or ribonucleotide bases read from the 5' to the 3' end. Nucleic acids may also include modified nucleotides that permit correct read through by a polymerase and do not significantly alter expression of a polypeptide encoded by that nucleic acid.

The phrase "nucleic acid encoding" or "polynucleotide encoding" refers to a nucleic acid which directs the expression of a specific protein or peptide. The nucleic acid sequences include both the DNA strand sequence that is transcribed into RNA and the RNA sequence that is translated into protein. The nucleic acid sequences include both the full length nucleic acid sequences as well as non-full length sequences derived from the full length sequences. It should be further understood that the sequence includes the degenerate codons of the native sequence or sequences which may be introduced to provide codon preference in a specific host cell.

Two nucleic acid sequences or polypeptides are said to be "identical" if the sequence of nucleotides or amino acid residues, respectively, in the two sequences is the same when aligned for maximum correspondence as described below. "Percentage of sequence identity" is determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. When percentage of sequence identity is used in reference to proteins or peptides, it is recognized that residue positions that are not identical often differ by conservative amino acid substitutions, where amino acid residues are substituted for other amino acid residues with similar chemical properties (*e.g*., charge or hydrophobicity) and therefore do not change the functional properties of the molecule. Where sequences differ in conservative substitutions, the percent sequence identity may be adjusted upwards to correct for the conservative nature of the substitution. Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated according to, *e.g.,* the algorithm of Meyers & Miller, Computer Applic. Biol. Sci. 4:11-17 (1988) *e.g*., as implemented in the program PC/GENE (Intelligenetics, Mountain View, California, USA).

The term "substantial identity" or "substantially identical," as used in the context of polynucleotide or polypeptide sequences, refers to a sequence that has at least 60% sequence identity to a reference sequence. Alternatively, percent identity can be any integer from 60% to 100%. Exemplary aspects of the disclosure include at least: 60%, 65%, 70%, 75%, 80%, 85%, 90%, 91%, 92%, 93%, 94%, 95%, 96%, 97%, 98%, or 99%, as compared to a reference sequence using the programs described herein; preferably BLAST using standard parameters, as described below. One of skill will recognize that these values can be appropriately adjusted to determine corresponding identity of proteins encoded by two nucleotide sequences by taking into account codon degeneracy, amino acid similarity, and reading frame positioning.

For sequence comparison, typically one sequence acts as a reference sequence to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window," as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad. Sci. (U.S.A.) 85: 2444 (1988), by computerized implementations of these algorithms (GAP, BESTFIT, BLAST, FAST A, and TFASTA in the Wisconsin Genetics Software Package, Genetics Computer Group (GCG), 575 Science Dr., Madison, WI), or by manual alignment and visual inspection.

Algorithms that are suitable for determining percent sequence identity and sequence similarity are the BLAST and BLAST 2.0 algorithms, which are described in Altschul et al. (1990) J. Mol. Biol. 215: 403-410 and Altschul et al. (1977) Nucleic Acids Res. 25: 3389-3402, respectively. Software for performing BLAST analyses is publicly available through the National Center for Biotechnology Information (NCBI) web site. The algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al, supra*)*.* These initial neighborhood word hits acts as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word size (W) of 28, an expectation (E) of 10, M=1, N=-2, and a comparison of both strands. For amino acid sequences, the BLASTP program uses as defaults a word size (W) of 3, an expectation (E) of 10, and the BLOSUM62 scoring matrix (*see* Henikoff & Henikoff, Proc. Natl. Acad. Sci. USA 89:10915 (1989)).

The BLAST algorithm also performs a statistical analysis of the similarity between two sequences (*see, e.g.,* Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.01, more preferably less than about 10⁻⁵, and most preferably less than about 10⁻²⁰.

The term "complementary to" is used herein to mean that a polynucleotide sequence is complementary to all or a portion of a reference polynucleotide sequence. In some aspects of the disclosure, a polynucleotide sequence is complementary to at least 15, at least 20, at least 25, at least 30, at least 40, at least 50, at least 75, at least 100, at least 125, at least 150, at least 175, at least 200, or more contiguous nucleotides of a reference polynucleotide sequence. In some aspects of the disclosure, a polynucleotide sequence is "substantially complementary" to a reference polynucleotide sequence if at least 70%, at least 75%, at least 80%, at least 85%, at least 90%, or at least 95% of the polynucleotide sequence is complementary to the reference polynucleotide sequence.

A polynucleotide sequence is "heterologous" to an organism or a second polynucleotide sequence if it originates from a foreign species, or, if from the same species, is modified from its original form. For example, when a promoter is said to be operably linked to a heterologous coding sequence, it means that the coding sequence is derived from one species whereas the promoter sequence is derived another, different species; or, if both are derived from the same species, the coding sequence is not naturally associated with the promoter (*e.g*., is a genetically engineered coding sequence, *e.g.,* from a different gene in the same species, or an allele from a different ecotype or variety).

An "expression cassette" refers to a nucleic acid construct, which when introduced into a host cell, results in transcription and/or translation of a RNA or polypeptide, respectively. Antisense constructs or sense constructs that are not or cannot be translated are expressly included by this definition. One of skill will recognize that the inserted polynucleotide sequence need not be identical, but may be only substantially similar to a sequence of the gene from which it was derived.

The term "promoter," as used herein, refers to a polynucleotide sequence capable of driving transcription of a coding sequence in a cell. Thus, promoters used in the polynucleotide constructs of the disclosure include cis-acting transcriptional control elements and regulatory sequences that are involved in regulating or modulating the timing and/or rate of transcription of a gene. For example, a promoter can be a cis-acting transcriptional control element, including an enhancer, a promoter, a transcription terminator, an origin of replication, a chromosomal integration sequence, 5' and 3' untranslated regions, or an intronic sequence, which are involved in transcriptional regulation. These cis-acting sequences typically interact with proteins or other biomolecules to carry out (turn on/off, regulate, modulate, *etc.*) gene transcription. A "plant promoter" is a promoter capable of initiating transcription in plant cells. A "constitutive promoter" is one that is capable of initiating transcription in nearly all tissue types, whereas a "tissue-specific promoter" initiates transcription only in one or a few particular tissue types. An "inducible promoter" is one that initiates transcription only under particular environmental conditions or developmental conditions.

The term "plant" includes whole plants, shoot vegetative organs and/or structures *(e.g.,* leaves, stems and tubers), roots, flowers and floral organs *(e.g.,* bracts, sepals, petals, stamens, carpels, anthers), ovules (including egg and central cells), seed (including zygote, embryo, endosperm, and seed coat), fruit (*e.g.,* the mature ovary), seedlings, plant tissue (*e.g.*, vascular tissue, and ground tissue), cells (*e.g*., guard cells, egg cells, and trichomes), and progeny of same. The class of plants that can be used in the method of the invention is generally as broad as the class of higher and lower plants amenable to transformation techniques, including angiosperms (monocotyledonous and dicotyledonous plants), gymnosperms, ferns, and multicellular algae. It includes plants of a variety of ploidy levels, including aneuploid, polyploid, diploid, haploid, and hemizygous.

### DETAILED DESCRIPTION OF THE INVENTION

### I. Introduction

It has been found that aggressive eukaryotic fungal pathogens, such as *Botrytis* and *Verticillium,* have evolved a novel virulence mechanism by employing small RNAs as effector molecules to suppress host immune responses to achieve successful infection. It has also been found that the majority of the small RNA effectors are generated from transposon regions, mainly the retrotransposon long terminal repeats (LTRs). As reported in genome studies of other fungal and oomycete pathogens, many fungal protein effector genes are also enriched in the transposon regions, including LTRs. These LTR-derived small RNAs, including most small RNA effectors, are generated by fungal Dicer-like proteins (DCLs).

As shown herein, DCL genes are essential for the pathogenicity of eukaryotic pathogens, such as the fungal pathogens *Botrytis* and *Verticillium,* with small RNA effectors. Thus, DCL genes are excellent targets for controlling those eukaryotic pathogens that use small RNAs as effectors. For example, *Botrytis* is a significant pathogen not only in the field, but also at post-harvesting stages, and can infect many different fruit, vegetable, and flowering plants.

Thus, one aspect of the present disclosure relates to controlling the diseases caused by aggressive fungal and oomycete pathogens by silencing their DCL genes and LTRs (e.g., using a host-induced gene silencing (HIGS) mechanism). In some embodiments, silencing is achieved by generating transgenic plants that express antisense (*e.g*., RNAi) constructs that target fungal or oomycete DCLs. In some embodiments, silencing is achieved by contacting (*e.g*., spraying) plants with small RNA duplexes or double stranded RNAs that target pathogen DCLs. *Botrytis* and *Verticillium* DCLs are exemplary genes that can be targeted.

### II. Fungal Pathogen DCL Genes and LTR Regions

In one aspect of the disclosure, methods of inhibiting or silencing expression of fungal pathogen dicer-like (DCL) genes or long terminal repeat (LTR) regions are provided. In some aspects of the disclosure, the method comprises expressing in a plant an expression cassette comprising a promoter operably linked to a polynucleotide that inhibits expression of a fungal pathogen DCL gene or an expression cassette comprising a promoter operably linked to a polynucleotide that inhibits expression of a fungal pathogen LTR region. In some aspects of the disclosure, the method comprises contacting the plant with a construct comprising a promoter operably linked to a polynucleotide that inhibits expression of a fungal pathogen DCL gene or a construct comprising a promoter operably linked to a polynucleotide that inhibits expression of a fungal pathogen LTR region. In some aspects of the disclosure, the polynucleotide comprises an antisense nucleic acid that is complementary to the DCL gene or a fragment thereof. In some aspects of the disclosure, the polynucleotide comprises a small RNA duplex or a double-stranded RNA that targets the DCL gene or a fragment thereof. In some aspects of the disclosure, the polynucleotide sequence comprises an inverted repeat of a sequence targeting the DCL gene, optionally with a spacer present between the inverted repeat sequences. In some aspects of the disclosure, the polynucleotide comprises an antisense nucleic acid that is complementary to the LTR region or a fragment thereof. In some aspects of the disclosure, the polynucleotide comprises a small RNA duplex or a double-stranded RNA that targets the LTR region or a fragment thereof. In some aspects of the disclosure, the polynucleotide sequence comprises an inverted repeat of a sequence targeting the LTR region, optionally with a spacer present between the inverted repeat sequences. In some aspects of the disclosure, the promoter is an inducible promoter. In some aspects of the disclosure, the promoter is a constitutively active promoter.

In another aspect of the disclosure, plants having inhibited or silenced expression of pathogen DCL genes or LTR region are provided. In some aspects of the disclosure, the plant is contacted with a polynucleotide that inhibits expression of a pathogen DCL gene or a pathogen LTR region, wherein the plant has increased pathogen resistance relative to a control plant that is not contacted with the polynucleotide. In some aspects of the disclosure, the plant comprises a heterologous expression cassette, the expression cassette comprising a polynucleotide that inhibits expression of a pathogen DCL or LTR region, wherein the plant has increased pathogen resistance relative to a control plant lacking the expression cassette. In some aspects of the disclosure, the polynucleotide comprises an antisense nucleic acid that is complementary to the DCL gene or LTR region or a fragment thereof. In some aspects of the disclosure, the polynucleotide comprises a double stranded nucleic acid that targets the DCL gene or LTR region or a fragment thereof.

In yet another aspect of the disclosure, expression cassettes comprising a promoter operably linked to a polynucleotide that inhibits expression of a pathogen DCL gene, or isolated nucleic acids comprising said expression cassettes, are provided. In some aspects of the disclosure, the expression cassette comprises a promoter operably linked to a polynucleotide comprising an antisense nucleic acid that is complementary to the DCL gene or a fragment thereof. In some aspects of the disclosure, the expression cassette comprises a promoter operably linked to a polynucleotide comprising a double stranded nucleic acid that targets the DCL gene or a fragment thereof. In some aspects of the disclosure, a plant in which the expression cassette is introduced has increased resistance to the pathogen compared to a control plant lacking the expression cassette.

### Pathogen DCL Genes and Polynucleotides Targeting Pathogen DCL Genes

In some aspects of the disclosure, the pathogen DCL gene or DCL promoter to be targeted or silenced is from a viral, bacterial, fungal, nematode, oomycete, or insect pathogen. In some aspects of the disclosure, the DCL gene is from a fungal pathogen. Examples of plant fungal pathogens include, but are not limited to, *Botyritis, Verticillium, Magnaporthe, Sclerotinia, Puccinia, Fusarium, Mycosphaerella, Blumeria, Colletotrichum, Ustilago,* and *Melampsora. See, e.g.,* Dean et al., Mol Plant Pathol 13:804 (2012). In some embodiments, the pathogen is *Botyritis.* In some embodiments, the pathogen is *Botyritis cinera.* In some embodiments, the pathogen is *Verticillium.* In some embodiments, the pathogen is *V. dahilae.*

In some aspects of the disclosure, one or more pathogen DCL genes is targeted, silenced, or inhibited in order to increase resistance to the pathogen in a plant by expressing in the plant, or contacting to the plant, a polynucleotide that inhibits expression of the pathogen DCL gene or that is complementary to the DCL gene or a fragment thereof. In some aspects of the disclosure, the polynucleotide comprises an antisense nucleic acid that is complementary to the DCL gene or a fragment thereof. In some aspects of the disclosure, the polynucleotide comprises a double stranded nucleic acid that targets the DCL gene, or its promoter, or a fragment thereof. In some aspects of the disclosure, the polynucleotide comprises a double-stranded nucleic acid having a sequence that is identical or substantially similar (at least 60%, 65%, 70%, 75%, 80%, 85%, 90%, 91%, 92%, 93%, 94%, 95%, 96%, 97%, 98%, or 99% identical) to the DCL gene or a fragment thereof. In some aspects of the disclosure, a "fragment" of a DCL gene or promoter comprises a sequence of at least 15, 16, 17, 18, 19, 20, 21, 22, 23, 24, 25, 26, 27, 28, 29, 30, 20, 30, 40, 50, 60, 70, 80, 90, 100, 150, 200, 250, 300, 350, 400, 450, 500 or more contiguous nucleotides of the DCL gene or promoter (e.g., comprises at least (e.g., at least 15, 16, 17, 18, 19, 20, 21, 22, 23, 24, 25, 26, 27, 28, 29, 30, 40, 50, 60, 70, 80, 90, 100, 150, 200, 250, 300, 350, 400, 450, 500 or more contiguous nucleotides of any of SEQ ID NO:1, SEQ ID NO:3, SEQ ID NO:5, SEQ ID NO:7, SEQ ID NO:9, SEQ ID NO:10, SEQ ID NO:11, SEQ ID NO:12, SEQ ID NO:28, SEQ ID NO:29, SEQ ID NO:30, or SEQ ID NO:31). In some aspects of the disclosure, the double stranded nucleic acid is a small RNA duplex or a double stranded RNA.

In some embodiments, the polynucleotide inhibits expression of a fungal pathogen DCL gene that encodes a *Botrytis* or *Verticillium* DCL protein. In some embodiments, the polynucleotide inhibits expression of a fungal DCL gene that encodes a *Botrytis* DCL protein that is identical or substantially identical (*e.g.,* at least 60%, 65%, 70%, 75%, 80%, 85%, 90%, 91%, 92%, 93%, 94%, 95%, 96%, 97%, 98%, or 99% identical) to SEQ ID NO:2 or SEQ ID NO:4, or a fragment thereof. In some embodiments, the polynucleotide inhibits expression of a fungal DCL gene that encodes a *Verticillium* DCL protein that is identical or substantially identical (*e.g.,* at least 60%, 65%, 70%, 75%, 80%, 85%, 90%, 91%, 92%, 93%, 94%, 95%, 96%, 97%, 98%, or 99% identical) to SEQ ID NO:6 or SEQ ID NO:8, or a fragment thereof.

In some aspects of the disclosure, the polynucleotide comprises a sequence that is identical or substantially identical (*e.g.,* at least 60%, 65%, 70%, 75%, 80%, 85%, 90%, 91%, 92%, 93%, 94%, 95%, 96%, 97%, 98%, or 99% identical) to SEQ ID NO:1 or SEQ ID NO:3 or a fragment thereof, or a complement thereof. In some aspects of the disclosure, the polynucleotide comprises a sequence that is identical or substantially identical (*e.g.,* at least 60%, 65%, 70%, 75%, 80%, 85%, 90%, 91%, 92%, 93%, 94%, 95%, 96%, 97%, 98%, or 99% identical) to SEQ ID NO:5 or SEQ ID NO:7 or a fragment thereof, or a complement thereof. In some aspects of the disclosure, the polynucleotide comprises a sequence that is identical or substantially identical (*e.g.,* at least 60%, 65%, 70%, 75%, 80%, 85%, 90%, 91%, 92%, 93%, 94%, 95%, 96%, 97%, 98%, or 99% identical) to SEQ ID NO:9, SEQ ID NO:10, SEQ ID NO:11, or SEQ ID NO:12, or a fragment thereof, or a complement thereof.

In some aspects of the disclosure, the polynucleotide comprises an inverted repeat of a sequence that is identical or substantially identical (*e.g.,* at least 60%, 65%, 70%, 75%, 80%, 85%, 90%, 91%, 92%, 93%, 94%, 95%, 96%, 97%, 98%, or 99% identical) to any of SEQ ID NO:1, SEQ ID NO:3, SEQ ID NO:5, SEQ ID NO:7, SEQ ID NO:9, SEQ ID NO:10, SEQ ID NO:11, or SEQ ID NO:12, or a fragment thereof, or a complement thereof. In some aspects of the disclosure, the polynucleotide comprises a spacer in between the inverted repeat sequences.

In some aspects of the disclosure, the polynucleotide targets a promoter region of a fungal pathogen DCL gene. For example, in some embodiments, the polynucleotide targets a promoter region within the sequence of any of SEQ ID NO:28, SEQ ID NO:29, SEQ ID NO:30, or SEQ ID NO:31.

In some aspects of the disclosure, two or more fungal pathogen DCL genes or promoters are targeted (e.g., two, three, four or more DCL genes or promoters from the same fungal pathogen or from two or more fungal pathogens). In some aspects of the disclosure, two or more *Botrytis* DCL genes or promoters are targeted. For example, in some aspects of the disclosure, two or more of SEQ ID NO:1, SEQ ID NO:3, SEQ ID NO:28, and SEQ ID NO:29, or a fragment of any thereof, are targeted for inhibition of expression. In some aspects of the disclosure, two or more *Verticillium* DCL genes or promoters are targeted. For example, in some aspects of the disclosure, two or more of SEQ ID NO:5, SEQ ID NO:7, SEQ ID NO:30, or SEQ ID NO:31, or a fragment of any thereof, are targeted for inhibition of expression.

### Pathogen LTR Regions and Polynucleotides Targeting Pathogen LTR Regions

The LTR regions that generate most small RNA effectors can be targeted for silencing. In some aspects of the disclosure, such as for *B. cinerea,* sRNA effectors are derived from LTR retrotransposon regions. Additionally, the promoter regions of LTRs can also be targeted for silencing. Targeting of LTR promoter regions can trigger transcriptional gene silencing, which would avoid random silencing of host genes by LTR small RNAs.

In some aspects of the disclosure, the polynucleotide targets or inhibits expression of a pathogen LTR region or of a promoter region of a pathogen LTR, wherein the pathogen is a fungal pathogen. In some aspects of the disclosure, the pathogen is *Botyritis.* In some aspects of the disclosure, the pathogen is *Botyritis cinera.* In some aspects of the disclosure, the pathogen is *Verticillium.* In some aspects of the disclosure, the pathogen is *V. dahilae.*

In some aspects of the disclosure, the polynucleotide targets a sequence of any of SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, or SEQ ID NO:27 or a fragment thereof, or a complement thereof. In some aspects of the disclosure, a "fragment" of a LTR region or LTR promoter comprises a sequence of at least 15, 20, 30, 40, 50, 60, 70, 80, 90, 100, 150, 200, 250, 300, 350, 400, 450, 500 or more contiguous nucleotides of the LTR region or LTR promoter (e.g., comprises at least 15, 20, 30, 40, 50, 60, 70, 80, 90, 100, 150, 200, 250, 300, 350, 400, 450, 500 or more contiguous nucleotides of any of SEQ ID NO: 13, SEQ ID NO: 14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, or SEQ ID NO:27).

In some aspects of the disclosure, the polynucleotide comprises an antisense nucleic acid that is complementary to any of SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO:20, SEQ ID NO:21, SEQ ID NO:22, SEQ ID NO:23, SEQ ID NO:24, SEQ ID NO:25, SEQ ID NO:26, SEQ ID NO:27 or a fragment thereof. In some aspects of the disclosure, the polynucleotide comprises a double-stranded nucleic acid having a sequence that is identical or substantially similar (at least 60%, 65%, 70%, 75%, 80%, 85%, 90%, 91%, 92%, 93%, 94%, 95%, 96%, 97%, 98%, or 99% identical) to any of SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, SEQ ID NO:23, SEQ ID NO:24, SEQ ID NO:25, SEQ ID NO:26, or SEQ ID NO:27 or a fragment thereof. In some aspects of the disclosure, the polynucleotide comprises an inverted repeat of a fragment of any of SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO:20, SEQ ID NO:21, SEQ ID NO:22, SEQ ID NO:23, SEQ ID NO:24, SEQ ID NO:25, SEQ ID NO:26, or SEQ ID NO:27, and further comprises a spacer region separating the inverted repeat nucleotide sequences.

In some aspects of the disclosure, the polynucleotide targets a promoter region of a fungal LTR. For example, in some aspects of the disclosure, the polynucleotide targets a promoter region within the sequence of SEQ ID NO:27.

### Host-Induced Gene Silencing

In some aspects of the disclosure, the methods of inhibiting or silencing expression of fungal pathogen DCL genes or LTR regions utilizes a host-induced gene silencing (HIGS) mechanism for producing in a host plant inhibitory RNA that subsequently moves into the pathogen to inhibit expression of a pathogen gene or region. In some aspects of the disclosure, HIGS is used to produce in a plant inhibitory RNAs (e.g., sRNAs) that target one or more pathogen DCLs or LTRs. In some aspects of the disclosure, wherein a pathogen has more than one DCL, HIGS is used to produce inhibitory RNAs (e.g., sRNAs) that target each of the DCLs of the pathogen (e.g., for *Botrytis,* targeting DCL1 and DCL2). In some aspects of the disclosure, HIGS is used to produce inhibitory RNAs (e.g., sRNAs) against DCLs or LTRs of multiple pathogens.

The use of HIGS for silencing expression of pathogen genes in plants is described, e.g., in Nowara et al. (Plant Cell (2010) 22:3130-3141); Nunes et al. (Mol Plant Pathol (2012) 13:519-529); and Govindarajulu et al. (Plant Biotechnology Journal (2014) 1-9). Pathogen sRNAs are described, for example, in US 2015/0203865.

### Antisense Technology

In some aspects of the disclosure, antisense technology is used to silence or inactive the pathogen DCL gene or LTR. The antisense nucleic acid sequence transformed into plants will be substantially identical to at least a fragment of the gene to be silenced. In some aspects of the disclosure, the antisense nucleic acid sequence that is transformed into plants is identical or substantially identical to the pathogen DCL sequence or LTR sequence to be blocked. In some aspects of the disclosure, the antisense polynucleotide sequence is complementary to the pathogen DCL sequence or LTR sequence to be blocked. However, the sequence does not have to be perfectly identical to inhibit expression. Thus, in some aspects of the disclosure, an antisense polynucleotide sequence that is substantially complementary (e.g., at least 70%, at least 75%, at least 80%, at least 85%, at least 90%, or at least 95% complementary) to the pathogen DCL sequence or LTR sequence to be blocked can be used (e.g., in an expression cassette under the control of a heterologous promoter, which is then transformed into plants such that the antisense nucleic acid is produced).

In some aspects of the disclosure, an antisense or sense nucleic acid molecule comprising or complementary to only a fragment of the pathogen DCL gene sequence or LTR sequence can be useful for producing a plant in which pathogen gene expression is silenced. For example, a sequence of about 15, 20, 30, 40, 50, 100, 150, 200, 250, 300, 350, 400, 450, or 500 nucleotides can be used.

Catalytic RNA molecules or ribozymes can also be used to inhibit expression of a pathogen DCL gene or LTR. It is possible to design ribozymes that specifically pair with virtually any target RNA and cleave the phosphodiester backbone at a specific location, thereby functionally inactivating the target RNA. In carrying out this cleavage, the ribozyme is not itself altered, and is thus capable of recycling and cleaving other molecules, making it a true enzyme. The inclusion of ribozyme sequences within antisense RNAs confers RNA-cleaving activity upon them, thereby increasing the activity of the constructs.

A number of classes of ribozymes have been identified. One class of ribozymes is derived from a number of small circular RNAs that are capable of self-cleavage and replication in plants. The RNAs replicate either alone (viroid RNAs) or with a helper virus (satellite RNAs). Examples include RNAs from avocado sunblotch viroid and the satellite RNAs from tobacco ringspot virus, lucerne transient streak virus, velvet tobacco mottle virus, solanum nodiflorum mottle virus and subterranean clover mottle virus. The design and use of target RNA-specific ribozymes is described in Haseloff et al. Nature, 334:585-591 (1988).

Another method of suppression is sense suppression (also known as co-suppression). Introduction of expression cassettes in which a nucleic acid is configured in the sense orientation with respect to the promoter has been shown to be an effective means by which to block the transcription of target genes. Generally, where inhibition of expression is desired, some transcription of the introduced sequence occurs. The effect may occur where the introduced sequence contains no coding sequence *per se,* but only intron or untranslated sequences homologous to sequences present in the primary transcript of the endogenous sequence. The introduced sequence generally will be substantially identical to the sequence intended to be repressed. This minimal identity will typically be greater than about 65% to the target gene sequence (e.g., DCL or LTR sequence), but a higher identity can exert a more effective repression of expression of the endogenous sequences. In some aspects of the disclosure, sequences with substantially greater identity are used, *e.g.,* at least about 80%, at least about 95%, or 100% identity are used. As with antisense regulation, the effect can be designed and tested so as to not significantly affect expression of other proteins within a similar family of genes exhibiting homology or substantial homology.

For sense suppression, the introduced sequence in the expression cassette, needing less than absolute identity, also need not be full length, relative to either the primary transcription product or fully processed mRNA. This may be preferred to avoid concurrent production of some plants that are overexpressers. A higher identity in a shorter than full length sequence compensates for a longer, less identical sequence. Furthermore, the introduced sequence need not have the same intron or exon pattern, and identity of non-coding segments will be equally effective. In some aspects of the disclosure, a sequence of the size ranges noted above for antisense regulation is used, *e.g.,* at least about 15, 20, 30, 40, 50, 60, 70, 80, 90, 100, 150, 200, 250, 300, 350, 400, 450, 500 or more nucleotides.

Gene expression may also be suppressed by means of RNA interference (RNAi) (and indeed co-suppression can be considered a type of RNAi), which uses a double-stranded RNA having a sequence identical or similar to the sequence of the target gene. RNAi is the phenomenon in which when a double-stranded RNA having a sequence identical or similar to that of the target gene is introduced into a cell, the expressions of both the inserted exogenous gene and target endogenous gene are suppressed. The double-stranded RNA may be formed from two separate complementary RNAs or may be a single RNA with internally complementary sequences that form a double-stranded RNA. Although complete details of the mechanism of RNAi are still unknown, it is considered that the introduced double-stranded RNA is initially cleaved into small fragments, which then serve as indexes of the target gene in some manner, thereby degrading the target gene. RNAi is also known to be effective in plants (*see, e.g.,* Chuang, C. F. & Meyerowitz, E. M., Proc. Natl. Acad. Sci. USA 97: 4985 (2000); Waterhouse et al., Proc. Natl. Acad. Sci. USA 95:13959-13964 (1998); Tabara et al. Science 282:430-431 (1998); Matthew, Comp Funct. Genom. 5: 240-244 (2004); Lu, et al., Nucleic Acids Research 32(21):e171 (2004)). For example, to achieve suppression of pathogen DCL expression using RNAi, a gene fragment (e.g., from a DCL gene) in an inverted repeat orientation with a spacer could be expressed in plants to generate double-stranded RNA having the sequence of an mRNA encoding the DCL protein, or a substantially similar sequence thereof (including those engineered not to translate the protein) or fragment thereof, is introduced into a plant or other organism of interest. The resulting plants/organisms can then be screened for a phenotype associated with the target protein and/or by monitoring steady-state RNA levels for transcripts encoding the protein. Although the genes used for RNAi need not be completely identical to the target gene, they may be at least 70%, 80%, 90%, 95% or more identical to the target gene sequence. *See, e.g.,* U.S., Patent Publication No. 2004/0029283 for an example of a non-identical siRNA sequence used to suppress gene expression. The constructs encoding an RNA molecule with a stem-loop structure that is unrelated to the target gene and that is positioned distally to a sequence specific for the gene of interest may also be used to inhibit target gene expression. *See, e.g.,* U.S. Patent Publication No. 2003/0221211. Gene silencing in plants by the expression of small RNA duplexes is also described, e.g., in Lu et al., Nucleic Acids Res. 32(21):e171 (2004).

The RNAi polynucleotides can encompass the full-length target RNA or may correspond to a fragment of the target RNA. In some cases, the fragment will have fewer than 100, 200, 300, 400, 500 600, 700, 800, 900 or 1,000 nucleotides corresponding to the target sequence. In addition, in some aspects of the disclosure, these fragments are at least, *e.g.,* 10, 15, 20, 50, 100, 150, 200, or more nucleotides in length. In some cases, fragments for use in RNAi will be at least substantially similar to regions of a target protein that do not occur in other proteins in the organism or may be selected to have as little similarity to other organism transcripts as possible, *e.g.,* selected by comparison to sequences in analyzing publicly-available sequence databases.

Expression vectors that continually express siRNA in transiently- and stably-transfected cells have been engineered to express small hairpin RNAs, which get processed *in vivo* into siRNAs molecules capable of carrying out gene-specific silencing (Brummelkamp et al., Science 296:550-553 (2002), and Paddison, et al., Genes & Dev. 16:948-958 (2002)). Post-transcriptional gene silencing by double-stranded RNA is discussed in further detail by Hammond et al., Nature Rev Gen 2: 110-119 (2001), Fire et al., Nature 391: 806-811 (1998) and Timmons and Fire, Nature 395: 854 (1998).

Yet another way to suppress expression of a gene in a plant is by recombinant expression of a microRNA that suppresses the target gene. Artificial microRNAs are single-stranded RNAs (e.g., between 18-25 mers, generally 21 mers), that are not normally found in plants and that are processed from endogenous miRNA precursors. Their sequences are designed according to the determinants of plant miRNA target selection, such that the artificial microRNA specifically silences its intended target gene(s) and are generally described in Schwab et al, The Plant Cell 18:1121-1133 (2006) as well as the internet-based methods of designing such microRNAs as described therein. *See also,* US Patent Publication No. 2008/0313773.

Another way to suppress expression of a gene in a plant is by application of a dsRNA to a surface of a plant or part of a plant (e.g., onto a leaf, flower, fruit, or vegetable), for example by spraying the dsRNA onto the surface or brushing the dsRNA onto the surface. Methods of applying dsRNA onto external plant parts are described, for example, in WO 2013/02560 and in Gan et al., Plant Cell Reports 29:1261-1268 (2010).

In some aspects of the disclosure, antisense sequences such as dsRNA or sRNA can be synthesized *in planta* and extracted from the plant for subsequent use on a target plant. As a non-limiting example, constructs for producing one or more dsRNA or sRNA sequences of interest can be transiently introduced into a plant (e.g., *N. benthamiana*)*,* for example by infiltration with *Agrobacterium.* The dsRNA or sRNA sequences are produced by the plant and then RNA is extracted from one or more tissues of the plant in order to extract the dsRNA or sRNA sequences of interest. An exemplary method of expressing and extracting antisense sequences from *N. benthamiana* is described in the Examples section below.

### III. Methods of Making Plants Having Increased Pathogen Resistance

In another aspect of the disclosure, methods of making plants having increased pathogen resistance are provided. In some aspects of the disclosure, the method comprises:
introducing into a plant a heterologous expression cassette comprising a promoter operably linked to a polynucleotide that inhibits fungal expression of a pathogen DCL gene; and
selecting a plant comprising the expression cassette.

In some aspects of the disclosure, the method further comprises introducing into the plant a second heterologous expression cassette comprising a second promoter operably linked to a second polynucleotide that inhibits fungal expression of a second pathogen DCL gene; and selecting a plant comprising the second expression cassette.

In some aspects of the disclosure, the polynucleotide that inhibits fungal expression of the pathogen DCL gene is described herein (*e.g.,* in Section II above, e.g., an antisense polynucleotide such as a hairpin RNA or microRNA precursor). For example, in some aspects of the disclosure, the polynucleotide inhibits the expression of one, two, three, four or more *Botrytis* or *Verticillium* DCL genes. In some aspects of the disclosure, the polynucleotide inhibits the expression of one, two, three, four or more of SEQ ID NO:1, SEQ ID NO:3, SEQ ID NO:5, SEQ ID NO:7, SEQ ID NO:9, SEQ ID NO:10, SEQ ID NO:11, SEQ ID NO:12, SEQ ID NO:28, SEQ ID NO:29, SEQ ID NO:30, or SEQ ID NO:31.

In some embodiments, a plant into which the expression cassette(s) has been introduced has increased pathogen resistance relative to a control plant lacking the expression cassette(s). In some embodiments, a plant into which the expression cassette has been introduced has enhanced resistance to a fungal pathogen (*e.g., Botyritis* or *Verticillium*) relative to a control plant lacking the expression cassette.

In some embodiments, the promoter is heterologous to the polynucleotide. In some embodiments, the polynucleotide encoding the sRNA-resistant target is operably linked to an inducible promoter. In some embodiments, the promoter is pathogen inducible (*e.g*., a *Botrytis* inducible promoter). In some embodiments, the promoter is stress inducible (*e.g*., an abiotic stress inducible promoter).

In some aspects of the disclosure, the method comprises:
contacting a plurality of plants with a construct comprising a promoter operably linked to a polynucleotide that inhibits fungal expression of a pathogen DCL gene or pathogen LTR region, wherein the plant has increased resistance to a pathogen compared to a control plant that has not been contacted with the construct.

In some embodiments, the method further comprises selecting a plant having increased pathogen resistance.

In some aspects of the disclosure, the method comprises:
contacting a plant or a part of a plant with a double-stranded RNA, a small RNA duplex, or a small RNA (sRNA) that targets a pathogen DCL gene or pathogen LTR region, wherein the plant or part of the plant has increased resistance to the pathogen compared to a control plant that has not been contacted with the double-stranded RNA or small RNA duplex.

In some aspects of the disclosure, the double-stranded RNA or small RNA duplex (*e.g.,* siRNA) or sRNA targets *Botrytis* DCLs or *Verticillium* DCLs. In some aspects of the disclosure, the double-stranded RNA or small RNA duplex or sRNA targets any of SEQ ID NO:1, SEQ ID NO:3, SEQ ID NO:5, or SEQ ID NO:7 or a fragment thereof. In some aspects of the disclosure, the double-stranded RNA is an siRNA. In some aspects of the disclosure, the siRNA comprises a sequence that is identical to any of SEQ ID NO:1, SEQ ID NO:3, SEQ ID NO:5, SEQ ID NO:7, SEQ ID NO:9, SEQ ID NO:10, SEQ ID NO:11, or SEQ ID NO:12, or a fragment thereof (e.g., a fragment of at least 15, 16, 17, 18, 19, 20, 21, 22, 23, 24, 25, 26, 27, 28, 29, 30, or more contiguous nucleotides) or a complement thereof.

In some aspects of the disclosure, the method comprises contacting the plant or the part of the plant with two, three, four, five, or more double-stranded RNAs or small RNA duplexes (*e.g*., siRNAs) or sRNAs for targeting two, three, four, five, or more pathogen DCL genes or pathogen LTR regions from one, two, three or more different pathogens. As a non-limiting example, in some aspects of the disclosure, the plant is contacted with a double-stranded RNA or small RNA duplex (*e.g.,* siRNA) or sRNA that targets *Botrytis* DCL1 and a double-stranded RNA or small RNA duplex (e.g., siRNA) or sRNA that targets *Botrytis* DCL2. As another non-limiting example, in some aspects of the disclosure, the plant is contacted with a double-stranded RNA or small RNA duplex (*e.g*., siRNA) or sRNA that targets *Verticillium* DCL1 and a double-stranded RNA or small RNA duplex (e.g., siRNA) or sRNA that targets *Verticillium* DCL2. As yet another non-limiting example, in some aspects of the disclosure, the plant is contacted with a double-stranded RNA or small RNA duplex (*e.g.,* siRNA) or sRNA that targets one or more DCLs of Botrytis (e.g., *Botrytis* DCL1 and/or *Botrytis* DCL2) and with a double-stranded RNA or small RNA duplex (*e.g.,* siRNA) or sRNA that targets one or more DCLs of *Verticillium* (e.g., *Verticillium* DCL1 and *Verticillium* DCL2).

In some embodiments, the double-stranded RNA or small RNA duplex (*e.g.,* siRNA) or sRNA is sprayed or brushed onto the plant or part of the plant (*e.g.,* onto a leaf, a fruit, vegetable, or flower). In some embodiments, the double-stranded RNA or small RNA duplex (*e.g*., siRNA) or sRNA is contacted to (e.g., sprayed or brushed onto) a part of a plant that has been removed from the plant. As a non-limiting example, the double-stranded RNA or small RNA duplex (*e.g*., siRNA) or sRNA can be contacted to (e.g., sprayed or brushed onto) a fruit, vegetable, or flower that has already been cut from a plant. In some embodiments, the double-stranded RNA or small RNA duplex (*e.g*., siRNA) or sRNA is contacted to (e.g., sprayed or brushed onto) a part of a plant (e.g., a fruit, vegetable, or flower) while the part is still attached to the plant.

### IV. Polynucleotides and Recombinant Expression Vectors

The isolation of polynucleotides of the disclosure may be accomplished by a number of techniques. For instance, oligonucleotide probes based on the sequences disclosed here can be used to identify the desired polynucleotide in a cDNA or genomic DNA library from a desired plant species. To construct genomic libraries, large segments of genomic DNA are generated by random fragmentation, e.g. using restriction endonucleases, and are ligated with vector DNA to form concatemers that can be packaged into the appropriate vector. Alternatively, cDNA libraries from plants or plant parts (e.g., flowers) may be constructed.

The cDNA or genomic library can then be screened using a probe based upon a sequence disclosed here. Probes may be used to hybridize with genomic DNA or cDNA sequences to isolate homologous genes in the same or different plant species. Alternatively, antibodies raised against a polypeptide can be used to screen an mRNA expression library.

Alternatively, the nucleic acids of interest can be amplified from nucleic acid samples using amplification techniques. For instance, polymerase chain reaction (PCR) technology to amplify the sequences of the genes directly from mRNA, from cDNA, from genomic DNA, from genomic libraries or cDNA libraries. PCR and other *in vitro* amplification methods may also be useful, for example, to clone nucleic acid sequences that code for proteins to be expressed, to make nucleic acids to use as probes for detecting the presence of the desired mRNA in samples, for nucleic acid sequencing, or for other purposes. For a general overview of PCR see PCR Protocols: A Guide to Methods and Applications. (Innis, M, Gelfand, D., Sninsky, J. and White, T., eds.), Academic Press, San Diego (1990).

Polynucleotides can also be synthesized by well-known techniques as described in the technical literature. *See,* e.g., Carruthers et al., Cold Spring Harbor Symp. Quant. Biol. 47:411-418 (1982), and Adams et al., J. Am. Chem. Soc. 105:661 (1983). Double stranded DNA fragments may then be obtained either by synthesizing the complementary strand and annealing the strands together under appropriate conditions, or by adding the complementary strand using DNA polymerase with an appropriate primer sequence.

Once a polynucleotide sequence that inhibits expression of a fungal dicer-like (DCL) gene or LTR region, or that is complementary to a fungal pathogen DCL gene or LTR region or a fragment thereof, is obtained, it can be used to prepare an expression cassette for expression in a plant. In some embodiments, expression of the polynucleotide is directed by a heterologous promoter.

Any of a number of means well known in the art can be used to drive expression of the polynucleotide sequence of interest in plants. Any organ can be targeted, such as shoot vegetative organs/structures (*e.g*. leaves, stems and tubers), roots, flowers and floral organs/structures (*e.g*. bracts, sepals, petals, stamens, carpels, anthers and ovules), seed (including embryo, endosperm, and seed coat) and fruit. Alternatively, expression can be conditioned to only occur under certain conditions (*e.g*., using an inducible promoter).

For example, a plant promoter fragment may be employed to direct expression of the polynucleotide sequence of interest in all tissues of a regenerated plant. Such promoters are referred to herein as "constitutive" promoters and are active under most environmental conditions and states of development or cell differentiation. Examples of constitutive promoters include the cauliflower mosaic virus (CaMV) 35S transcription initiation region, the 1'- or 2'- promoter derived from T-DNA of *Agrobacterium tumafaciens,* and other transcription initiation regions from various plant genes known to those of skill.

Alternatively, the plant promoter may direct expression of the polynucleotide sequence of interest in a specific tissue (tissue-specific promoters) or may be otherwise under more precise environmental control (inducible promoters). Examples of tissue-specific promoters under developmental control include promoters that initiate transcription only in certain tissues, such as leaves or guard cells (including but not limited to those described in WO/2005/085449; U.S. Patent No. 6,653,535; Li et al., Sci China C Life Sci. 2005 Apr;48(2):181-6; Husebye, et al., Plant Physiol, April 2002, Vol. 128, pp. 1180-1188; and Plesch, et al., Gene, Volume 249, Number 1, 16 May 2000 , pp. 83-89(7)). Examples of environmental conditions that may affect transcription by inducible promoters include the presence of a pathogen, anaerobic conditions, elevated temperature, or the presence of light.

In some embodiments, the promoter is a constitutive promoter. In some embodiments, the promoter is an inducible promoter. In some embodiments, the promoter is stress inducible (*e.g*., inducible by abiotic stress). In some embodiments, the promoter is pathogen inducible. In some embodiments, the promoter is induced upon infection by *Botyrtis.* Non-limiting examples of pathogen inducible promoters include Botyritis-Induced Kinase 1 (BIK1) and the plant defensing gene PDF1.2. *See, e.g.,* Penninckx et al., Plant Cell 10:2103-2113 (1998); *see also* Veronese et al., Plant Cell 18:257-273 (2006).

In some embodiments, a polyadenylation region at the 3'-end of the coding region can be included. The polyadenylation region can be derived from a NH3 gene, from a variety of other plant genes, or from T-DNA.

The vector comprising the sequences will typically comprise a marker gene that confers a selectable phenotype on plant cells. For example, the marker may encode biocide resistance, particularly antibiotic resistance, such as resistance to kanamycin, G418, bleomycin, hygromycin, or herbicide resistance, such as resistance to chlorosluforon or Basta.

### V. Production of Transgenic Plants

As detailed herein, aspects of the present disclosure provide for transgenic plants comprising recombinant expression cassettes for expressing a polynucleotide sequence as described herein (e.g., a polynucleotide that inhibits expression of a fungal pathogen dicer-like (DCL) gene or a polynucleotide that inhibits expression of a fungal pathogen LTR region, such as a polynucleotide that expresses a hairpin RNA or microRNA precursor). In some aspects of the disclosure, a transgenic plant is generated that contains a complete or partial sequence of a polynucleotide that is derived from a species other than the species of the transgenic plant. It should be recognized that transgenic plants encompass the plant or plant cell in which the expression cassette is introduced as well as progeny of such plants or plant cells that contain the expression cassette, including the progeny that have the expression cassette stably integrated in a chromosome.

In some aspects of the disclosure, the transgenic plants comprising recombinant expression cassettes for expressing a polynucleotide sequence as described herein have increased or enhanced pathogen resistance compared to a plant lacking the recombinant expression cassette, wherein the transgenic plants comprising recombinant expression cassettes for expressing the polynucleotide sequence have about the same growth as a plant lacking the recombinant expression cassette. Methods for determining increased pathogen resistance are described, e.g., in Section VI below.

A recombinant expression vector as described herein may be introduced into the genome of the desired plant host by a variety of conventional techniques. For example, the DNA construct may be introduced directly into the genomic DNA of the plant cell using techniques such as electroporation and microinjection of plant cell protoplasts, or the DNA construct can be introduced directly to plant tissue using ballistic methods, such as DNA particle bombardment. Alternatively, the DNA construct may be combined with suitable T-DNA flanking regions and introduced into a conventional *Agrobacterium tumefaciens* host vector. The virulence functions of the *Agrobacterium tumefaciens* host will direct the insertion of the construct and adjacent marker into the plant cell DNA when the cell is infected by the bacteria. While transient expression of the polynucleotide sequence of interest is encompassed by the invention, generally expression of construction of the invention will be from insertion of expression cassettes into the plant genome, *e.g.,* such that at least some plant offspring also contain the integrated expression cassette.

Microinjection techniques are also useful for this purpose. These techniques are well known in the art and thoroughly described in the literature. The introduction of DNA constructs using polyethylene glycol precipitation is described in Paszkowski et al. EMBO J. 3:2717-2722 (1984). Electroporation techniques are described in Fromm et al. Proc. Natl. Acad. Sci. USA 82:5824 (1985). Ballistic transformation techniques are described in Klein et al. Nature 327:70-73 (1987).

*Agrobacterium tumefaciens-mediated* transformation techniques, including disarming and use of binary vectors, are well described in the scientific literature. See, for example, Horsch et al. Science 233:496-498 (1984), and Fraley et al. Proc. Natl. Acad. Sci. USA 80:4803 (1983).

Transformed plant cells derived by any of the above transformation techniques can be cultured to regenerate a whole plant that possesses the transformed genotype and thus the desired phenotype such as enhanced pathogen resistance. Such regeneration techniques rely on manipulation of certain phytohormones in a tissue culture growth medium, typically relying on a biocide and/or herbicide marker which has been introduced together with the desired nucleotide sequences. Plant regeneration from cultured protoplasts is described in Evans et al., Protoplasts Isolation and Culture, Handbook of Plant Cell Culture, pp. 124-176, MacMillilan Publishing Company, New York, 1983; and Binding, Regeneration of Plants, Plant Protoplasts, pp. 21-73, CRC Press, Boca Raton, 1985. Regeneration can also be obtained from plant callus, explants, organs, or parts thereof. Such regeneration techniques are described generally in Klee et al. Ann. Rev. of Plant Phys. 38:467-486 (1987).

### VI. Selecting for Plants with Increased Pathogen Resistance

The expression cassettes and antisense constructs (e.g., double-stranded RNA or small RNA duplexes, e.g., siRNAs) of the disclosure can be used to confer increased or enhanced pathogen resistance on essentially any plant or part of a plant. Thus, the invention has use over a broad range of plants, including but not limited to species from the genera *Allium, Asparagus, Atropa, Avena, Brassica, Citrus, Citrullus, Capsicum, Cucumis,* *Cucurbita, Daucus, Fragaria, Glycine, Gossypium, Helianthus, Heterocallis, Hordeum, Hyoscyamus, Lactuca, Linum, Lolium, Lycopersicon, Malus, Manihot, Majorana, Medicago, Nicotiana, Oryza, Panieum, Pannesetum, Persea, Pisum, Pyrus, Prunus, Raphanus, Rosa, Secale, Senecio, Sinapis, Solanum, Sorghum, Trigonella, Triticum, Vitis, Vigna,* and *Zea.* In some embodiments, the plant is a vining plant, e.g., a species from the genus *Vitis.* In some embodiments, the plant is an ornamental plant, e.g., a species from the genus *Rosa.* In some embodiments, the plant is a vegetable- or fruit-producing plant, e.g., a tomato plant or a strawberry plant. In some embodiments, the plant is a monocot. In some embodiments, the plant is a dicot.

Plants (or parts of plants) with increased pathogen resistance can be selected in many ways. One of ordinary skill in the art will recognize that the following methods are but a few of the possibilities. One method of selecting plants or parts of plants (e.g., fruits, vegetables, leaves, and flowers) with increased pathogen resistance is to determine resistance of a plant to a specific plant pathogen. Possible pathogens include, but are not limited to, viruses, bacteria, nematodes, fungi or insects (see, *e.g.,* Agrios, Plant Pathology (Academic Press, San Diego, CA) (1988)). One of skill in the art will recognize that resistance responses of plants vary depending on many factors, including what pathogen, compound, or plant is used. Generally, increased resistance is measured by the reduction or elimination of disease symptoms (*e.g*., reduction in the number or size of lesions or reduction in the amount of fungal biomass on the plant or a part of the plant) when compared to a control plant. In some embodiments, resistance is increased when the number or sizes of lesions or amount of fungal biomass on the plant or on a part of the plant is decreased by at least about 10%, 20%, 30%, 40%, 50%, 60%, 70%, 80%, 90% or more relative to a control (e.g., relative to a plant in which a heterologous polynucleotide targeting a fungal pathogen DCL or LTR has not been expressed).

In some cases, increased resistance can also be measured by the production of the hypersensitive response (HR) of the plant (see, *e.g.,* Staskawicz et al. (1995) Science 268(5211): 661-7). Plants with increased pathogen resistance can produce an enhanced hypersensitive response relative to control plants.

Increased pathogen resistance can also be determined by measuring the increased expression of a gene operably linked a defense related promoter. Measurement of such expression can be measured by quantifying the accumulation of RNA or subsequent protein product (*e.g*., using Northern or Western blot techniques, respectively (see, *e.g*., Sambrook *et al.* and Ausubel *et al.*)*.*

### VII. Examples

### Example 1: Targeting DCL Genes to Attenuate Fungal Virulence

Eukaryotic small RNAs (sRNAs) are short regulatory noncoding RNAs that induce silencing of target genes at transcriptional and posttranscriptional levels. The endoribonuclease Dicer or Dicer-like proteins (DCLs) process double-stranded RNAs (dsRNAs) or RNAs with hairpin structures, giving rise to mostly 20-30-nt long sRNAs, which are loaded into Argonaute (AGO) proteins to induce gene silencing of their complementary targets by guiding mRNA cleaving or degradation, translational inhibition, DNA methylation, and histone modification. The role of sRNAs in plant-pathogen interactions, including the role of noncoding sRNAs from bacterial and eukaryotic plant pathogens in pathogenicity, is described in Weiberg et al., Annu. Rev. Phytopathol. 2014, 52:22.1-22.22.

sRNA effectors, like those found in *B. cinerea,* are transcribed from transposable elements (TEs) and suppress host immune-related genes. Host plant resistance genes are often clustered in genomic loci enriched with TEs. Similarly, protein effector genes are often found in clusters and interspersed with TEs. *See, e.g.,* Weiberg at Figure 2.

Because most of the *Botrytis* small RNA effectors are generated from LTR regions, there are multiple copies for each LTR, which makes Bc-sRNA knockouts impractical if not impossible. Therefore, to solve this problem, *Botrytis* DCL knockout mutants were generated.

As shown in Figure 1, the *B. cinerea* genome has two DCLs (dcl-1 and dcl-2). Single- and double-mutant (*dcl1, dcl2,* and *dcl1 dcl2* mutant) strains were generated. As shown in Figure 2, all of the *B. cinerea dcl1, dcl2,* and *dcl1 dcl2* mutant strains showed growth retardation and delayed development of conidiospores (Fig. 2A), but only the double mutant strain (*dcl1 dcl2*) could not produce Bc-sRNA effectors (Fig. 2B).

*B. cinerea* DCLs are essential for the pathogenicity of *B. cinerea.* As shown in Figure 3, *dcl1 dcl 2* double mutants, but not *dcl1* or *dcl2* single mutants, produced much weaker disease symptoms than did the wild type in both *Arabidopsis* and *S. lycopersicum,* and largely attenuated the virulence *of B. cinerea.* Similarly, Figure 4 shows that *B. cinerea dcl1 dcl 2* double mutants are much less virulent on fruit, vegetables, and flowers.

A genome-wide comparative sRNA analysis on a *dcl1 dcl2* mutant strain and wild-type revealed that *Botrytis* DCLs are responsible for generating LTR-derived sRNAs, many of which are sRNA effectors. *See,* Fig. 5A-B, Fig. 6, and Fig. 7A-D.

*B. cinerea* delivers small RNAs into host cells (e.g., plant cells) to suppress host immune systems. *See, e.g.,* Weiberg at Figure 2. Another fungal plant pathogen, *Verticillium dahliae,* also depends on AGO1 function for its pathogenicity. See, e.g., Ellendorf et al., J. Exp Bot 2009; 60:591-602. This suggests that *Verticillium* is likely to have a similar RNAi virulence mechanism as *B. cinerea.* Because *Verticillium* infects the plants through roots, we used root culture to get more material for immunoprecipitation of Arabidopsis AGO1-associated small RNA. sRNAs that are associated with Arabidopsis AGO1 were pulled down and subjected to deep sequencing. We found that some of the *Verticillium* small RNAs were highly enriched after infection. We found that 41 Vd-sRNAs had *A. thaliana* (At) targets (using 100 rpm and 10 fold enrichment as a cutoff). Table 1 below shows examples of infection-enriched *Verticillium* small RNAs that have potential host targets.

These results suggest that RNAi constructs, which target fungal Dicer-like protein genes to attenuate fungal virulence, can be expressed in host plants (including, but not limited to, tomato, grape and other commercially important crops). Alternatively, the RNAi constructs can be contacted to the plant, such as by being sprayed on a surface of the plant (e.g., onto the surface of a leaf) for promoting fungal resistance. As shown in Figure 9, host induced gene silencing (HIGS) against *B. cinerea dcl1 dcl2* (drop inoculation) increased plant tolerance against *B. cinerea.* Additionally, as shown in Figure 10, virus induced gene silencing (VIGS) against *B. cinerea dcl1dcl2* (spray inoculation) increased plant tolerance against *B. cinerea.*

**Table 1. Infection-enriched Verticillium small RNAs have potential host targets**

| ***Verticillium* Small RNA Sequences** | **Reads in Infected** | **Reads in control** | **Enriched fold** | **Score** | **Targeting sequences** | **Target Genes** |
|---|---|---|---|---|---|---|
| TAAGGATCGGAGG TTCGAATCAGTT | 724.7 | 0.0 | U/A | 3 | 3'-CTAAGCTTGGAGGCTAGG-5' ;;;;x;;;;;;;x;;;;; 5'-GATTGGAACCTCAGATCC-3' 3'-CTAAGCTTGGAGGCTAGG-5' ;x;;x;;;;;;;:;;;;; 5'-GCTTGGAACCTCTGATCC-3' 3'-CTAAGCTTGGAGGCTAGG-5' ;;x;;x;;;;;:;;;;;: 5'-GACTCTAACCTTCGATCT-3' 3'-ACTAAGCTTGGAGGCTAGG-5' ;;:;;x;;;:;;;:;:;;; 5'-TGGTTGGAATCTCTGGTCC-3' 3'-ACTAAGCTTGGAGGCTAGG-5' ;;:;;x;;;:;;;:;:;;; 5'-TGGTTGGAATCTCTGGTCC-3' | Cysteine/Histidine-rich C1 domain family protein |
| | | | | 3 | | Cysteine/Histidine-rich C1 domain family protein |
| | | | | 3.5 | | WRKY DNA-binding protein 2 |
| | | | | 4.5 | | Disease resistance protein (TIR-NBS-LRR class) |
| | | | | 4.5 | | Disease resistance protein (TIR-NBS-LRR class) family |
| GCGAGGTGAGAGG ACGACCAGCCAAG | 10116.0 | 5.2 | 1938 | 3 | 3'-GACCAGCAGGAGAGTGGA-5' ;;;;;;x;;;;;;;;;;x 5'-CTGGTCCTCCTCTCACCA-3' | mitogen-activated protein kinase kinase kinase 5 |
| CATCTGAGAGGAC GTCCCGCCATGGC | 5785.1 | 4.6 | 1266 | 4.5 | 3'-CCGCCCTGCAGGAGAGTC-5' ;;:;;x;;x;;:;;;;;; 5'-GGTGGAACTTCTTCTCAG-3' | Leucine-rich repeat protein kinase family protein |
| | 2717.7 | 4.6 | 595 | 4.5 | 3'-GAGTTCGACCAGTAAAGGG-5' :;;;;x;;;;;x;;;;;:; 5'-TTCAACCTGGTGATTTCTC-3' 3'-GAGTTCGACCAGTAAAGGG-5' :;;;;x;;;;;x;;;;;:; 5'-TTCAACCTGGTGATTTCTC-3' 3'-GAGTTCGACCAGTAAAGGG-5' :;;;;x;;;;;x;;;;;:; 5'-TTCAACCTGGTGATTTCTC-3' 3'-GAGTTCGACCAGTAAAGGG-5' x;;;:x;;;;;;;;;;;;x 5'-GTCAGACTGGTCATTTCCA-3' | Cysteine/Histidine-rich C1 domain family protein |
| GTCCGGGAAATGA CCAGCTTGAGCAG | | | | 4.5 | | Cysteine/Histidine-rich C1 domain family protein |
| | | | | 4.5 | | Cysteine/Histidine-rich C1 domain family protein |
| | | | | 4.25 | | AGD2-like defense response protein 1 |
| ATGTCGATGGTCGG ATGTATCCTTTTCT | 3164.0 | 46.3 | 68 | 4.5 | 3'-TTTTCCTATGTAGGCTGGTAGC-5' ;;;;x;;;:;;;;;;;:;x;;; 5'-AAAAAGATGCATCCGATCTTCG-3' 3'-TTTTCCTATGTAGGCTGGTAGC-5' x;;;x;;;;:;;:;;;;;;;;x 5'-TAAAAGATATATTCGACCATCC-3' | Ankyrin repeat family protein |
| | | | | 4.5 | | Cysteine/Histidine-rich C1 domain family protein |

### Example 2: Increasing fungal resistance or tolerance in fruits and vegetables by in vitro silencing of fungal pathogen DCLs

Enhanced fungal resistance was observed when fruits, leaves, and vegetables were treated with sRNAs targeting fungal pathogen DCL genes. The following protocol was used for treating fruits, leaves, or vegetables with RNAs extracted from *N. Benthamiana* expressing Bc-DCL-targeting sRNAs.

### Protocol

**1. Plasmid construction.** *B. cinerea* DCL1 (BcDCL1) RNAi fragment was amplified by using *B. cinerea* cDNAs as template, forward primer BcDCL1RNAi-F: 5'-GGGGACAAGTTTGTACAAAAAAGCAGGCTTGCGGAAGAACTTGAAGGTTTGCTA CA-3' and reverse primer BcDCL1RNAi-R: 5'-GTCCAGATCTGGTCAACACACCAAG-3', 252bp. BcDCL2 RNAi fragment was amplified by the forward primer BcDCL2RNAi-F: 5'-CTTGGTGTGTTGACCAGATCTGGACGGATGCCATTTGCTGCACGC-3' and reverse primer BcDCL2RNAi-R: 5'-GGGGACCACTTTGTACAAGAAAGCTGGGTACTCTTGAGTACTTTCGC CAGCTCAC-3', 238bp. These two RNAi fragments were integrated together by overlapping PCR as BcDCL-RNAi, which was cloned into pDONR207 by BP reactions (Life Technologies), and finally to destination vector pHELLSGATE 8.0 by LR reactions (Life Technologies) as pHELLSGATE-BcDCL-RNAi. This vector as well as a negative control pHELLSGATE 8.0 empty vector were transformed into *A. tumefaciens* GV3101 strain.
**2. Generate DCL-targeting sRNAs in *N. benthamiana.*** The *A. tumefaciens* GV3101 strain carrying pHELLSGATE-BcDCL-RNAi (RNAi strain) and pHELLSGATE 8.0 empty vector (EV strain) were cultured in liquid LB with antibiotics (100 µg/µl Spectinomycin, 50 µg/µl Gentamycin and 50 µg/µl Rifampicin) overnight at 28°C shaker. Both EV and RNAi *A. tumefaciens* cultures were centrifuged at 4000rpm for 15min at room temperature, and resuspended bacterial pellets with 5mL infiltration buffer (10mM MgCl2, 10mM MES, and 0.2 mM Acetosyringone). The OD600 of both EV and RNAi strain solutions were adjusted to 1.0, and kept on room temperature for 4 hours. Both solutions were diluted to OD600=0.5 by infiltration buffer right before infiltration. Then, the EV and RNAi strain solutions were used to infiltrate the 4 weeks old leaves of *N. benthamiana,* and allowed 2 days infection to over express DCL-targeting sRNAs in RNAi strain treated tissues.
**3**. **Extract the sRNAs from** ***N. benthamiana.*** The infiltrated *N. benthamiana* leaf tissues were harvested separately based on the strains (EV or RNAi) that infiltrated, and freeze them immediately in liquid nitrogen. 10g of each tissue were ground into fine powder in the liquid nitrogen with mortars and pestles, and total RNAs were extracted by using TRIzol Reagent (Life Technologies). Resolved the RNA pellets in 500µl DEPC treated H₂O, and the concentration of both EV and RNAi total RNAs was examined by nanodrop. The final concentration of these two RNAs samples were both adjusted to 50ng/µl for further use. The sRNAs targeting Bc-DCLs were only present in the RNAi set, but not in the EV set, which was used as a negative control.
**4. Treat the vegetables or fruits with the RNA extracts.** Two sets of vegetables or fruits under similar conditions, (e.g., similar freshness, maturity, size, shape, etc.) were gently washed and put in a plastic box with a wetted filter paper on the bottom to keep moisture. The first set was evenly treated with RNA extracts from EV strain infiltrated tissues, the other set was evenly treated with RNA extracts from RNAi strain infiltrated tissues. RNA extracts were applied by spray or by drop inoculation.
**5. *B. cinerea* infection.** *B. cinerea* infection was carried out after the spray of RNA extracts. The spores from 10 days old *B. cinerea* grown on Malt Extract Agar medium were eluted in sterile H₂O, and the fungal mycelia were filtered from the spores by nylon cloth. *B*. *cinerea* spore concentration was calculated by hemocytometer and diluted in B5 medium (10mM Sucrose, 10mM KH₂PO₄, Tween-20 0.025%). 15 µl of *Botrytis* spore solution (2 × 10⁻⁵) was sprayed on the surface of the sprayed fruits or vegetable, and allowed 2-4 days *B*. *cinerea* inoculation. Different vegetables or fruits took different time to obtain obvious disease symptoms.

Alternatively, a "mix" method was used for administering a 1:1 mixture of total RNAs as described above and Botrytis spore solution (4 ×10⁻⁵). 15 ul of the mixed solution was directly dropped on the surface of 2 sets of fruits or vegetables under similar conditions.

### Results

As shown in Figure 11, tomato was more resistant against *B. cinerea* when sprayed with RNA containing siRBcDCLs. Additionally, figures 12-14 show that *B. cinerea* was less virulent when mixed with *N. benthamiana* total RNA containing siRBcDCLs and applied to tomato (Figure 12), strawberry (Figure 13), or cucumber (Figure 14).

In another experiment, pretreating tomato leaves and fruits, strawberry fruits, and grape fruits with the total RNA from *N. Benthamiana* infiltrated with pHELLSGATE-BcDCLs and pHELLSGATE-EV by spray for 24 hours, followed by *B. cinerea* drop inoculation on the sprayed area of the fruits, reduced gray mold disease symptoms caused by *B. cinerea. See,* Figure 15A-B.

In yet another experiment, Spraying tomato leaves and fruits with in vitro transcribed dsRNA against BcDCLs reduced gray mold disease caused by *B. cinerea* on tomato and strawberry. Tomato leaves and fruits and strawberry fruits were pre-treated with water or *in vitro* transcribed long dsRNA against BcDCLs for 24 hours, followed by *B. cinerea* drop inoculation right on the pretreated area of the leaves or fruits. *See,* Figure 16.

### Example 3: Increasing plant tolerance to multiple pathogens

Host induced gene silencing (HIGS) was used to silence the dicer-like protein (DCL) genes of two fungal pathogens in plants. An RNAi approach for targeting two DCLs from *Botrytis* and two DCLs from *Verticillium* was used in Arabidopsis, resulting in the generation of "HIGS-4DCLs" lines. The HIGS-4DCLs lines, as well as wild type Arabidopsis plants, were infected with *B. cinerea* by drop inoculation on the leaves and were infected with *V. dahilae* by root inoculation. Three weeks after infection, the HIGS-4DCLs lines exhibited increased tolerance to both pathogens relative to the wild-type plants. *See,* Figure 17. Thus, the gene targeting approaches described herein can be utilized for targeting multiple pathogens at the same time.

### Example 4: Dicer-targeting small RNA-mediatedplant protection against Botrytis cinerea and other pathogens that utilize small RNA effectors

Eukaryotic pathogens, such as fungi and oomycetes, cause billions of dollars crop losses worldwide annually. *B. cinerea* poses a serious threat to over 200 plant species, including nearly all vegetables and fruits as well as many flowers, in their pre- and post-harvest stages by causing grey mold disease. Williamson et al., Mol Plant Pathol 8, 561-580 (2007); van Kan, Trends in Plant Science 11:247-253 (2006)). Current disease control is mainly achieved by fungicide application, which is costly and environmentally hazardous.. HIGS has worked effectively against a variety of fungal and oomycete pathogens, such as *Blumeria graminis* (Nowara et al., Plant Cell 22, 3130-3141 (2010); Pliego et al., Molecular Plant Microbe Interactions 26, 633-642 (2013); Whigham et al., Molecular Plant Microbe Interactions 28, 968-983 (2015)), *Puccinia tritici* (Panwar et al, Plant Molecular Biology 81, 595-608 (2013)), *Fusarium* spp. (Koch et al., PNAS 110, 19324-19329 (2013); Ghag et al., Plant Biotechnology Journal 12:541-553 (2014); Cheng et al., Plant Biotechnology Journal, doi:10.1111/pbi. 12352 (2015)), *Phytophthora infestans* (Jahan et al., Journal of Experimental Botany 66:2785-2794 (2015); Sanju et al., Functional & Integrative Genomics 15:697-706 (2015), and *Phytophthora capsici* (Vega-Arreguin et al., Molecular Plant Microbe Interactions 27, 770-780 (2014). However, successful HIGS largely depends on plant transformation technology that is not available for many crops, and furthermore, the safety of genetically modified organisms (GMOs) is still a major concern to the public.

### Results

As discussed above in Example 1, only the *dcl1 dcl2* double mutant of *B. cinerea,* but not the *dcl1* or *dcl2* single mutants, failed to produce Bc-sRNA effectors, suggesting that these Bc-sRNA effectors are dependent on both *Bc-DCL1* and *Bc-DCL2.* To fully evaluate the contribution of Bc-DCL-dependent Bc-sRNA effectors to *B. cinerea* pathogenicity on a wide range of economically important crops, various fruits (tomato-*Solanum lycopersicum* 'Roma', strawberry-Fragaria × ananassa, and grape*-Vitis labrusca* 'Concord'), vegetables (iceberg lettuce*-Lactuca sativa* and onion*-Allium cepa* L.), and flower petals (rose-Rosa hybrida L.) were inoculated with the *dcl1 dcl2* double mutant. The *dcl2* single mutant, and wild-type (WT) strains were used as controls. The *dcl1 dcl2* double mutant showed much weaker pathogenicity and produced significantly smaller lesions than the WT strain on all the samples (Fig. 18A-B, P < 0.01), whereas the *dcl2* single mutant generated smaller lesions than the WT strain on tomato, but it created comparable lesions to the WT strain on strawberry, grape, lettuce, onion, and rose petal (Fig. 18A-B). These results indicate that sRNA effectors play a critical role in *B. cinerea* pathogenicity, because even though *dcl2* strain showed clear reduction of fungal growth, it could still produce Bc-sRNA effectors and cause obvious disease symptoms.

To assess the DCL1 and DCL2-dependent Bc-sRNA population globally, we profiled total sRNAs isolated from the *dcl1 dcl2* double mutant as well as the WT strain. In the WT strain, Bc-sRNAs ranged from 20 to 35 nucleotides (nt) in length (data not shown), with an enrichment of 24-27-nt species. The abundance of 20-27-nt sRNA species was clearly reduced but not completely eliminated in the *dcl1 dcl2* double mutant (data not shown), pointing to the existence of DCL-independent sRNA biogenesis pathways in *B*. *cinerea,* as reported in *Neurospora crassa* (Lee et al., Molecular Cell 38:803-814 (2010); Jin al., Molecular Cell 38:775-777 (2010)). Most strikingly, the sRNAs derived from retrotransposons (ranging from 20-26-nt and peaking at 21-22-nt) were almost completely eliminated in the *dcl1 dcl2* double mutant (data not shown), which indicates that Bc-DCL1/2 are mostly responsible for generating sRNAs from retrotransposons. The sRNAs from intergenic non-coding (IGN) regions (mainly the 21-, 22- and 24-nt sRNAs) and antisense to open reading frames (ORFs-antisense, mainly the 21-22-nt sRNAs) were also largely reduced in *dell dcl2,* although the sRNAs from the sense transcripts of ORFs (ORFs-sense) were not changed significantly. As reported previously (Weiberg et al.) and as discussed in Example 1, the majority of predicted sRNA effectors are from retrotransposon long terminal repeats (LTRs); thus, Bc-DCL1/2 are largely responsible for generating sRNA effectors and contribute significantly to *B. cinerea* pathogenicity. This makes *Bc-DCL1*/*2* ideal targets to test whether sRNA-mediated silencing would be an efficient strategy for controlling gray mold disease.

To test whether host-generated sRNAs can move from plant cells to *B. cinerea* and whether silencing *Bc-DCL1*/*2* would efficiently suppress disease symptoms of *B. cinerea* on plants, we generated transgenic Arabidopsis plants expressing sRNAs that target both *Bc-DCLs.* A DNA fragment containing 252 base-pair (bp) and 238 bp segments from the non-conserved regions of *Bc-DCL1* and *Bc-DCL2,* respectively, was cloned into the pHELLSGATE vector system (Helliwell et al., Methods in Enzymology 392:24-35 (2005)). Two DNA segments were used, one each from Bc-DCL1 and Bc-DCL2, because there is no DNA region outside the conserved domains with sufficient homology between the two DCLs to silence both DCLs. DNA regions in the conserved functional domains were avoided to eliminate any off-target effects on *DCL* genes from other species. These selected *Bc-DCL* DNA regions have only 3.5% to 4.8% sequence identity to Arabidopsis *DCLs,* and indeed, no host *DCL* genes were silenced (data not shown). The hairpin RNA products transcribed from the pHELLSGATE construct in the transgenic plants were processed into sRNAs by plant DCLs to target *Bc-DCL1* and *Bc-DCL2* (Fig. 19A). These plants exhibited much smaller lesions and less fungal growth after *B. cinerea* infection when compared with the WT plants (Fig. 19B-C). Relative expression of *Bc-DCL1* and *Bc-DCL2* was clearly suppressed in *B. cinerea* collected from the transgenic plants compared to those from WT plants (Fig. 19D). These results suggest that *Bc*-*DCL1*/*2*-targeting sRNAs (Bc-DCL1/2-sRNAs) produced in plant cells moved into the fungal cells and efficiently silenced *Bc-DCL1* and *Bc-DCL2,* which led to suppression of fungal virulence and growth, and inhibition of disease.

To determine whether this *Bc-DCL1*/*2*-targeting RNAi strategy could also efficiently control gray mold disease in tomato plants, we introduced the same *Bc-DCL1*/*2* fragment into the tobacco rattle virus (TRV) silencing system (Liu et al., Plant Journal 31:777-786 (2002), which triggered the expression of Bc-DCL1/2-sRNAs in tomato. Three weeks after agro-infiltration, the tomato plants were infected with *B. cinerea* using spray inoculation. The tomato leaves expressing Bc-DCL1/2-sRNAs displayed very mild to almost no disease symptoms at 3 dpi (Fig. 19E-G), whereas the control leaves, which expressed sRNAs targeting a potato late blight resistance gene *RB* that is not present in tomato (Song et al., PNAS 100:9128-9133 (2003), showed very severe water soaked disease lesions (Fig. 19F-G). The growth of *B. cinerea* was also significantly reduced on the leaves expressing *Bc-DCL1*/*2-sRNAs* compared with the control, and the expression of *Bc-DCL1* and *Bc-DCL2* was largely reduced in *B. cinerea* grown on these leaves as well (Fig. 19H). These data further support that sRNAs can move from plant cells to fungal cells, and they can efficiently silence fungal *DCL* genes and suppress pathogen virulence.

*B. cinerea* also infects many other plant species, in addition to Arabidopsis and tomato. Gray mold disease is a very serious problem in post-harvest management, as it destroys millions of fruits, vegetables, and flowers during the packing, transportation, and storage processes each year. Unfortunately, stable transformation and virus-induced gene silencing technologies have not been developed in many host species. Uptake of RNAs from environments, a phenomenon named environmental RNAi, was observed in *C. elegans* and other insects. *See, e.g.,* Winston et al., PNAS 104:10565-10570 (2007); Ivashuta et al., RNA 21:840-850 (2015). Spraying dsRNAs targeting the *actin* gene of potato beetle *Leptinotarsa decemlineata* on potato leaves could inhibit the larva growth (San Miguel et al., Pest Management Science, doi:10.1002/ps.4056 (2015)). Therefore, we attempted to externally apply synthetic Bc-DCL1/2-sRNAs or their double-stranded RNA precursors (Bc-DCL1/2-dsRNAs) on the surface of fruits, vegetables, and flowers, to test whether *B. cinerea* is capable of taking up dsRNAs and/or sRNAs from the environment and inducing silencing of its own genes. The same *Bc-DCL1*/*2* fragment was transcribed *in vitro* from both ends and gave rise to Bc-DCL1/2-dsRNAs. The Bc-DCL1/2-dsRNAs were subjected to RNAse III treatment to generate Bc-DCL1/2-sRNAs *in vitro.* The Bc-DCL1/2-sRNAs and the precursor dsRNAs (20 ng/µl) were externally applied on fruits (tomato, strawberry, and grape), vegetables (lettuce and onion), and flower petals (rose), and followed with *B. cinerea* infection. All the plants treated with Bc-DCL1/2-sRNAs and -dsRNAs developed much weaker disease symptoms (Fig. 20A-B) and supported significantly less growth of *B. cinerea* (Fig. 20B) compared to the YFP-dsRNAs-treated controls, suggesting that externally applied Bc-DCL1/2-sRNAs and -dsRNAs can inhibit pathogen virulence, probably via the suppression of both fungal growth and Bc-sRNA effector biogenesis. Bc-DCL1/2-sRNAs had slightly stronger effects than Bc-DCL1/2-dsRNAs.

Although external treatment of Bc-DCL1/2-sRNAs and the precursor Bc-DCL1/2-dsRNAs can inhibit fungal disease, *in vitro* RNA synthesis is too costly. In order to obtain a large amount of Bc-DCL1/2-sRNAs and -dsRNAs at a much lower cost, we transiently expressed pHELLSGATE-Bc-DCL1/2 vector in *N. benthamiana,* which yielded a large amount of Bc-DCL1/2-sRNAs and -dsRNA precursors two to three days after Agrobacterial inoculation (Fig. 21A). The pHELLSGATE empty vector (EV) was used as a control. The purified total RNAs were applied onto the surface of fruits, vegetables, and rose petals and followed with *B. cinerea* infection. All the plants treated with the RNA extracts containing Bc-DCL1/2-sRNAs and -dsRNAs developed less severe disease symptoms and showed decreased fungal growth than those treated with RNAs extracted from EV plants (Fig. 21B-C). This result demonstrates that the Bc-DCL1/2-sRNAs and -dsRNAs, but not *N. benthamiana* total RNA, can protect plants from *B. cinerea* infection.

The effect of externally applied Bc-DCL1/2-sRNAs and -dsRNAs molecules on *B*. *cinerea* virulence and growth could be a result of the direct uptake of dsRNAs and sRNAs from the ambient environment by *B. cinerea* or an indirect process that involves plant uptake and processing prior to transport into *B. cinerea* cells. In order to test whether sRNA and dsRNA can directly enter *B. cinerea* cells or require an intermediate step involving plant hosts, we removed the plants out of the picture, and directly applied the Bc-DCL1/2-sRNAs and -dsRNAs on *B. cinerea* spores and germinated them on solid malt extract (ME) medium. To visualize the RNAs, we labeled the RNAs with fluorescein-12-UTP by *in vitro* transcription. Microscopic inspection of germinating conidiospores after 12 hours revealed fluorescent RNA signals accumulating in fungal cells (Fig. 22A). To eliminate the possibility that the observed fluorescent RNAs are adhering to the exterior cell wall, we treated the fungal hyphae with Micrococcal nuclease (MNase), and the fluorescence signal still remained, indicating the RNA indeed was taken up into *B. cinerea* cells. To further confirm this conclusion, the fluorescent RNAs were added into liquid culture of germinating *B*. *cinerea* spores, and the grown *B. cinerea* were subjected to protoplast preparation. The fluorescence signal was clearly detected in the *B. cinerea* protoplasts even after MNase treatment (Fig. 22B). *Bc-DCL1l2* were silenced by both Bc-DCL1/2-sRNAs and -dsRNAs, and only *Bc-DCL2,* but not *Bc-DCL1,* was silenced by Bc-DCL2-sRNAs and -dsRNAs (Fig. 22C). The growth of *B. cinerea* was reduced by treatment with Bc-DCL1/2-sRNAs compared to water treatment, and it was reduced to a lesser extent by treatment with Bc-DCL1/2-dsRNAs, Bc-DCL2-sRNAs, and -dsRNAs (data not shown). These data support that the Bc-DCL1/2-sRNAs and -dsRNAs were indeed translocated into the fungal cells, and they efficiently silenced fungal *DCL* genes. This RNA uptake mechanism makes it possible to develop a new generation of environmentally friendly RNA-based "fungicides."

Such an RNAi-based disease control method could be powerful if it is effective against multiple pathogens. For example, we could design *DCL*-targeting dsRNAs or sRNAs to silence *DCL* genes of multiple pathogens that utilize sRNA effectors. The soil borne fungal pathogen *Verticillium dahilae* is another economically important fungal pathogen, which causes verticillium wilt on a wide range of plant species, including herbaceous annuals, perennials, and woody species (Fradin et al., Mol Plant Pathol. 7:71-86 (2006); Klosterman et al., Annual Review of Phytopathology 47:39-62 (2009). To date, there is no effective control method other than toxic fungicide application. As discussed above in Example 1, *V. dahilae* also utilizes host AGO1 for its sRNA effector functions; thus, targeting *Vd-DCL* genes could also be a potential strategy for controlling verticillium wilt disease.

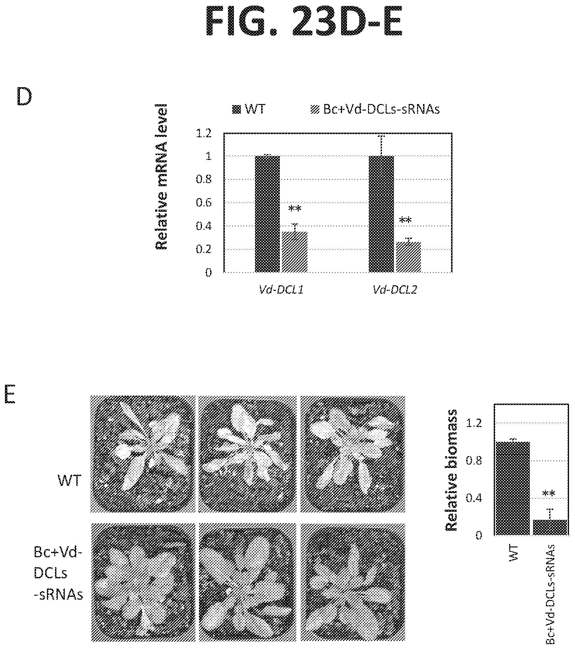

To test whether the RNAi-based method can simultaneously control *V. dahilae* and *B. cinerea,* we generated Arabidopsis transgenic plants that express hairpin RNAs targeting *DCL* genes of both fungi. *V. dahilae* also has two *DCL* genes. Again, we were not able to find a DNA region outside the conserved domains that has sufficient homology between *Vd-DCL1* and *Vd-DCL2 (Vd-DCL1*/*2),* or between the *DCLs* of *V. dahilae* and *B. cinerea* to silence two or multiple *DCLs.* Thus, we had to select two DNA segments, each from *Vd-DCL1* (156 bp) and *Vd-DCL2* (156 bp), which had 2.2% to 3.1% identity to the four *At-DCLs* (data not shown), and fused them to a 164 bp fragment of *Bc-DCL1* and 151 bp fragment of *Bc-DCL2,* both derived from the *Bc-DCL1*/*2* RNAi DNA fragment. This ligated product was then cloned into the pHELLSGATE vector. These transgenic plants expressed high levels of both Bc-DCL1/2-sRNAs and Vd-DCL1/2-sRNAs (Fig. 23A). As expected, the expression of Arabidopsis *DCL* genes was not affected in these transgenic lines (data not shown). Pathogen infection assays revealed that these transgenic plants are indeed more resistant to both *B*. *cinerea* and *V. dahilae* (Fig. 23C and 23E). Expression levels of *Bc-DCL1*/*2* and *Vd-DCL1*/*2* were significantly reduced in the *B. cinerea* and *V. dahilae* grown on these transgenic plants than those from the WT plants (Fig. 23B and 23D). Thus, this RNAi-based disease management strategy that targets pathogen *DCL* genes is efficient in controlling multiple fungal pathogens that use sRNA effectors.

### Discussion

We have provided the first example of bi-directional sRNA trafficking between a fungal pathogen and its interacting hosts. Plant host-generated *Bc-DCL*-targeting sRNAs can be transported into *B. cinerea* to silence *Bc-DCL* genes for disease control. In *C*. *elegans,* dsRNA uptake from the environment requires dsRNAs that are longer than 50 bp, instead of shorter dsRNAs or mature sRNAs. See, e.g., Winston et al., PNAS 104:10565-10570 (2007); McEwan et al., Molecular Cell 47:746-754 (2012). Some herbivorous insects are also capable of taking up dsRNAs that are longer than 50-60 bp, but not sRNAs (Ivashuta et al., RNA 21:840-850 (2015). Here, we show that *B. cinerea* can take up both sRNAs and dsRNAs directly, and both can induce silencing of *B. cinerea* genes efficiently, suggesting that the RNA uptake channels or pathways may differ in different organisms.

Eukaryotic pathogens, including fungi and oomycetes, cause serious crop losses annually. Currently, fungicide and chemical spray is still the most common disease control strategy, yet they pose serious threats to human health and environments. Over the last few years, the stable plant transformation-based HIGS system was proven to efficiently control a range of pests, nematodes, filamentous pathogens, and parasitic plants in various plant models and crop species. See, e.g., Baum et al., Nat Biotechnol 25:1322-1326 (2007); Huang et al., PNAS 103:14302-14306 (2006); Nowara et al. (Plant Cell 22:3130-3141 (2010). However, these successful HIGS studies relied on a plant transformation system that is not available for many crops. Some commonly selected pathogen target genes that are important for pathogen propagation and infection include parasitism genes, effector encoding genes, or fungal ergosterol biosynthesis related genes. We found that *B. cinerea DCL* genes are essential for the biogenesis of sRNA effectors as well as fungal growth and development. They are the ideal targets for controlling pathogens that use sRNA effectors. Here, we have discovered that *B. cinerea* can take up dsRNAs or sRNAs from the environment and induce environmental RNAi, making it possible to directly use such dsRNAs and sRNAs for disease management. We show that spraying *BcDCL1*/*2*-sRNAs and -dsRNAs on the surface of various fruits, vegetables, and flowers can efficiently control gray mold disease. This RNAi-based new generation of "RNA fungicides" could circumvent the technical limitation of transformation and the public's concerns about GMOs and provide an easy-to-use and environmentally friendly disease management tool for crop products both pre- and post-harvest. Furthermore, such RNA-based strategies could be easily designed to target multiple pathogens.

### Materials and Methods

**Plasmid construction:** The plasmids pHELLSGATE-Bc-DCL1/2 and pHELLSGATE-Bc+Vd-DCLs were constructed following the methods outlined for the Hellsgate8.0 system (Helliwell et al., Methods in Enzymology 392:24-35 (2005)). The Bc-DCL1/2 RNAi fragment was obtained by integrating *Bc-DCL1* (252 bp) and *Bc-DCL2* (238 bp) fragments via overlapping PCR. The Bc+Vd-DCLs RNAi fragment was obtained by integrating the 315 bp Bc-DCL1/2 RNAi fragment (164 bp of *Bc-DCL1* and 151 bp of *Bc-DCL2)* and RNAi fragments of *Vd-DCL1* (156 bp) and *Vd-DCL2* (156 bp) via overlapping PCR. The RNAi fragments were cloned separately into pDONR207 using Gateway^{®} BP clonase (Life Technologies, Carlsbad, CA), then into the destination vector pHELLSGATE8.0 using Gateway^{®} LR clonase (Life Technologies, Carlsbad, CA). For the pTRV2-Bc-DCL1/2 plasmid, the pDONR207-Bc-DCL1/2 vector was cloned into pTRV2 EV to obtain pTRV2-Bc-DCL1/2 using LR clonase (Life Technologies, Carlsbad, CA) (Weiberg et al., Science 342:118-123 (2013)).

***In vitro* synthesis of YFP-dsRNAs, Bc-DCL1/2-dsRNAs and -sRNAs:** Following the MEGAscript^{®} RNAi Kit instructions (Life Technologies, Carlsbad, CA), T7 promoter sequence were introduced into both 5' and 3' ends of the *YFP* and *Bc-DCL1*/*2* RNAi fragments by PCR, respectively. After purification, the DNA fragments containing T7 promoters at both ends were used for *in vitro* transcription. To obtain Bc-DCL1/2-sRNAs, the synthesized Bc-DCL1/2-dsRNAs were digested into sRNAs with Shortcut^{®} RNase III (NEB Ipswich, MA).

**In vitro RNA fluorescence labeling and confocal microscopy examination:** Bc-DCL-dsRNAs were labeled with fluorescence following the Fluorescein RNA Labeling Mix kit instructions (Sigma, St. Louis, MO). The fluorescent Bc-DCL-sRNAs were obtained from the digestion of fluorescent Bc-DCL-dsRNAs with Shortcut^{®} RNase III (NEB Ipswich, MA). For the confocal examination of fluorescent RNA-treated *B. cinerea* grown on the microscopic slides, 4 µl of a 10⁵ spores/ml solution and 4 µl of 100ng/µl fluorescent Bc-DCL-sRNAs or -dsRNAs was mixed and dropped on the solid ME medium prepared on the microscopic slides. After 12 hours of incubation in the dark, fluorescence was examined using confocal microscope SP5. To detect fluorescent RNA trafficking into the *B. cinerea* protoplast, *10⁷ B. cinerea* spores were cultured in 10 ml YEPD, and 2 µg of fluorescent Bc-DCL-sRNAs or -dsRNAs were added. The protoplasts were isolated as described previously (Gronover et al., Molecular Plant Microbe Interactions 14:1293-1302 (2001)) after 40 hours, followed by KCl buffer or 75 U Micrococcal Nuclease (Thermo Scientific, Waltham, MA) treatment at 37°C for 30 min. Fluorescence was examined using confocal microscope SP5.

**Preparation of *N. Benthamiana* total RNA with or without Bc-DCL1/2-sRNAs and -dsRNAs:** Four-week old *N. benthamiana* plants were infiltrated with *A. tumefaciens* strain carrying pHellsgate-Bc-DCL1/2 or pHellsgate-EV. The leaf tissue was harvested 2 dpi and used for total RNA extraction.

**External application of RNAs on the surface of plant materials:** All RNAs were adjusted to a concentration of 20 ng/µl with RNase-free water before use. For *in vitro* synthetic YFP-dsRNA, Bc-DCL1/2-sRNAs and Bc-DCL1/2-dsRNA, 20 µl RNA (20 ng/µl) was dropped or sprayed onto the surfaces of the plant materials. For *N. benthamiana* total RNA extracts, the surfaces of plant materials were evenly sprayed with 20 ng/µl RNAs.

**Fungal pathogen assay and disease quantification:** The *B. cinerea* strain B05.10 and *V. dahilae* strain JR2 were cultured on Malt extract agar (2% malt extract, 1% Bacto Peptone) and potato dextrose agar (PDA), respectively. The *B. cinerea* spores were diluted in 1% Sabouraud Maltose Broth buffer to a final concentration of 10⁴ spores/ml for spray inoculation on tomato leaf and 10⁵ spores/ml for the drop or spray of other plant materials (Mengiste et al., Plant Cell 14:2551-2565 (2003)). 10 µl spore suspension was used in drop or sprayed area of all plant materials, except tomato fruits in which 20 µl was dropped. *V. dahilae* soil-inoculation assay was performed as described previously.

**Arabidopsis root culture and infection:** For Arabidopsis liquid root culture inoculation, 2-week old Arabidopsis were grown in root culture (0.32% MS Salt, 2%Sucrose, 0.1%MES, pH5.8 by KOH), and *V. dahilae* spores were added into the culture to the final concentration of 10⁶ spores/ml. After 5 min inoculation, the root culture was replaced with fresh sterile solution. The fungal biomass quantification was performed as described previously (Gachon et al., Plant Physiol Bioch 42:367-371 (2004)).

**Cloning and data analysis of Arabidopsis AGO1- and AGO2-associated sRNAs:** *V. dahilae* infected Arabidopsis prepared in liquid root culture were collected at 2 and 4 dpi. At-AGO1 and At-AGO2 immunoprecipitations were performed using anti-AGO1 and anti-AGO2 peptide antibodies (Zhang et al., Mol Cell 42:356-366 (2011)). At-AGO1- and At-AGO2-associated RNAs were extracted and used for sRNA library construction and Illumina HiSeq 2000 deep sequencing (Weiberg et al., Science 342:118-123 (2013)). The read number of *Vd-sRNAs* in At-AGO1 and At-AGO2 IP libraries was normalized with total *V. dahilae* sRNAs after removing tRNA, rRNA, snoRNA, snRNA, etc. The Vd-sRNAs that had a higher than 100 RPM after normalization and also had host target genes in Arabidopsis were selected. At-AGO1 associated Vd-sRNA effectors were defined as the selected Vd-sRNAs with a 10 times greater read number in the At-AGO1 IP library compared to the At-AGO2 IP library. At-AGO2 associated Vd-sRNA effectors were defined as the selected Vd-sRNAs with a 10 times greater read number in the At-AGO2 IP library compared to the At-AGO1 IP library.

### Example 5: Exemplary DCL Gene and Protein Sequences

**SEQ ID NO:1 - *Botrytis cinerea* DCL1 genomic DNA sequence (selected RNAi fragment marked by bolded text)** 
ATGACGAGAGACGCAGCAGCAGCAAAAAGTCTCTACCATTGGCGAAGAAAAGGCGTCA
CTCCTTCAGCCGAAGAGGATCTTCTATCGTTTGATGATATTGTTACTGCCGTTCCACCTAC
AATCTTGTCTTCGTCTGTCGCTCCATATACTTCTCGAGATAAGATACCTTCTGCATCTGGC
AACGGAGATGCTATAGCAGATGTTAGCAGTGGTTACCTCAAACAGGCTACCGTATCTTCT
CATTCTGCTCAAGTCCGATCATCTTCAAACGGCAATCAAGGTGATGCCAAAAGTTCTCCC
TCTCTTTCACCTGATAGTAAACTGGAATTCATCTTTGGGCCTCCTTTAAGGGAGCCAGAG
AAGCCATTCTTTAATAAATCTTCTTATTCGTTTCGAGATTCGAGAGGGTTGAGCAGAAAT
CGGGCTTCTTCTTCTATGGAAAATTCGAGAACTCTCGATCCAAAGATACTCAAACCAGTT
ATCATCAATAATCACCAGGGCGAATGCTTCCAAGAGGCTTCCAGAACAGGTATACCTCA
GGCTGATACTTTTGATAAATCTTCCCTTGCTAAGACTGCGGATATGGATTTGTCACCAGTT
TCTCACCATGCGGATGTGCTTGCGACGACGGTCACTGCACAGCATTCTGCAATAGCCGCC
CAGAACGCAGCTCAAAGCTCTAAGATGCCAGGTCCTGAAGCTTTTTTACTTGCCGAAAAG
GACGAGGCAGGTTCTCCCGTTGTTATATCACTGGGTTCTGCAAACCAAATTCCTTCTGGA
AACATTTCTTTGCAGCTTGATTCACCATCTCTGGAAAACCATTCTCCAAATGTGACCCCA
ATCAACAAAGTCCCTACACCATTCGCACTTTCTACAAGGACAACCGATGACGTTTTCGCA
GAACTTAGGCGGCCTTTGCATCCCCAAGCTATTCAGAGCCAGATTGATATCAAGACTTCC
TCTTGTGTTGATAGTTATAACACGAATGATGAGATTCTAGACAACAATCAAGGTTCCAAT
CAAAAAGATCTGCATGTTGTTGAAAAGGATAAGGAAGAGGAAGAGGAAGAGGATATGA
ACCAAGCCATACCCGATATCAAACGTATCTCAGCACGAAAACAAAAGAACGCTGCCATA
TTTGACGTTTTTCTTAAGGAAGCTACCAAACTACCAAAGACAGAAAAGACTTCACATGCG
AATGATGAAGCAATTCAGTCTACTAGGTGGTTGATTGACCAAGCAGAAAAACAGCATAT
TATAGAAAGTCCCAGGGACTATCAACTTGAATTGTTTGAGAAGGCAAAGAAACAGAACA
TTATAGCTGTACTTGATACAGGATCTGGCAAGACATTCATTGCAGTTCTCTTACTTCGGTG
GATCATAGACCAAGAGCTTGAAGATAGAGCTATTGGCAAGCCTCATCGTGTTTCATTCTT
CCTGTGGAAGAAACGACTGGATACGAATATGGTCATTGTCTGCACTGCAGAAATTTTGCG
CCAATGCCTGCACCATTCGTTTGTTACAATGGCTCAAATAAATCTGCTAATTTTCGATGA
AGCCCACCATGCAAAGAAGGATCATCCTTATGCTAGGATTATTAAAGATTTTTATCGCAA
TGACACGGAAAAGGATATCGCTCTGCCTAAAATATTTGGGATGACAGCATCACCGGTAG
ATGCTAGAGATAATGTCAAGAAAGCTGCGGAAGAACTTGAAGGTTTGCTACACAGTC
AAATATGTACTGCAGAAGATCCCAGCTTGCTGCAGTACTCAATCAAAGGTAAACCT
GAGACTCTTGCCTACTATGATCCCTTGGGCCCGAAATTCAATACTCCTCTTTATCTT
CAAATGCTCCCGCTTCTAAAAGACAATCCTATCTTTCGGAAGCCATTTGTATTTGGG
ACAGAAGCCAGTAGAACTCTAGGATCTTGGTGTGTTGACCAGATCTGGACTTTCTGT
CTTCAAGAAGAAGAGTCTAAGAAACTACAAGCAAGGACGGAGCAGGCGCATCATAAGA
AGAGAGTCCCGGAGCCACTTGAAGTGCTAGAGAAACGCAAGGAACAACTTGAACAAGC
 
CAAATCCATTGTCGAAAATCACACTTTCGAGCCACCACACTTTGCATCAAGATTATTGGA
TGATTTCACAACAAAAGTTCACTATTCGAATAATTTATCTACTAAAGTCGTTGCTCTCTTG
AGTATTCTCAAAGATCGTTTCCAACGACCCACCAATGACAAGTGTATTGTATTTGTCAAA
GAAAGATACACCGCACGCCTTCTAGCCTCACTTCTCTCCACACC
TGAAGCTGGGACACCATTCTTGAAGGCTGCACCGCTGGTTGGTACTACGTCTGCTTCAGC
CGGGGAAATGCATATCACATTTAGATCACAAACTCTTACTATGCACAACTTTCGCAATGG
TAAAATCAACTGCCTTATCGCAACATCAGTTGCTGAAGAAGGTCTTGACATTCCTGACTG
TAACCTCGTTGTCAGATTCGATTTGTACAATACAGTCATTCAGTACATTCAATCTAGAGG
TCGTGCTAGGCATATCAATTCAAGGTACTACCATATGGTAGAGAGCCACAACGAGGAAC
AGATTCGTACAATCAAAGAGGTTTTGAAGCATGAGAAAATGCTAAAGCTTTTTGCTTCTG
CTCTTCCAGAAGATCGAAAATTGACCGGAAACAACTTCAATATGGATTACTTCCTCAGAA
AAGAACGAGGCCACAGAATTTACCCTGTCCCGAATAGTGACGCAAAACTTACTTACAGA
ATGAGCTTAACGGTCCTATCTGCCTTCGTTGACTCACTTCCTCGAGCCCCAGAGTCGGTTC
TTCGAGTGGATTATGTCGTCACAACTGTCGATAAGCAGTTTATCTGTGAGGCCATTTTGC
CAGAAGAAGCACCCATACGCGGAGCAATTGGTCGGCCAGCAACAACTAAACAAGTGGCC
AAATGCTCAGCAGCCTTTGAAACTTGTGTGATTCTGCACCAGAAAGGATACATCAACGAC
TACCTACTTTCTACATTTAAAAGATCAGCACACATGATGAGAAATGCACTTTTGGCTGTG
GATGGAAAGAAGCAAGAAGCTTATGATATGCAGACTAAACCAACTTTATGGTCTTCGAA
AGGGAAACAAGGCATATTTTATATGACTGTCTTGTCTCTCAAATCTCCAGATAATCTTGA
CAGAGCATCTCAGCCATTGGGCTTACTGACAAGATCACCCTTGCCTGATTTGCCAGAATT
TGTTCTTCATTTCGGAGCAGGGCGAAACTCTCCAACCTCGTGCGTACCTCTCGCTTCCTCA
ATTACGCTCGAAAAAAACAAGCTTGACCAAGTTAATATGTTCACCCTATGTTTATTCCAA
GATGTGTTCAGTAAAGCATACAAATCAGATCCGGATAGTATGCCATACTTTCTGGTTCCT
ATCAACTGCCTGAATGCTATTGTCGACTGGAAATCACAAAACCCAATGTCAATAATCGAT
TGGGAGACAGTTGAATATGTCCAAGACTTCGAGAATAAGCAAGCTGATAAGCCATGGGA
GCACAAGCCATGGTTAGGAAAGCCTGACGATTATTTCAAAGACAAATTCATAACTGATC
CCTTTGACGGGTCTCGAAAATTGTGGTCCGTTGGAATCACAAAAGAATACAGACCATTGG
ATCCAGTCCCACCAAACACGGCGCCCAGGAAGGGAGCTAGAAAGAACAATAGTAATATC
ATGGAGTATAGTTGTAGTCTCTGGGCAAAGGCTAGAGCAAAACGAACTTTTGATGAAGA
ACAGCCTGTTATTGAAGCAACCTACATTTCACTTCGGAGAAATTTGCTTGATGAATTTGA
TGGAGGTGAGCTCGAGACTTCAAAGAAGAGTTTTATTATTTTAGAACCATTGAAGGTATC
ACCTCTTCCAACTACCGTGGGTGCAATGGCCTATCTTTTACCTGCAATTATTCATCGAGTT
GAGTCATATCTCATTGCTCTTGAAGCAACAGACTTGTTACATCTTGATATCCGTCCTGATC
TTGCGCTAGAGGCTGTTACCAAGGATTCCGACAATTCTGGAGAGCATGGTGAGGAACAG
ACAAACTTTCAACGTGGAATGGGCAATAATTATGAACGATTGGAATTTCTTGGGGACTGC
TTCTTGAAGATGGGAACGTCAATATCTCTATACGGTCTAAATCCTGATAGTGATGAATTC
CGCTACCATGTTGATCGTATGTGTCTGATTTGCAACAAAAATCTGTTCAATACGGCTTTG
AAATTAGAGCTTTACAAATACATTCGGTCGGCAGCCTTCAACCGACGAGCTTGGTATCCC
GAAGGCCCCGAATTATTAAGAGGAAAGACAGCCACGGCACCAAATACCCACAAGCTCGG
CGATAAGTCAGTTGCAGATGTTTGTGAAGCAATGATTGGAGCTGCTTTACTAAGCCACCA
CGAAAGCAAGTCCATGGATAATGCGGTTCGCGCCGTTACTGAAGTTGTCAATAGTGACA
ACCACAATGCTGTTGTATGGTCTGATTATTACAAATTGTATGAGAAACCAAAATGGCAAA
CTGCTACAGCTACAGCTGCACAAATAG
ATATGGCAAGACAAGTTGAAATGAAACATCCATATCATTTCAAACACCCACGCCTGTTAA
GATCAGCTTTCATCCATCCGGCATACTTGTTCATCTATGAACAAATTCCTTGTTATCAACG
TCTCGAATTTTTGGGTGATTCGCTACTCGATATGGCATGTGTCAACTTCCTTTTTCACAAC
CACCCAACAAAAGATCCTCAGTGGCTCACTGAGCACAAGATGGCTATAGTATCCAATCA
GTTTCTTGGAGCTCTTTGTGTCAAATTAGGCTTCCACAAACATCTACTGACACTCGATTCT
CAAGTTCAAAAAATGATTGCAGATTACTCCTCAGATATCAATGAAGCTCTCATTCAAGCC
AAAACGGACGCAAAGAGAGTCGGCAAAGTAGAAGATGATTACGCTCGTGATTATTGGAT
TGCCGTCCGTCAACCTCCTAAATGTCTTCCCGATATTGTAGAAGCATTCATTGGTGCCATT
TTTGTCGACTCTGAGTATGACTACGGTGAAGTTGAGAAGTTCTTTGAAATGCATATCAGA
TGGTACTTTGAGGATATGGGCATCTACGATACCTATGCTAACAAGCACCCAACCACTTTC
CTTACTAATTTCTTGCAAAAGAACATGGGATGTGAGGACTGGGCACCAGTTAGTAAGGA
AGTACCTGGAGAGGATGGTAGAAAGAATGTTGTAGTTTGCGGGGTCATCATACACAATA
 
AGGTGGTATCAACTGCCACTGCCGAAAGTATGAGATATGCTAGGGTCGGAGCAGCGAGG
AATGCCTTGAGAAAATTGGAGGGAATGAGTGTCCGAGAATTCAGGGATGAATACGGGTG
CTCATGTGAAGGTGATGTTGTTGATGAAGAGGGCAATATTGAATTTGTTGAACGTGAAGA
CGGGATGGAGGGGATCGGTATGGGATATTGA
**SEQ ID NO:2 - *Botrytis cinerea* DCL1 protein sequence** 
MTRDAAAAKSLYHWRRKGVTPSAEEDLLSFDDIVTAVPPTILSSSVAPYTSRDKIPSASGNG
DAIADVSSGYLKQATVSSHSAQVRSSSNGNQGDAKSSPSLSPDSKLEFIFGPPLREPEKPFF
NKSSYSFRDSRGLSRNRASSSMENSRTLDPKILKPVIINNHQGECFQEASRTGIPQADTFDK
SSLAKTADMDLSPVSHHADVLATTVTAQHSAIAAQNAAQSSKMPGPEAFLLAEKDEAGSPVV
ISLGSANQIPSGNISLQLDSPSLENHSPNVTPINKVPTPFALSTRTTDDVFAELRRPLHPQA
IQSQIDIKTSSCVDSYNTNDEILDNNQGSNQKDLHVVEKDKEEEEEEDMNQAIPDIKRISAR
KQKNAAIFDVFLKEATKLPKTEKTSHANDEAIQSTRWLIDQAEKQHIIESPRDYQLELFEKA
KKQNIIAVLDTGSGKTFIAVLLLRWIIDQELEDRAIGKPHRVSFFLWKKRLDTNMVIVCTAE
ILRQCLHHSFVTMAQINLLIFDEAHHAKKDHPYARIIKDFYRNDTEKDIALPKIFGMTASPV
DARDNVKKAAEELEGLLHSQICTAEDPSLLQYSIKGKPETLAYYDPLGPKFNTPLYLQMLPL
LKDNPIFRKPFVFGTEASRTLGSWCVDQIWTFCLQEEESKKLQARTEQAHHKKRVPEPLEVL
EKRKEQLEQAKSIVENHTFEPPHFASRLLDDFTTKVHYSNNLSTKVVALLSILKDRFQRPTN
DKCIVFVKERYTARLLASLLSTPEAGTPFLKAAPLVGTTSASAGEMHITFRSQTLTMHNFRN
GKINCLIATSVAEEGLDIPDCNLVVRFDLYNTVIQYIQSRGRARHINSRYYHMVESHNEEQI
RTIKEVLKHEKMLKLFASALPEDRKLTGNNFNMDYFLRKERGHRIYPVPNSDAKLTYRMSLT
VLSAFVDSLPRAPESVLRVDYVVTTVDKQFICEAILPEEAPIRGAIGRPATTKQVAKCSAAF
ETCVILHQKGYINDYLLSTFKRSAHMMRNALLAVDGKKQEAYDMQTKPTLWSSKGKQGIFYM
TVLSLKSPDNLDRASQPLGLLTRSPLPDLPEFVLHFGAGRNSPTSCVPLASSITLEKNKLDQ
VNMFTLCLFQDVFSKAYKSDPDSMPYFLVPINCLNAIVDWKSQNPMSIIDWETVEYVQDFEN
KQADKPWEHKPWLGKPDDYFKDKFITDPFDGSRKLWSVGITKEYRPLDPVPPNTAPRKGARK
NNSNIMEYSCSLWAKARAKRTFDEEQPVIEATYISLRRNLLDEFDGGELETSKKSFIILEPL
KVSPLPTTVGAMAYLLPAIIHRVESYLIALEATDLLHLDIRPDLALEAVTKDSDNSGEHGEE
QTNFQRGMGNNYERLEFLGDCFLKMGTSISLYGLNPDSDEFRYHVDRMCLICNKNLFNTALK
LELYKYIRSAAFNRRAWYPEGPELLRGKTATAPNTHKLGDKSVADVCEAMIGAALLSHHESK
SMDNAVRAVTEVVNSDNHNAVVWSDYYKLYEKPKWQTATATAAQIDMARQVEMKHPYHFKHP
RLLRSAFIHPAYLFIYEQIPCYQRLEFLGDSLLDMACVNFLFHNHPTKDPQWLTEHKMAIVS
NQFLGALCVKLGFHKHLLTLDSQVQKMIADYSSDINEALIQAKTDAKRVGKVEDDYARDYWI
AVRQPPKCLPDIVEAFIGAIFVDSEYDYGEVEKFFEMHIRWYFEDMGIYDTYANKHPTTFLT
NFLQKNMGCEDWAPVSKEVPGEDGRKNVVVCGVIIHNKVVSTATAESMRYARVGAARNALRK
LEGMSVREFRDEYGCSCEGDVVDEEGNIEFVEREDGMEGIGMGY*
**SEQ ID NO:3 - *Botrytis cinerea* DCL2 genomic DNA sequence (selected RNAi fragment marked by bolded text)** 
ATGGAATACACTTCGGAACCTGACACTGACCCGGATACACGCGGTAGCCTTATCGATGGT
CGAGATGGGATTGAAGGGGATCTTATTGCTTTGACGTCTGGGGAACGACTTAATGAGACT
GTAGAGGATTTATGTAGTGACTCATCAGGATTGATTGTTGAGAATGAAGATGATGATAAC
AGCGCAGGGGAGAAGGGAGAGATTGTGATAGTAACACCAAGAACATACCAACTGGAAA
TGTTGGAAGAGAGTTTGAAAAGGAATGTCATCGTTGCGATGGATACAGGAAGTGGCAAG
ACACATGTGGCCGTTCTCCGAATACTAGCGGAACTTGAGCGGATGAAGCCTGGCAAGAT
AATATGGTTTCTTGCGCCTACCGTTGCGCTCTGTGCTCAGCATCACGAATATCTCCAGCTG
AATATTCCCTCTGTTTTGATCAAAATGCTTATTGGTGCTGATGGTGTGGATCGATGGACA
GAGCAGAGACAGTGGGATACGGTCTTGAAGGATGTCAAGGTAGTCGTATCTTCCTATCA
AGTTCTTCTAGATGCCCTTACACACGGATTCGTACGCATGGGGCGTCTGTCCTTGATCATT
TTTGATGAAGCACATAATTGTGTAAATAAAGCGCCAGGGGCTAAAATTATGAAATCTTTC
TATCATCCGTATAAATCGATATTCCCACTTCCCCACATTCTGGGCCTCTCGGCCAGCCCTG
TCATGAGATCCAGTCCACAATCTTTAAGTGATATCGAGGAGACTTTGGATGCCATTTG
 
CTGCACGCCAAAAATACATCGAGCAGATCTTCGCCTTCGAGTAAAGCTACCACTTCT
ATCTATTATCTACTATACCCCAGAGTCAAATATCATCGTGACGAAAACTGTGGCGAG
CCTGAGAAAGATTGTGCAAAGTCTCAACATTTTCGAAGACCCCTACGTTTTGACACT
AAAAAGGAGTGATAGCGAAAAAAGTCAACGTGAGCTGGCGAAAGTACTCAAGAGTT
TTAAGACATATAGTCAAACCCAATTAAAGTCAATCGACAAAACTAGCAACGAGATTAT
TCTTGTAGAGCTAGGCCCATGGGCTGCAGATTACTATATCTCAACAGTGGTGACGAGATA
CTTGAAGGCAATGTCGGCAAAGGACACTTTCATTGTTGAAGATTCACCAGCTGCCGAGA
AGCTATATATTGCCAAGGCTCTCAGACAAGTCGAAATCTCTCCTTCAACTCTCTCAGATA
CAGGCAAAATTTCTAACAAGGTTGAAAAGCTACTGGGGATAATTGCGCAACAGAAGCCT
CCCTTTTCCGCTATTATATTTGTCCAAGAAAGAGCCACGGTGTCTGTGCTAGCCCATCTAT
TATCGCATCATCCATTGACAAAGGATCGTTTTAAGATTGGAACCATGGTTGGCACATCCT
TAAATGGCAAGCGTACAGACCAAATAGGAGAGCTTGTCGATGTTAATCAACAAAAAGAC
ACTTTGTCAAGTTTCAAGCGTGGAAAAATTGATATCCTTATAGCTACAAATGTATTGGAA
GAGGGAATTGATGTTCCTGCCTGTAATCTAGTGATCTGCTTTAGTAAACCAGCAAACCTC
AAATCTTTCGTACAAAGACGAGGGCGAGCAAGACAGCAAGATTCTAAGCTGATTCTTCTT
GATGCTTCAGGTGATAAAGCGACAAATTGGCATGAGCTTGAAAGAAAAATGCGAGAGGA
GTACGGAAAGGAAATGCGAGAATTGCAACACATCTACGAAATTGAGACAGCTGATGAAC
AGTCGGAAGATGATAGGGTCTTGCGAATAGAAAGCACTGGGGCTCAATTAGACCTTGAC
AGTGCTTTACCACATCTCTATCATTTCTGTTCAGTCTTAACAACAAAAGATTTTGTTGACC
TCAGGCCAGACTTCGTCTACTCCTCCGAACTGGGATCGGAATATGTTCGAGCAAAGGTCA
TCCTGCCTGGATCGGTTTCTAAACCCCTGCGAGTCCATGAAAGCCGCGGATCGTGGTTGA
GCGAGAGGTCGGCTGCAAAAGATGCAGCGTTTGAGGCGTATTCCGCATTATACAGGGGG
GGCTTAGTGAATGATAACCTACTGCCCCTGATGGTGCACGACAAAGTCATCGATGAGTTG
ACTTCAAAGCCCGTGGATACTCGCGCGTCTCTTCTGGAGGTGAAGGAAAGATTAAATCCA
TGGATTGACATTGCTAGAGCATGGAAAGAGGCAGAACACCATGCTGGAATTGTTCGCAC
ATCGGTAATGATCTTCAATGGGATGAAGCTGGAACTCTGTCTTCC
AATTGATCCACCGGCAATACCCCCATTAAAGCTTTATTGGGATGCTGACACCGAGTTCTT
TGTTGACTTTACAAACGATATCGAGATCGGCACCAGCGAGAATATGTTGGCACAGGCGTT
GAACGATACCAATCTACTATTATCAGATCGTGGTCGTAAAGTTCACATCCAGTCACGTCG
AACAGTTGTGCAATTTATCTTGCTTCAAGATTCGGGCTCGCTCAGTTCAGATTGTTTTCCG
GTTGACCCCAACGGTAATATTAAAAGTACAGGTTTTATCAGAGAAGTCGGTAAACTAGA
ATCGCCCTACATCTTTGAAAAATGGTTGCCCAATGCACCAGAAGACGTCCCATATCTAGC
TGTGGTTAAAGTAAGTCGCCGTGCAGACTTTTTGCACAAGGTACAGAACGAAAAACCCT
CGTCATTCACTAAACAATTCTCGTCTGTTCTACCTGCCTCGACATGTGTACAGGATGTAAT
GCCCGCACAGTTGTCTCGGTTCGGCATGATGATTCCTTCCATCACACACCACATTGAGGT
GCAACTCGTTGTAGACCGACTATCCAGGACCATCCTCAAGGATCTCGAAATTAGTGACCA
GAGTCTTATTCAGACCGCCATCACACATGCCAGTTATTCGTTAGACTCGAATTATCAGCG
TCTCGAATTTCTGGGCGACTCAATTCTCAAATTGTGTACATCGGTACAATTGGTGGCAGA
GCATCTAGATTGGCACGAAGGATATTTGTCGGCTATGAAGGATCGTATCGTGTCCAATTC
ACGGTCATCAAGAGCGGCGGCTGAAGTCGGTTTGGATGAGTATATAATGACCAAGAAAT
TCACAGGTGCAAAATGGCGACCAATGTACGTGGATGATCTGGTCGTCACAGAACAAAAA
ACAAGAGAAATGTCCTCCAAAATTCTTTCCGACGTTGTGGAAGCACTCATCGGCGCATCT
CTCCGGCCCGTCGAGCAAATCCTCGCATATACCTTCACCAAAAAATCTCTCCTCGTCGAA
GCCATGACGCACCCCTCTTACACCAGCGGCACGCAATCCCTCGAGCGACTCGAGTTCCTC
GGCGATTCCATTCTCGACAACATCATCGTCACAGCCATGTGGTCGCACTCGACGCCGCTC
TCCCACTTCCACATGCATCTCCTGCGCTCTGCGCTCGTCAACGCCGATTTCCTCGCCTTTC
TCTGCATGGAAATGAGCATCGACCAAAACGTCACCAATCTGACCGAAGGAAAAAACCAT
CGCATCCACGAAACCCACTCGCGACGCCGCGTTTCCCTCGTCAGTTTTCTCCGTCACTCA
AGCGTTCGTCTCTCTATCTATCAAAAAGAAGCGCTTTCTCGCCATGCAGAATTGCGCGAT
CAGATCCTCGAGGCAATATACACCGGTGATACATTCCCCTGGGCTCTATTATCCCGATTG
GACGCGCGGAAATTTTTCTCCGATATGATTGAGAGTTTGCTGGGCGCGGTATGGATTGAT
AGCGGCTCGATGGAAGTGTGCACGCAGCTGATCGAAAGAATGGGCGTCCTGAGATACAT
GCGACGGATTTTGAAAGATGGCGTGCGCATCATGCATCCGAAGGAGGAACTGGGCATCG
TGGCCGATTCTGAAAACGTCAGGTACGTTTTGCGGCGGGAGAAGATGGGTGGGGATGCT
ACCGAGGTAAATGCGGACGCGGATGAAGAGGTACGCACGGAGTACCGGTGCACAGTATT
 
TGTGGGCGGGGAGGAAATTGTAGAGGTGAGGGGTGGAGCGAGGAAAGAGGAGATTCAG
GCAAGGGCTGCGGAGCAGGCGGTGCGGATTTTGAAGGCGAGGGGTCATGAGAAGAGGA
ATGGGGGTGCGGGGGAGGGGAAAAAGAGAAAATCGCTGGATGAATAG
**SEQ ID NO:4 - *Botrytis cinerea* DCL2 protein sequence** 
MEYTSEPDTDPDTRGSLIDGRDGIEGDLIALTSGERLNETVEDLCSDSSGLIVENEDDDNSA
GEKGEIVIVTPRTYQLEMLEESLKRNVIVAMDTGSGKTHVAVLRILAELERMKPGKIIWFLA
PTVALCAQHHEYLQLNIPSVLIKMLIGADGVDRWTEQRQWDTVLKDVKVVVSSYQVLLDALT
HGFVRMGRLSLIIFDEAHNCVNKAPGAKIMKSFYHPYKSIFPLPHILGLSASPVMRSSPQSL
SDIEETLDAICCTPKIHRADLRLRVKLPLLSIIYYTPESNIIVTKTVASLRKIVQSLNIFED
PYVLTLKRSDSEKSQRELAKVLKSFKTYSQTQLKSIDKTSNEIILVELGPWAADYYISTVVT
RYLKAMSAKDTFIVEDSPAAEKLYIAKALRQVEISPSTLSDTGKISNKVEKLLGIIAQQKPP
FSAIIFVQERATVSVLAHLLSHHPLTKDRFKIGTMVGTSLNGKRTDQIGELVDVNQQKDTLS
SFKRGKIDILIATNVLEEGIDVPACNLVICFSKPANLKSFVQRRGRARQQDSKLILLDASGD
KATNWHELERKMREEYGKEMRELQHIYEIETADEQSEDDRVLRIESTGAQLDLDSALPHLYH
FCSVLTTKDFVDLRPDFVYSSELGSEYVRAKVILPGSVSKPLRVHESRGSWLSERSAAKDAA
FEAYSALYRGGLVNDNLLPLMVHDKVIDELTSKPVDTRASLLEVKERLNPWIDIARAWKEAE
HHAGIVRTSVMIFNGMKLELCLPIDPPAIPPLKLYWDADTEFFVDFTNDIEIGTSENMLAQA
LNDTNLLLSDRGRKVHIQSRRTVVQFILLQDSGSLSSDCFPVDPNGNIKSTGFIREVGKLES
PYIFEKWLPNAPEDVPYLAVVKVSRRADFLHKVQNEKPSSFTKQFSSVLPASTCVQDVMPAQ
LSRFGMMIPSITHHIEVQLVVDRLSRTILKDLEISDQSLIQTAITHASYSLDSNYQRLEFLG
DSILKLCTSVQLVAEHLDWHEGYLSAMKDRIVSNSRSSRAAAEVGLDEYIMTKKFTGAKWRP
MYVDDLVVTEQKTREMSSKILSDVVEALIGASLRPVEQILAYTFTKKSLLVEAMTHPSYTSG
TQSLERLEFLGDSILDNIIVTAMWSHSTPLSHFHMHLLRSALVNADFLAFLCMEMSIDQNVT
NLTEGKNHRIHETHSRRRVSLVSFLRHSSVRLSIYQKEALSRHAELRDQILEAIYTGDTFPW
ALLSRLDARKFFSDMIESLLGAVWIDSGSMEVCTQLIERMGVLRYMRRILKDGVRIMHPKEE
LGIVADSENVRYVLRREKMGGDATEVNADADEEVRTEYRCTVFVGGEEIVEVRGGARKEEIQ
ARAAEQAVRILKARGHEKRNGGAGEGKKRKSLDE*
**SEQ ID NO: 5 - *Verticillium dahilae* DCL (VAD_00471.1) genomic DNA sequence selected RNAi fragment marked by bolded text)** 
ATGACGACTGACGAGCTCTCTGTTGGTCTGGACGCCACCGGCATCTCAATCCTCGCAGAT
GGACCGGAAAACATATCGTCCAGCACATCAACATCTACGACTGGAAAGGAAGATGGATA
CCTCTGTATCAACAGATTCACTCAGAATACCGCCACGACCCAGGACAACCAGAGCCGAG
ATTCTGACGACGATGAGGATGACTGCGGCAGCCACGATGAAGCTGACGAAGATTCAGAC
GAAAGACAGTACAGCATGACCCCAGAAAGGCCTCATAAAATTACCGAGAAGAAGCGCG
CAGATCATGCTGCCTTTCACGACTGGCTTCAGAGCAACTCCAGCGAGATTGCTCAGTCAA
CCCCTCAGCCGGCTCAAAACCTCAACCACACCTCCACGGCCCTGATGGTACGCGAGAGT
GAGAATCGTAAGATCATCGAAAATCCTCGGGAGTATCAGATTGAGCTCTTCGAGCGGGC
GAAGCGAAAGAACATCATTGCCGTGTTACCCACTGGATCAGGAAAGACCTTAATCGCAG
CCCTTCTTCTGCGACACACCCTCGAACAAGAAACCGCGGATCGACGCGCGGGCAAGCCC
AAGAGAATCGCCTTTTTCCTCGTGGAAAAGGTTGCTCTTGCCCTCCAACAGCACGC
GGTTCTGGAGTGCAATCTGGAATTTCCCATTGACCGGGTATGCGGTGACATGGTAC
GGTCGGACTGGATCAAGGAGTCATGGATGAAAAGATGGGATGACAACATGGTCATG
GTCTGCACCGCCGCCATCCTTCAGCAATGCCTTGCCAGATCATTCATCCGCATGGAT
CAGATCAACCTGCTTGTCTTCGATGAAGCACATCACGCCAAGGGAAATCATCCGTA
CGCCCGGATCATCAAGGACTACTACATTACGGAACCTGACAAAGAAAGGCGCCCCAAGA
TCTTCGGCATGACTGCCTCTCCGGTGGATGCCCTCACCGACGTCAAGATTGCTGCCGCTC
AACTCGAAGGTTTGTTGCATAGTGAGATTGCGACAATCGAGGAGGACTCTGTATCATTCA
AACAAATCCAGAAAGAGGTCGTCGAACAAGACTGCAAGTACCCTGCCCTCGAACCACCC
TTCACCACCAATCTTCATAAGAAGATCCAAGAACAGGTGCGCTACAACAAGAACTTCGC
AAAGGCGCTGAGCAATTCTTTAGAAATGTCGAGCTCCCTTGGCAGCTGGTGTGTCGATCG
 
CTTCTGGCAGATATTTCTGACCGAAGAAACCCTCGCGAGATTGGCAGCGCAAACTGCAC
AAGACAACATTTTTGCCGATCGCGCCGAAAAGGAGCGCGTTGCCATTGAGGAGGTCCGC
AACATCATCAAGCAACATCAGTTCCTCCCAATCACCAAAACCCTGCAAGACTTGTCGTCC
AAAGTGCTGTGCCTCCTCGGCCAACTGGAATTGCGCTTCAGTGCCCCTACCGATCACAAG
TGCATCATCTTCGTGGAGAAACGAAACACAGCCATGATTCTGGCTCACCTCCTCTCCTTG
CCTGGTATTGGACCTCTATATCTGAAACCGGCTGCGCTTGTCGGGAACCCATCTGACAAC
AGCCCTCTTGCCATGTCGTACAAAGAGCAAGTGATGACAATAACAAAGTTCAGACGTGG
TGAATACAACTGTCTTCTCGCCACTTCTGTGGCCGAGGAGGGCATTGACATCGCAGACTG
CAACATTGTCATTCGATTCGATCTTTTCAACTCGGTGATTCAGTACATACAATCCAAAGG
CCGCGCTCGGCACTTGAACTCGGAGTATATTTGCATGGCCGAGCTAGGCAACGGCAAGC
ATACAAGGGCGAAGATACAAGCAAATTATGACCTCTCCCTCATCCGCCAATTCTGCAGCA
CACTGCCAGAAGACCGCAAGATCGTGGGCTGGGACCCCGAGGCAGCTCTTCACCATGGC
GAGCGCGACCATAAGTTCCACATCGTTCCATCCACCGGGGCCAAACTCACCTGGAC
CGGCAGCCTCGTGGTTCTGTCAAATTTTGCCTCTTCTCTACAGGTGAACGACGAAACACT
AAGTCCTTCCTATATGGTCTCTCTCATCGGTAGCGAGTACATCTGCGAGGTCCAGCTTCC
GAGCAAGTCTCCCATTTTGAGCGTGTCAGGCACGCTCCAAAAGAACAAAGCAGAGGCCA
GGTGCTCCGCAGCGTTTGAGATGTGCATGAAGCTCATCAAAGGTGGGTTCATCAGCAGTC
ACCTTCAGCCGACGTTTACCAGGAAGCTCCCGGCCATGCGAAACGCACGCCTAGCCATC
AGCTCCAAGAAGCGTGAACGGTACAATATGAGGGTCAAGCCAGAGGTATGGTCACGGCG
TGGACCGGCATCCTCTCTGTTCCTCACAGTCCTGAAGCTTCGTACACCTGGTGCATTGAA
CAGACCATCACAGCCACTCGCCCTCCTCACACGAGAGGCACTGCCAGAGCTTCCAGGAG
TTCCGCTATTTTTCGGTAACTGTGGTCGGTCCATAGCGGAGGTAGTATCTGTGGCGAAAC
CCATGCACTTGGATGAAGTACGTCTAGACAGCCTCAGAGTATTCACCCTGCGCATTTTCA
AAGATGTCTTCAGCAAGGTATACGATTCTCAAGTCGCAGACCTTCCATACTTCCTGGCAC
CTGCTGCTCATGACCACAGTCATGAGTTCTCACCGAATGAAGACCCAGGGTCACTGATCG
ACTGGAGCCATCTGCTGTCGACCAAAGAGGTTGAGTACTTGCCTTGGGATGAAGATCAC
AGTCCCAGCTTCTATCAAAGCAAGTTTGTGATTGATCCATACACGGGATCGCGCAAGCTG
TTTCTCAGAGGTATTCGGACAGATCTCAAGCCGACCGACTTGGTTCCAGATGGAGTTCCC
GAACCCACATTCAGGCTCTGGAAGGACGTTGAGCATACCATAAAGGAATACAGCATCAG
CCTCTGGGCAAAGAGTCGAGCCCGGAGAGCTGGCGAATGGTTGGACACTCAACCCGTGG
TAGAAGCCGAGTTGGTCTCGCTGCGCCGGAATCTTCTCGACGAATTTGCCGATTCCAAGC
ATGAAGGGTCTAGGGTCTGTTATGTGATTCTCCAGCCGCTACAGATCTCAACACTCCCTG
TCGAGGTCGTCGCTATGGCCTACAACTTTCCCGCCATCATCCATCGGATTGAATCGAATA
TGATCGCCCTTGACGCCTGCCGTATGTTGAACCTTCGAGTTCGTCCCGACCTGGCTCTCGA
GGCGATGACCAAAGATTCAAGCAACAGTGAAGAGCACGATCAGGAAAAGATTGATTTCC
AGGCCGGCATGGGCAATAATTATGAGCGACTCGAGTTTCTCGGAGACTGCTTTCTCAAAA
TGGCAACCACCATCGCACTTTTTACTCGGATCCCTGACAGCAACGAGTTTGAGTGTCACG
TCGAGCGAATGCTTCTTATTTGCAACCAGAATCTGTTCAATGTCGCATTAAAGAAGAACT
TGCAAGAGTACATTCGATCAAAGCAATTCGATCGACGCAGTTGGTACCCCCAGGGTCTG
AAGCAGAAGGCGGGCAAAGCCCAAGGAGCACAAAACTCACACTCATTGGCCGACAAGT
CTATTGCTGATGTATGCGAGGCCATCATTGGCGCCTCATATTTGTCGTACACTGACGAGG
GCAACTTTGACATGGCCGTACGCGCTGTGACGGCCGTCGTGAGGAACAAAAATCACGAC
ATGAAATCATACGAGGACTATTACAAAGCATTTAAGATGCCGATCTGGCAAGCGGCGGA
GCCAAGTGCTGTGCAGATGGAAGCGTCTTTACAGATTAAAGAGCAGATGGGATATGAGT
TCAAGTCTCCTGCCCTGCTGCGGAGTGCCTTCAAGCACCCGTCCTACCCCCGTCAGTTTG
AGAGCGTGCCCAATTATCAGCGCCTCGAGTTCCTCGGTGACGCGCTTCTAGACATGGTCT
GCGTAGACTTTCTCTTCAGGAAGTTTCCCGACG
CCGATCCTCAATGGCTCACTGAACACAAGATGGCCATGGTTTCGAACCACTTCCTCGGAA
GTCTGAGTGTAGAGTTGGGCTTCTACCGGCGTGTCCTTCACTTTAACAGCATCATGGCCA
ATCAAATCAAGGACTACGTCGACGCACTTACTCATGCACGCCAAGAAGCCGAAGCGGTG
GCCCAGATCTCTGGCACAGTCTCGCGAGATTACTGGCTCAACGTGAAGCACCCCCCCAAA
TTCCTCTCAGACGTGGTCGAGGCATACATCGGTGCTATTTTCGTTGATTCAGGATACGATT
ATGGCCAGGTACAGGCGTTCTTCGAGAAGCATATCCGGCCTTTCTTCGCAGACATGGCGC
TATATGATTCCTTTGCCAGCAGCCACCCTGTCACAACGCTGGCGCGTATGATGCAGCAGG
ACTTTGGCTGCCAGGACTGGCGGCTTCTTGTAAGTGAACTGCCGCCGAGCTGCGAAGACG
 
GCGGGGCAGCTGCGATCACTGAGACGGAAGTGATTTGTGGGTTCATGGTCCACGGAAGA
ATCCTGCTACATGCCAAGTCGTCGAGTGGACGGTACGCCAAAGTGGGTGCTGCAAAGAG
AGCGGTCGAGAAGCTCATGGGTCTCGGCAACGACAAAGAGGTCTTTCGGACGGACTTCG
GCTGTGACTGTGACTGTGAAGGTCAAGCAATCTAG
**SEQ ID NO:6 - *Verticillium dahilae* DCL (VAD_00471.1) protein sequence** 
MTTDELSVGLDATGISILADGPENISSSTSTSTTGKEDGYLCINRFTQNTATTQDNQSRDSD
DDEDDCGSHDEADEDSDERQYSMTPERPHKITEKKRADHAAFHDWLQSNSSEIAQSTPQPAQ
NLNHTSTALMVRESENRKIIENPREYQIELFERAKRKNIIAVLPTGSGKTLIAALLLRHTLE
QETADRRAGKPKRIAFFLVEKVALALQQHAVLECNLEFPIDRVCGDMVRSDWIKESWMKRWD
DNMVMVCTAAILQQCLARSFIRMDQINLLVFDEAHHAKGNHPYARIIKDYYITEPDKERRPK
IFGMTASPVDALTDVKIAAAQLEGLLHSEIATIEEDSVSFKQIQKEVVEQDCKYPALEPPFT
TNLHKKIQEQVRYNKNFAKALSNSLEMSSSLGSWCVDRFWQIFLTEETLARLAAQTAQDNIF
ADRAEKERVAIEEVRNIIKQHQFLPITKTLQDLSSKVLCLLGQLELRFSAPTDHKCIIFVEK
RNTAMILAHLLSLPGIGPLYLKPAALVGNPSDNSPLAMSYKEQVMTITKFRRGEYNCLLATS
VAEEGIDIADCNIVIRFDLFNSVIQYIQSKGRARHLNSEYICMAELGNGKHTRAKIQANYDL
SLIRQFCSTLPEDRKIVGWDPEAALHHGERDHKFHIVPSTGAKLTWTGSLVVLSNFASSLQV
NDETLSPSYMVSLIGSEYICEVQLPSKSPILSVSGTLQKNKAEARCSAAFEMCMKLIKGGFI
SSHLQPTFTRKLPAMRNARLAISSKKRERYNMRVKPEVWSRRGPASSLFLTVLKLRTPGALN
RPSQPLALLTREALPELPGVPLFFGNCGRSIAEVVSVAKPMHLDEVRLDSLRVFTLRIFKDV
FSKVYDSQVADLPYFLAPAAHDHSHEFSPNEDPGSLIDWSHLLSTKEVEYLPWDEDHSPSFY
QSKFVIDPYTGSRKLFLRGIRTDLKPTDLVPDGVPEPTFRLWKDVEHTIKEYSISLWAKSRA
RRAGEWLDTQPVVEAELVSLRRNLLDEFADSKHEGSRVCYVILQPLQISTLPVEVVAMAYNF
PAIIHRIESNMIALDACRMLNLRVRPDLALEAMTKDSSNSEEHDQEKIDFQAGMGNNYERLE
FLGDCFLKMATTIALFTRIPDSNEFECHVERMLLICNQNLFNVALKKNLQEYIRSKQFDRRS
WYPQGLKQKAGKAQGAQNSHSLADKSIADVCEAIIGASYLSYTDEGNFDMAVRAVTAVVRNK
NHDMKSYEDYYKAFKMPIWQAAEPSAVQMEASLQIKEQMGYEFKSPALLRSAFKHPSYPRQF
ESVPNYQRLEFLGDALLDMVCVDFLFRKFPDADPQWLTEHKMAMVSNHFLGSLSVELGFYRR
VLHFNSIMANQIKDYVDALTHARQEAEAVAQISGTVSRDYWLNVKHPPKFLSDVVEAYIGAI
FVDSGYDYGQVQAFFEKHIRPFFADMALYDSFASSHPVTTLARMMQQDFGCQDWRLLVSELP
PSCEDGGAAAITETEVICGFMVHGRILLHAKSSSGRYAKVGAAKRAVEKLMGLGNDKEVFRT
DFGCDCDCEGQAI*
**SEQ ID NO:7 - *Verticillium dahilae* DCL (VAD_06945.1) genomic DNA sequence (selected RNAi fragment marked by bolded text)** 
ATCACTCTACGGGTAAAAGCGCTGAGAGAATGATCATGATGAATTTCTATCATCCACGCA
AACAATCGGCACTATCTGTTCCCCACGTCCTGGGACTGACCGCAAGCCCCATAATGCGAT
CTAGGCTCGAAGGCCTTGAGGCACTGGAACAGACACTGGACTCGGTTTGCGTTACGCCC
AGATTGCACCGAGATGACTTAATGACCCATGTCAAAAGGCCCACCGTCTGTTATGTCCAT
TACGAAACGACAGATGCTAAGGATGAGCCCAAGCCGGTCAGCATTTCAAGTCTTCGCGA
AGCATGCAGAAATATGGACATCAGGCAAGATCCATACGTTATCTGTCTAAGAGACAAAG
GCACTGATCGAGCACGACGTGAGCTCATCAAGGTCCTTACAAGCCATAAAACAGATTCG
CAACAGCAAATGAAGTCTTTCTTCAATCAAAGCTTGCGAGTCCTGCGAGATCTCGGGCCC
TGGGCGGCCGAGTACTACATTTGGAAGGTTGTTACAGATTTTCTGGCAATCATTGAAGCA
AGAGATCACCGCATGAATCAACGGAATACCGAAGAAAAGCAGTATCTGGCCAACATCCT
TCGACAAATCAGTATCAGCGAGCCGCCAGTCAGCATGTTGAGTGCTCATAACACGTCGA
ACAAAGTAATGGTGCTCATGGAATACTTGTCATCTAAAGCTACCGATGGTACTGTCGGGA
TCATATTTGTCAAAGAGCGATCAACTGCGGCGATGCTTGCACACGTGATTGAGTCGCATC
CACTGACACAGAATAGGCACTCGAGCGTTGGGGTTGTTGTTGGTGCTTCCACTCATCTGG
TAAGGAAGAAAGACATGTGGGATCTGTCTCGAGCAGCCCACGAGACAGAGCCCCTTCTT
CAGTTCAGATCTGGCCACCTCAATTTGCTCATCGCCACGAGTGTGCTTGAAGAGGGCATC
GACGTTCCTGCCTGCAACCTCGTGATCTGTTTTGATGAGCCCGAGAATCTCAAAGCCTTT
 
GTCCAGCGGCGCGGCCGAGCCCGGAAGAAGGATTCTAGCCTCGTGGTTCTTCTCCCCGGG
ACAGACCACGTGCCTCAGGACTGGGAAAGCATGGAAGCGACAATGAGGACACACTACG
AGAGAGAACAGCGCGAAATACAAATCATGGAGCAGATCGAAGCATCCGAGTCTGCAAA
GTACGAAGAGTACGTTGTCGAGAGTACTAATGCCAGACTCGACTTCGAGAACGCCAAAG
CGCATCTCAGCAACTTTTGTGGGCAGCTCTCTCCCGGGGAGTTTATAGACAAGAGGCCCG
AATACATACCCCGTGTGGTAGACAACGGAGTACCTCCATCTCTGAGGGTCACGGTACTGT
TGCCAAGCTATGTTCCAGCTGCCGTCCGCCATGCTGAGAGTCGTCGAAGCTGGAAGTCGG
AGCATCAGGCCTCAAAGGATGCCGCTTTTCAGGCATACGTGGCTCTTTACAAAGCGGGAC
TGGTCAATGAACACATGCTTCCACTCACGGTAAAAGATATCGTACCCGCAAACGAACCTC
GAGTAGCAACCTTGCAGGTCAATGGCCTCTTGAATGTCTGGCTTGGTATTGCCCAGGCCT
GGATCACGAGCACTGAAACCTGGTTAACTCCAGTGCACCTCCGAGACGCGACGGGATTG
ACGCGAGGAACGTATATCATGAGAATGCCGGTAGCATTGCCGGCACTGCCTTCCACGCC
GGTGTACTTCGATCGCGAAGGACCATGGCTTCTGGATTTTGGCCCACAAGAACGAAAGG
AGAATCTTGAAATGCCTGATCATACTTCAGTGCTGCTTGCACTCCACTTTGGCCATCACTG
GTCTATTGCTCATGGTCAGCAGCAGGTTATCAGCTTCGCTTCACAAGATGGCGA
ACTGAATATCAGGCAATTAAGTGCACGGGGTTTCACAACCGCAGATGCCGACCGAGAGG
AAATGCTGTACCTGGTACGGGACGAGTCAGGATGCCCGTATGTGTACGACCACTTTCTAA
ATGGCAAGCCGTCACTTGAACTTGTTCAACGACCTTTCCGGCGCATCGGGGACTCTCCAG
GCTTTCAAGACGCACCCAGTAACATCCCCTACTTGGCTCTCAGAAAGTGGCCGCGGTACC
TGGCCCTCTTGCACCAACAGAAGGTCAACGATCTACTGCCACAGGCGACAAACAAGAAG
CCATATGCTAGGGTTTATCCGGCACCGTGGGCGAAAGTCGACACGATTCCATTAGATCAT
GCTTACTTTGGGGCGTTGATCCCTTTCATTTCACACATTGTCGAGGTTCGACTGGTTGCAG
AACAGCTTTCCTCGAGCCTACTTCGTGACCTCAATTTCTCAGATCCCTCTCTTGTCCTGGC
GGCCATTAGCACTAAGGGTTCCTTGGAAGCCACAAACTACGAGCGCCTTGAGCTTTTGGG
TGACTCTATCCTCAAGCTTTGCACCACGGCCAATGCCGCCGCTCTGCATGGCTTAGTGTC
GAACTCGAGATTGTGTAGGGCTGCACTGGATGCTGGCCTTGACAAATTTGTTCTAACTGA
AAACTTCACTTGTCGCACGTGGCGCCCTATCTACGTCAACGACATGATGGAAAAGGGTGC
TCGCGACTCAGGACCCCGTATCATGTCGACGAAGACGCTCGCCGATATTGTGGAAGCACT
CATAGGGGCCGCATACATTGACGGTGGCCTCCCAAAGGCACTTGGGTGCATTTCGATCTT
CCTGAGGGAGCTCGATTGGAAACCGTTGCCAGCTTGCCAGGAGATCCTTTACAGTTTGGC
GTCCCCTGATGTGCCTTTGCCGCCAATGCTTGTTCCGCTGGAGGACCTGATCGGCTACAC
GATGCATCTCCTCAAGACTGCTTCGGTCAACGGCGATCTTCTAGGCTTCCTTGCACTCGA
GTGCCATGCCGAGGAAGACGAGGTGATCATTGATATCGATTTTTCTCCTTCCGATACGGA
CTTCAATCCTCAAAATTCCGCCGGGGTGGAACAGAAGCTCAAACAGACACGCCGGAAA
ATCCCCCTTTGGAAGTTTATGCGCCACTCCTCAATAGAGGTTGTGCAGCAGCAGAC
CAAAGCTGCCAGCGTTCATGCCGATCTCCGAGGACAGATCATGCACGCTCTGGAAC
ATGGGTCAAGCTACCCCTGGTCTCTTCTCGCCCGTTTACATCCCGCAAAGTTCTTCT
CCGACATGGTCGAAGCTGTACTGGGTGCCGTCTGGGTCGATTCGGGCGACATGGGC
GCGTGCATTCGTGTGGCGGAACGACTGGGCATTCTGCCTGTGCTCTCCCGACTGGC
AAAGGAGGACGTTCATGTGCTGCATCCGAAGCAAGAGCTGGGAGAGATCGCTGGTCCC
CGGACAGTCAAATATCTCCTCACTTTGCCCGAGGACGCAGCCGGCCTGCAAAGTGCAAC
AAGAAAATATGCCTGCAAGGTCATGGTCGGGGATCGCTGTGTTGCAGAGGTGGATGACG
GGGTCGCTCGAGATGAGGTTGAGACAAAGGCTGCAGAGGTTGCGGTACAGACCTTGAAG
AATGAACAGGCTGACGCGAAACAAGTAGCAGAACACTAA
**SEQ ID NO:8 - *Verticillium dahilae* DCL (VAD**_**06945.1) protein sequence** 
MIMMNFYHPRKQSALSVPHVLGLTASPIMRSRLEGLEALEQTLDSVCVTPRLHRDDLMTHVK
RPTVCYVHYETTDAKDEPKPVSISSLREACRNMDIRQDPYVICLRDKGTDRARRELIKVLTS
HKTDSQQQMKSFFNQSLRVLRDLGPWAAEYYIWKVVTDFLAIIEARDHRMNQRNTEEKQYLA
NILRQISISEPPVSMLSAHNTSNKVMVLMEYLSSKATDGTVGIIFVKERSTAAMLAHVIESH
PLTQNRHSSVGVVVGASTHLVRKKDMWDLSRAAHETEPLLQFRSGHLNLLIATSVLEEGIDV
PACNLVICFDEPENLKAFVQRRGRARKKDSSLVVLLPGTDHVPQDWESMEATMRTHYEREQR
EIQIMEQIEASESAKYEEYVVESTNARLDFENAKAHLSNFCGQLSPGEFIDKRPEYIPRVVD
NGVPPSLRVTVLLPSYVPAAVRHAESRRSWKSEHQASKDAAFQAYVALYKAGLVNEHMLPLT
 
VKDIVPANEPRVATLQVNGLLNVWLGIAQAWITSTETWLTPVHLRDATGLTRGTYIMRMPVA
LPALPSTPVYFDREGPWLLDFGPQERKENLEMPDHTSVLLALHFGHHWSIAHGQQQVISFAS
QDGELNIRQLSARGFTTADADREEMLYLVRDESGCPYVYDHFLNGKPSLELVQRPFRRIGDS
PGFQDAPSNIPYLALRKWPRYLALLHQQKVNDLLPQATNKKPYARVYPAPWAKVDTIPLDHA
YFGALIPFISHIVEVRLVAEQLSSSLLRDLNFSDPSLVLAAISTKGSLEATNYERLELLGDS
ILKLCTTANAAALHGLVSNSRLCRAALDAGLDKFVLTENFTCRTWRPIYVNDMMEKGARDSG
PRIMSTKTLADIVEALIGAAYIDGGLPKALGCISIFLRELDWKPLPACQEILYSLASPDVPL
PPMLVPLEDLIGYTMHLLKTASVNGDLLGFLALECHAEEDEVIIDIDFSPSDTDFNPQNSAG
VEQKLKQTRRKIPLWKFMRHSSIEVVQQQTKAASVHADLRGQIMHALEHGSSYPWSLLARLH
PAKFFSDMVEAVLGAVWVDSGDMGACIRVAERLGILPVLSRLAKEDVHVLHPKQELGEIAGP
RTVKYLLTLPEDAAGLQSATRKYACKVMVGDRCVAEVDDGVARDEVETKAAEVAVQTLKNEQ
ADAKQVAEH*
**SEQ ID NO:9 - RNAi fragment from *B. cinerea* DCL1 cDNA** 
TGCGGAAGAACTTGAAGGTTTGCTACACAGTCAAATATGTACTGCAGAAGATCCCAGCTT
GCTGCAGTACTCAATCAAAGGTAAACCTGAGACTCTTGCCTACTATGATCCCTTGGGCCC
GAAATTCAATACTCCTCTTTATCTTCAAATGCTCCCGCTTCTAAAAGACAATCCTATCTTT
CGGAAGCCATTTGTATTTGGGACAGAAGCCAGTAGAACTCTAGGATCTTGGTGTGTTGAC
CAGATCTGGACTTTCTGTC
**SEQ ID NO:10 - RNAi fragment from *B. cinerea* DCL2 cDNA** 
TCTTTAAGTGATATCGAGGAGACTTTGGATGCCATTTGCTGCACGCCAAAAATACATCGA
GCAGATCTTCGCCTTCGAGTAAAGCTACCACTTCTATCTATTATCTACTATACCCCAGAGT
CAAATATCATCGTGACGAAAACTGTGGCGAGCCTGAGAAAGATTGTGCAAAGTCTCAAC
ATTTTCGAAGACCCCTACGTTTTGACACTAAAAAGGAGTGATAGCGAAAAAAGTCAACG
TGAGCTGGCGAAAGTACTCAAGAGTTTTAAGACATATAGTCAAACCCAATTAAAGTC
**SEQ ID NO:11 - RNAi fragment from *V. dahliae* DCL (VDAG_00471) cDNA** 
GGCAAGCCCAAGAGAATCGCCTTTTTCCTCGTGGAAAAGGTTGCTCTTGCCCTCCAACAG
CACGCGGTTCTGGAGTGCAATCTGGAATTTCCCATTGACCGGGTATGCGGTGACATGGTA
CGGTCGGACTGGATCAAGGAGTCATGGATGAAAAGATGGGATGACAACATGGTCATGGT
CTGCACCGCCGCCATCCTTCAGCAATGCCTTGCCAGATCATTCATCCGCATGGATCAGAT
CAACCTGCTTGTCTTCGATGAAGCACATCACGCCAAGGGAAATCATCCGTACGC
**SEQ ID NO:12 - RNAi fragment from *V. dahliae* DCL (VDAG 06945.1) cDNA** 
ACAGACACGCCGGAAAATCCCCCTTTGGAAGTTTATGCGCCACTCCTCAATAGAGGTTGT
GCAGCAGCAGACCAAAGCTGCCAGCGTTCATGCCGATCTCCGAGGACAGATCATGCACG
CTCTGGAACATGGGTCAAGCTACCCCTGGTCTCTTCTCGCCCGTTTACATCCCGCAAAGTT
CTTCTCCGACATGGTCGAAGCTGTACTGGGTGCCGTCTGGGTCGATTCGGGCGACATGGG
CGCGTGCATTCGTGTGGCGGAACGACTGGGCATTCTGCCTGTGCTCTCCCGACTGGCAAA
GGAGGACGTTCATGTGCTG
**SEQ ID NO:13 - LTR for siR3**
   >B. cinerea (B05.10) Botrytis cinerea supercontig 1.56 [DNA] 218751-219771 - 
CTCCTGGATCAGGCAGATGAATTAGGGAACTGATTTCGACCTTCCAGAGTTCTCTTTGCG
TGATGGGTCACTTGGGTTTGGTTGTCGGTATGCTGTGGGTTCGGAGGAGTTGTCCTTTCT
GGTTTCTTTGTTGGATAGTCCTTTTTGGGTAGCTTGGTGTGATGCATGCGTTCTGGGTGT
GGGTCTCGTGAGGTCTTTTTGTATCAAGTATTTTTAAGCTTTTTTCTTGTTCTCTTCTTT
TTCTGTATTGGTAATGCTTCTTCTTTATGATATTCTCCCATCGCTGCTTTCGCATTTTCT
AGGTTGTAGGGTGCTTCCCACGTGTCTTCCGCCGGTTGGTAGCCCTTCCATCGCACCAGG
TATTGCACACCTCTGCCTCTTTTTCTATGTGCTAAAATCCTTTCCACCTCATGCTCTATG
TGATCGTCAATTTCTTCTGGCGGCGGCGGGTCGGCGGTACCCTGTCGTTCGTGCCATGGT
 
TCGAGTAAAGAGACGTGGAATACATTGTGGATCTTGTAGGTGGGCGGTAATCTAAGTT
CG
TATGCTTGCCCGCTGGTTTTTATACCCGTCACGACAAAGGGGCCTATAAATCGATCGGAG
AATTTTTTCTTAGGTCGCAGTTGTTTAATGTTCTTTGTGCTTAGCATCACCTTGTCCCCG
ATGCTATATCGCTGTGGTGACCCCTTCGTAGTGTTCTTGTTTTTCTGATTGGTTGCCGAT
TTCCAGAATTCTTTCAGCTTTTCTCTTTCTTTCTCTAAAGCGTCGATGCGCTCGCGTGCT
GCCGGCGCCCTTCCCTCTAAATCGGCGTCCTCGCCGATATAATGGAATGTGGGTTGGAAC
CCATACATAGCCTGGAATGGGCTTGTATTGGTTGTACTGTGCCATGTCGCGTTATATGTG
AATTCAGCAAGGGGCAATAGCGATGCCCAGTCGTCTTGCCTATAGTTGGTGTAGCAACTT
ATATAGTGAATCAAATTTTGGTTTTGTCGTTCGGTTTGACCGTCGGTCTGCGGGTGGAAC
G
**SEQ ID NO:14 - LTR for siR5**
   **> BC1G_08572.1 retrotransposable element Tf2 1 protein type 1** (Transcript:BC1T_08572) 
ATGGCATCCAGAGCTACCGCCACAGGTCAATCTGCCGGAGACACCAACGACATCGAGAT
GACCGACGCCCCAAAGGAGATCACTATCAACGAAACCCTTAAGATCGCCTTACCAGACA
AGTACCAAGGTAGTCGACAAGAGCTCGATACTTTCCTCTTACAACTTGAGATCTACTTTC
GATTCAATGAGGACAAGTTCACTACCAAGGAATCCAAGAGTATATGGGCCGCATCATAC
CTTCGAGGTGAAGCAACCAAATGGATCCAACCATATTTGCGCGACTATTTCGAGCATGAC
GATAAGGATCGTATGCAACCCACCCGAACAATCTTCAATAGTTTTGAAGGATTTAAGACA
GAGATTCGTAGAATCTTCGGAAATTCCAACGAGTTAGAGGTAGCGGAAGATAAGATCTT
CAACCTCAAGCAGACAGGATCAGCATTGAAATATGCTACGGAATTTCGAAGATATGCTG
GAACAACCAAGTGGGACGAAATCGCTATCATGAGTCACTACCGCAAGGGACTCAAACCA
GAAGTCAGACTGGAATTAGAAAGATCTGCCGAGAGTACAGATCTGAACGATCTAATTCA
GGACTCCATCGAATCAGATGATCGTCTCTACAGATATCGACAAAGCCAGAGATCATACA
AACCCCAAGGAAATCAGAAGCAAGGGCGTTACCGCAAAAATGAGGGTAGACCACGTTA
CAATCCACAGAGATACGGAGACCCCATGGAACTAGACGCCACGCACTACACAAACGGGA
ACGATGACTCGGAAAAGAGACGAAGACGAGAAAACAACTTATGCTTTGAATGTGGAAA
AGCAGGGCACCGAGCAGCAGACTGCCGAAGCAAGAAGACAGGAGGAAAAAGGGGCAA
CTTCAAACCTAAGTTCGGCAAAGGCCAACTTAACGCTACCTTTACAATCCCAGAAAATCC
AACTAAATCCGAAAATACTGAGACTTTCACCGTTGAGGAATTCCAGCAATTACTAAAGG
AATTACCACGAAATCAAGAGGGCATGAATGCAATAGACTTATGGGAGCAAGAGTATTAC
AGAACCCCAACACCCTCTGTGACAGAAGAAAGTCATCAGGACGAGGCAGAAGCAGACC
ACGCCACGATGAGCTGGACAGCTTGCTATGATGAATTCTGCGGAATCCATCGATCAGATA
AAGAAGCAACCGGATGGTCCCCTAAGAAGAGAAAGACGAAGAACCATCAGAATAATGT
AACATGCGAGGATTTAACTCCCAATATAACTTCGCAAGAAGTTCGCAAAGTTACCCAGC
AGTTAAATGCTACGGGACAGGCAGGACAGATATACTGCAAGGTTCAGATAAATGGACAC
ATACAATCAGCCATGATAGATTCAGGGGCTACAGGAAATTTTATTGCACCGGAAGCTGA
GACAATCCCAATACGAATGGGCATAACCCAACATACAGAGGTTATACAGCTTGACGTTG
TGCCATTGGGCCAACAACAGATCATCTTAGGAATGCCATGGTTAAAGGCACATAATCCG
AAAATAGATTGGGCACAAGGAATTGTGACATTTGATCAGTGCAAAAGCGGTCACAGGGA
CACGCTAGAGGCGTTCGCGAGACGTAACACGCGCCAAGGAGAGTTGAACGCGAACAAC
ACCGGCGACGTAGGACACCCAGTCCAGGGTCCTCCATTAAGAGCGAAGGCCAGTACACC
TCCTCTACAAATGCAGAAGCCAACGACACGGCACGAAATCGCAATCGAGGCAAAAGAA
AAGCCTACGATACCAGAACAGTACAAGAATTATGAACATGTTTTCAAAGAACCAGGGAT
CCATGAGGCTTTACCGGAACACAAGCCATGGGATCATGAGATAATATTGGAGGAAGGCA
AGATGCCTGTGCACACCCCAATTTATTCAATGTCAGCCGATGAGTTAAAGAGGCTCAGAG
AGTACATCGACGACAATTTAGCCAAGGGATGGATCAGGGAATCCGCGTCCCAAGTGGCC
AGTCCAACTATGTGGAAGGATCGATATCCACTTCCATTAGCTACGGAATTAAGAGATCGA
TTAGGCGGAGCTACGATATTTACCAAGATGGACCTACGTAATGGTTACCACTTGATCAGA
ATGAAGGAAGGCGAAGAATGGAAAACCGCTTCAAAACAAGATACGGGCTATACGACTA
CTTTCATGAGGCTTATGAACAATGTGTTGTCACAATATTTGGATACTTGCTGTATATGCTA
CTTGGACGACATCCTAGTATATTCAAACAACAAGGTTCAACACATTAAGGACGTTAG
CAACATCCTCGAAAGCCTATCCAAGGCAGACTTGCTGTGCAAACCAAGCAAATGCGAAT
 
TCCATGTCACAGAGACAGAATTCTTGGGATTCACCGTATCAAGCCAAGGGCTCAAGATG
AGCAAAGGCAAGGTTAAGGCAGTGCTCGAATGGAAGCAGCCGACCACAATCAAGGAAG
TACAATCCTTTCTAGGGTTCGTCAACTTCTACAGAAGATTCATCAAGGGTTATTCAGGGA
TTACTACACCCTTGACCACGTTAACCAGAAAAGATCAAGGAAGCTTCGAATGGACTGCC
AAAGCACAGGAGTCATTCGATACGCTCAAACAAGCAGTGGCAGAAGAGCCAATACTGTT
GACTTTTGACCCAGAGAAAGAAATCATAGTGGAGACGGACTCCTCGGATTTCGCTATAG
GAGCAGTTCTGAGCCAACCGGGCCAGAATGGAAAATACCAGCCAATCGCATTCTACTCC
CGAAAACTATCACCAGCCGAATTGAATTACGAGATATATGACAAAGAATTACTGGCGAT
AGTCGATGCATTTAGAGAATGGCGAGTGTATTTGGAAGGATCGAAATACACGGTACAGG
TGTATACAGATCATAAGAACTTGGTTTACTTCACCACAACGAAGCAGTTAAACAGACGA
CAGGTCAGATGGTCGGAGACCATGGCCAACTACAATTTCAGAATTTCATATGTCAAAGG
ATCAGAAAACGCTAGAGCCGACGCTCTTAGCCGAAAACCAGAATATCAAGAAAACAAA
ACGTACGAGTCATACGCTATATTCAAGAAAGACGGCGAATCACTGGTTTACAATGCACC
ACAGCTTGCAGCAACACACCTGTTGGAAGACAACCACCTCAGGAAACAGATCCAATCAC
ACTACGACAAGGATGCTACTGCCACACGCATACGCAAGACAATAGAACCAGGATTCACT
ATAGAAAATGATACCATATACTTTCATGGAAAAGTATACATTCCGAGTCAAATGACCA
AGGAATTTGTGACGGAACAACACGGGTTGCCGGCACATGGACACCAAGGAATTGCAAGG
ACATTTGCAAGAATACGGGAAATCAGTTACTTCCCACGAATGAGAACGATAGTTGAAGA
AGTTGTTGGAAATTGTGACACCTGCATACGAAACAAGTCATCACGACATGCTCCGTATGG
TCAGCTCCAGACCCCAGACATGCCTTCTCAGCCATGGAAGTCCATCACATGGGACTTTGT
GGTCAAACTACCACTCTCAAAAGATCCTACTACAGGAATTGAGTACGACGCGATACTCA
ATATAGTAGACAGGCTAACGAAATTTGCATATATGATACCATTCAAGGAAACATGGGAT
GCTGAGCAACTAGCATATGTGTTCCTAAGGATCATAGTAAGCATACACGGAGTACCAGA
TGAGATAATCTCGGATCGAGACAAGCTCTTTACCTCGAAATTCTGGACTACCTTATTAGC
ACTTATGGGTATCAAGAGAAAGCTATCGACATCTTTCCACCCACAAACAGATGGTCAAA
CAGAGAGGACCAATCAGACAATGGAAGCATATCTTAGATGCTATCGTATAAAATCCCGA
TACCAC AAGAAGTTAATGCCGAATCAGCGATAG
**SEQ ID NO:15 - LTR for siR5**
   > BC1G_15284.1 - enzymatic polyprotein 
ATGGCATCCAGAGATATCGCCACAGGTCAATCTGCCGGAGACACCAACGACATCGAGAT
GACCGATGCCCCAAAAGAGATCACTATCAACGAAACACTTAAGATCGCCTTACCAGACA
AGTACCAAGGTAGTCGACAAGAACTCGATACTTTCCTCTTACAACTTGAGATCTACTTCC
GATTCAATGAGGACAAGTTCACTACCAAGGAATCCAAGAGTATATGGGCCGCATCATAC
CTTCGAGGTGAAGCAACCAAATGGATCCAACCATATTTGCGCGACTATTTCGAGCATGAC
GATAAGGATCGCATGCAACCCACCCGAACAATCTTCAATAGTTTTGAAGGATTTAAGAC
AGAGATTCGTAGAATCTTCGGAAATTCCAACGAGTTAGAGGTAGCGGAAGATAAGATCT
TCAACCTCAAGCAGACAGGATCAGCATTGAAATATGCTACGGAATTTCGAAGATATGCT
GGAACAACCAAGTGGGACGAAATCGCTATCATGAGTCACTACCGTAAGGGACTCAAACC
AGAAGTCAGACTGGAATTAGAAAGATCTGCCGAGAGTACAGATCTGAACGATCTAATTC
AGGACTCCATCGAATCAGATGATCGTCTCTACAGATATCGACAAAGCCAGAGATCATAC
AAACCCCAAGGAAATCAGAAGCAAGGGCGTTACCGCAAGAATGAGGGTAGACCACGTT
ACAACCCACAGAGATACGGAGACCCCATGGAACTAGACGCTACGCACTACACAAACGGG
AACGATGACTCAGAAAAGAGACGAAGACGAGAAAACAACTTATGCTTTGAATGTGGAA
AAGCAGGGCACCGAGCAGCAGAATGCCGAAGCAAGAAGACAGGAGGAAAAAGGGGCA
ACTTCAAACCTAAGTTCGGCAAGGGCCAACTTAATGCCACCTTTGCAATCTCAGAAAACT
CAACTAAACCCGAAAATACTGAGACTTTCACCGTTGAGGAATTCCAGCAATTATTAGAG
GAATTACCACGAAACCAAGAGGGCATGAATGCAATAGACTTATGGGAACAAGAGTATTA
CAGAACTCCAACACCCTCTGTGACAGAAGAAAGTCACCAGGACGAGGCAGAAGCGGAC
CACGCCACGATAAGCTGGACAGCTTGCTATGACGAATTCTGCGGAATCCATCGATCAGAT
AAAGAAGCAACCGGATGGTTCCCTAAGAAGAGAAAGACGAAGAACCATCAGAATAATG
TAACATGCGAGGATTTAACTCCCAATATAACTTCGCAAGAAGTTCGCAAAGTTACCCAGC
AGTTAAATGCTACGGGACAGGCAGGACAGATATACTGCAAGGTTCAGATAAATGGACAC
ATACAATCAGCCATGATAGATTCAGGGGCTACAGGAAATTTTATTGCACCAGAAGCGGC
AAAGTACTTGGAAATACCACTTCAGAGGAAACAATACCCCTATCGATTGCAGTTAGTTGA
 
CGGACAGCTAGCAGGGTCTGACGGAAAGATTTCGCAGGAGACAATCCCAGTACGAATGA
GCATAACCCAACATACAGAGGTTATACAGCTTGATGTTGTGCCATTGGGCCAACAACAG
ATCATCTTAGGAATGCCATGGTTAAAGGCACATAATCCGAAAATAGATTGGGCACAAGG
AGTTGTGACATTTGATCAGTGCAAAAGCGGTCACAGGGACACGATAGAGGCGTCCGCGA
GACGTAACACGCGCCAAGGAGAGTTGAACGCGAACAACACCGGCGACGTAGGACACCC
AGTCCAGGGTCCTCCATTAAGAGCGAAGGCCAGTACACCTCCTCTACAAATGCAGAAGC
CAACGACACGGCACGAAATCGCAATCGAGGCAAAAGAAAGGCCTACGATACCAGAACA
GTACAAGAAATATGAACATGTTTTCAAAGAACCAGGGATCCATGAGGCTTTACCGGAAC
ACAAGCCATGGGATCATGAGATAATATTGGAGGAAGGCAAGATGCCTGTGCACACCCCA
ATTTATTCAATGTCAGCCGATGAGTTAAAGAGGCTCAGAGAGTACATCGACGACAATTTA
GCCAAGGGATGGATCAGGGAATCCGCGTCCCAAGTGGCCAGTCCAACTATGTGGGTACC
CAAGAAGGATGGACCCGATAGACTAGTTGTAGACTATAGAAAGCTTAACGCACTCACTA
AGAAGGATCGATATCCACTTCCATTAGCTACGGAATTAAGAGATCGATTAGGCGGAGCT
ACGATATTCACCAAGATGGACCTACGTAATGGTTACCACTTGATCAGAATGAAGGAAGG
CGAAGAATGGAAAACCGCTTTCAAAACAAGATACGGGCTATACGAGTACCAAGTTATGC
CATTCGGGCTAACCAACGCACCAGCTACTTTCATGAGGCTTATGAACAATGTGTTGTCAC
AATATTTGGATACTTGCTGTATATGCTACTTGGACGACATCCTAGTATATTCAAACAACA
AGGTTCAACACATTAAGGACGTTAGCAACATCCTCGAAAGCCTATCCAAGGCAGACTTG
CTGTGCAAACCAAGCAAATGCGAATTCCATGTCACAGAGACAGAATTCTTGGGATTCAC
CGTATCAAGCCAAGGGCTCAAGATGAGCAAAGACAAGGTTAAGGCAGTGCTCGAATGGA
AGCAGCCGACCACAATCAAGGAAGTACAATCCTTTCTAGGGTTCGTCAACTTCTACAGAA
GATTTATCAAGGGTTATTCAGGGATTACTACACCCTTGACCACGTTAACCAGAAAAGATC
AAGGAAGCTTCGAATGGACTGCCAAAGCACAGGAGTCATTCGATACGCTCAAACAAGCA
GTGGCAGAAGAACCAATACTGTTGACTTTTGACCCAGAGAAAGAAATCATAGTGGAGAC
GGACTCCTCGGATTTCGCTATAGGAGCAGTTCTGAGCCAACCGGGCCAGAATGGAAAAT
ACCAGCCAATCGCATTCTACTCCCGAAAACTATCACCAGCCGAATTGAATTACGAGATAT
ATGACAAAGAATTACTGGCGATAGTCGATGCATTTAGAGAATGGCGAGTGTATTTGGAA
GGATCGAAATACACGGTACAGGTGTATACAGATCATAAGAACTTGGTTTACTTCACCACA
ACGAAGCAGTTAAACAGACGACAGGTCAGATGGTCGGAGACCATGGCCAACTACAATTT
CAGAATTTCATATGTCAAAGGATCAGAAAACGCTAGAGCCGACGCTCTTAGCCGAAAAC
CAGAATATCAAGAAAACAAAACGTACGAGTCATACGCTATATTCAAGAAAGACGGCGAA
TCACTGGTTTACAATGCACCACAGCTTGCAGCAACACACCTGTTGGAAGACAACCACCTC
AGGAAACAGATCCAATCACACTACGACAAGGATGCTACTGCCACACGCATACGCAAGAC
AATAGAACCAGGATTCACTATAGAAAATGATACCATATACTTTCATGGAAAAGTATACA
TTCCGAGTCAAATGACCAAGGAATTTGTGACGGAACAACACGGGTTGCCGGCACATGG
ACACCAAGGAATTGCAAGGACATTTGCAAGAATACGGGAAATCAGTTACTTCCCACGAA
TGAGAACGATAGTTGAAGAAGTTGTTGGAAATTGTGACACCTGCATACGAAACAAGTCA
TCACGACATGCTCCGTATGGTCAGCTCCAGACCCCAGACATGCCTTCTCAGCCATGGAAG
TCCATCACATGGGACTTTGTGGTCAAACTACCACTCTCAAAGGATCCTACTACAGGAATT
GAGTACGACGCGATACTCAATATAGTAGACAGGCTAACGAAATTTGCATATATGATACC
ATTCAAGGAAACATGGGATGCTGAGCAACTAGCATATGTGTTCCTAAGGATCATAGTAA
GCATACACGGAGTACCAGATGAGATAATCTCGGATCGAGACAAGCTCTTTACCTCAAAA
TTCTGGACTACCTTATTAGCACTTATGGGTATCAAGAGAAAGCTATCGACATCTTTCCAC
CCACAAACAGATGGTCAAACAGAGAGGACCAATCAGACAATGGAAGCATATCTTAGATG
CTATGTAAATTATCGACAAGACAATTGGGTAGAGCTATTACCCATGGCACAGTTCGCATA
CAATACATCAGAAACGGAAACCACGAAAATCACACCAGCACGAGCTAATTTTGGGTTTA
ATCCACAAGCGTATAAAATCCCGATACCACAAGAAGTTAATGCCGAATCAGCGATAGTA
CAAATCGAACAGCTGAAAGATCTCCAAGAGCAACTGGCTCTTGATCTAAGATTCATATCT
TCCAGAACAGCAGCGTACTACAATACGAAACGTAGTATGGAACCTACGCTTAAAGAGGG
GGATAAAGTTTATTTGCTACGACGAAACATCGAAACCAAGAGACCAAGCAATAAACTCG
ACCACAGGAAACTAGGACCATTCAAGATTGATAAGGTAATAGGAACGGTTAATTATCGA
TTGAAATTACCAGACACAATGAATATCCACCCAGTATTCCACATATCCTTGCTCGAACCA
GCACCACCAGGAGCGCCAAATGCGCCATTTACAGAAATTGAACCAG
TCAACCCAAACGCCATATACGATGTCGAAACAATACTAGACTGCAAATACGTCAGAAAC
AAGGTCAAGTATTTGATCAAATGGTTAGACTACCCACATTCAGAAAACACATGGGAACT
 
CAAGGAAGATCTCAGCTGCCCTGAGAAGCTACGGGCATTCCACCTGAAGTACCCACACC
TGCCAATAAAGCCTCAAGATCCGCTTCGGACAACTCAGGCAAAGAAGGATCGAAGAAAT
CGAAGGAAGAAGAATCAATAG
**SEQ ID NO:16 - LTR for siR5**
   **>BC1G 04408.1 retrotransposable element Tf2 1 protein type 1** 
ATGGCATCCAGAGCTACCGCCACAGGTCAGTCTACCGGAGATACCAACGACATCGAGAT
GACCGATGCCCCAAAGGAGATCACTATCAACGAAACACTTAAGATCGCCTTACCAGACA
AGTACCAAGGTAGTCGACAAGAGCTCGATACTTTCCTCTTACAACTTGAGATCTACTTCC
GATTCAATGAGGACAAGTTCACTACCAAGGAATCCAAGAGCATATGGGCTGCATCATAC
CTCCGAGGTGAAGCAACCAAATGGATTCAACCATATTTGCGCGACTATTTCGAGCATGAC
GATAAGGATCGCATGCAACCCACCCGAACAATCTTCAATAGTTTTGAAGGATTTAAGAC
AGAGATTCGTAGAATCTTCGGAAATTCCAACGAGTTAGAGGTAGCGGAAGATAAGATCT
TCAACCTCAAGCAGACAGGATCAGCATTGAAATATGCTACGGAATTTCGAAGATATGCT
GGAACAACCAAGTGGGACGAAATCGCTATCATGAGTCACTATCGTAAGGGACTCAAACC
AGAAGTCAGACTGGAATTAGAAAGATCTGCCGAGAGTACAGATCTGAACGATCTAATTC
AGGACTCCATCGAATCAGATGATCGTCTCTACAGATATCGACAAAGCCAGAGATCATAC
AAACCCCAAGGAAATCAGAAGCAAGGGCGTTACCGCAAAAATGAGGGTAGACCACGTT
ACAATCCACAGAGATACGGAGACCCCATGGAACTAGACGCCACGCACTACACAAACGGG
AACGATGACTCGGAAAAGAGACGAAGACGAGAAAACAACTTATGCTTTGAATGTGGAA
AAGCAGGGCACCGAGCAGCAGACTGCCGAAGCAAGAAGACAGGAGGAAAAAGGGGCA
ACTTCAAACCTAAGTTCGGCAAAGGCCAACTTAACGCTACCTTTACAATCCCAGAAAATC
CAACTAAATCCGAAAATACTGAGACTTTCACCGTTGAGGAATTCCAGCAATTACTAAAG
GAATTACCACGAAATCAAGAGGGCATGAATGCAATAGACTTATGGGAGCAAGAGTATTA
CAGAACCCCAACACCCTCTGTGACAGAAGAAAGTCATCAGGACGAGGCAGAAGCAGAC
CACGCCACGATGAGCTGGACAGCTTGCTATGATGAATTCTGCGGAATCCATCGATCAGAT
AAAGAAGCAACCGGATGGTTCCCTAAGAAGAGAAAGACGAAGAACCATCAGAATAATG
TAACATGCGAGGATTTAACTCCCAATATAACTTCGCAAGAAGTTCGCAAAGTTACCCAGC
AGTTAAATGCTACGGGACAGGCAGGACAGATATACTGCAAGGTTCAGATAAATGGACAC
ATACAATCAGCCATGATAGATTCAGGGGCTACAGGAAATTTTATTGCACCGGAAGCTGT
AAAGTACTTGGGAATACCACTTCAAACGAAACAACACCCCTATCGATTGCAGGACACGC
TAGAGGCGTCCGCGAGACGTAACACGCGCCAAGGAGAGTTGAACGCGAACAACACCGG
CGACGTAGGACACCCAGTCCAGGGTCCTCCATTAAGAGCGAAGGCCAGTACACCTCCTC
TACAAATGCAGAAGCCAACGACACGGCACGAAATCGCAATCGAGGCAAAAGAAAGGCC
TACGATACCAGAACAGTACAAGAAATATGAACATGTTTTCAAAGAACCAGGGATCCATG
AGGCTTTACCAGAACACAAGCCATGGGATCATGAGATAATATTGGAGGAAGGCAAGATG
CCTGTGCACACCCCAATTTATTCAATGTCAGCCGATGAGTTAAAAAGGCTCAGAGAATAC
ATCGACGACAATTTAGCCAAGGGATGGATCAGGGAATCCGCGTCCCAAGTGGCCAGTCC
AACTATGTGGGTACCCAAGAAGGATGGACCCGATAGACTAGTTGTAGACTATAGAAAGC
TTAACGCACTCACTAAGAAGGATCGATATCCACTTCCATTAGCTACGGAATTAAGAGATC
GATTAGGCGGAGCTACGATATTTACCAAGATGGACCTACGTAATGGTTACCACTTGATCA
GAATGAAGGAAGGCGAAGAATGGAAGACCGCTTTCAAAACAAGATACGGGCTATACGA
GTACCAAGTTATGCCGTTCGGGCTAACCAACGCACCAGCTACTTTCATGAGGCTTATGAA
CAATGTGTTGTCACAATACTTGGATACTTGCTGTATATGCTACTTGGACGACATCCTAGT
ATATTCAAACAACAAGGTTCAACACATTAAGGACGTTAGCAACATCCTCGAAAGCCT
ATCCAAGGCAGACTTGCTGTGCAAACCAAGCAAATGCGAATTCCATGTCACAGAGACAG
ACTTCTTGGGATTCACCGTATCAAGCCAAGGGCTCAAGATGAGCAAAGACAAGGTTAAG
GCAGTGCTCGAATGGAAACAGCCAACCACAATCAAGGAGGTACAATCCTTTCTAGGGTT
CGTCAACTTCTACAGAAGATTTATCAAGGGTTATTCAGGGATTACTACACCCTTGACCAC
GTTAACCAGAAAAGATCAAGGAAGCTTCGAATGGACTGCCAAAGCACAGGAGTCATTCG
ATACGCTCAAACAAGCAGTGGCAGAAGAGCCAATACTATTGACTTTTGACCCAGAGAAA
GAAATCATAGTGGAGACGGACTCCTCGGATTTCGCTATAGGAGCAGTTCTGAGCCAACC
GGGCCAGAATGGAAAATACCAGCCAATCGCATTCTATTCCCGAAAACTATCACCAGCTG
AGTTGAATTACGAGATATATGACAAAGAATTGCTGGCGATAGTCGATGCATTTAGAGAA
TGGCGAGTATATTTGGAAGGATCGAAATACACAGTACAGGTGTATACAGATCATAAGAA
 
CTTGGTTTACTTCACCACAACGAAGCAGTTAAACAGACGACAGGTCAGATGGTCGGAGA
CCATGGCCAACTACAATTTCAGAATTTCATATGTCAAAGGATCAGAAAACGCTAGAGCC
GACGCTCTTAGCCGAAAACCAGAATATCAAGAAAACAAAACGTACGAGTCATACGCTAT
ATTCAAGAAAGACGGCGAATCACTGGTTTACAATGCACCACAGCTTGCAGCAACACACC
TGTTGGAAGACAACTACCTTAGGAAACAGATCCAATCACACTACGACAAGGATGCTACT
GCCACACGCATACGTAAGACAATAGAACCAGGATTCACTATAGAAAATGATACCATATA
CTTTCATGGAAAAGTATACATTCCGAGTCAAATGACCAAGGAATTTGTGACGGAACAA
CACGGATTGCCGGCACATGGACACCAAGGAATTGCAAGGACATTTGCAAGAATACGGGA
AATCAGTTACTTCCCACGAATGAGAACGATAGTTGAAGAAGTTGTTGGAAATTGTGACA
CCTGCATACGAAACAAGTCATCACGACATGCTCCGTATGGTCAGCTCCAGACCCCAGAC
ATGCCTTCTCAGCCATGGAAGTCCATCACATGGGACTTTGTGGTCAAACTACCACTCTCC
AAGGATCCTACTACAGGAATTGAGTACGACGCGATACTCAATATAGTAGACAGGCTAAC
GAAATTTGCATATATGATACCATTCAAGGAAACATGGGATGCTGAGCAACTAGCATATG
TGTTCCTTAGGATCATAGTAAGCATACACGGAGTACCAGATGAGATAATCTCGGATCGA
GACAAGCTCTTTACCTCGAAATTCTGGACTACCTTATTAGCACTTATGGGTATCAAGAGA
AAGCTATCGACATCTTTCCACCCACAAACAGATGGTCAAACAGAGAGGACCAATCAGAC
AATGGAAGCATATCTTAGATGCTATCGTATAAAATCCCGATACCACAAGAAGTTAATGCC
GAAT CAGCGATAG
**SEQ ID NO:17 - LTR for siR5**
   >BC1G_12842.1 retrotransposable element Tf2 1 protein type 1 (Transcript:BC1T_12842) 
ATGGCATCCAGAGCTACCGCCACAGGTCAGCCTACCGGAGATACCAACGACATCGAGAT
GACCGATGCCCCAAAGGAGATCACTATCAACGAAACCCTTAAGATCGCCTTACCAGACA
AGTACCAAGGTAGTCGACAAGAGCTCGATACTTTCCTCTTACAACTTGAGATCTACTTTC
GATTCAATGAGGACAAGTTCACTACCAAGGAATCCAAGAGTATATGGGCCGCATCATAC
CTTCGAGGTGAAGCAACCAAATGGATCCAACCATATTTGCGCGACTATTTCGAGCATGAC
GATAAGGATCGCATGCAACCCACCCGAACAATCTTCAATAGCTTTGAAGGATTTAAGAC
AGAGATTCGTAGAATCTTCGGAAATTCCAACGAGCTAGAGGTAGCGGAAGATAAGATCT
TCAACCTCAAGCAGACAGGATCAGCATTGAAATATGCTACGGAATTTCGAAGATACGCT
GGAACAACCAAGTGGGACGAAATCGCTATCATGAGTCACTACCGCAAGGGACTCAAACC
AGAAGTCAGACTGGAATTAGAAAGATCTGCCGAGAGTACAGATCTGAACGATCTAATTC
AGGACTCCATCGAATCAGATGATCGTCTCTACAGATATCGACAAAGCCAGAGATCATAC
AAACCCCAAGGAAATCAGAAGCAAGGGCGTTACCGCAAGAATGAGGGTAGACCACGTT
ACAATCCACAAAGATACGGAGACCCCATGGAACTAGACGCTACGCACTACACAAACGGG
AACGATGACTCAGAAAAGAGACGAAGACGAGAAAACAACTTATGCTTTGAATGTGGAA
AAGCAGGGCACCGAGCAGCAGACTGCCGAAGCAAGAAGACAGGAGGAAAAAGGGGCA
ACTTCAAACCTAAGTTCGGCAAAGGCCAACTTAACGCTACCTTTACAATCCCAGAAAACC
CAACTAAATCCGAAAATACTGAGACTTTCACCGTTGAGGAATTCCAGCAATTACTAAAG
GAATTACCACGAAATAAAGAGGGCATGAATGCAATAGACTTATGGGAACAAGAGTATTA
CAGAACCCCAACACCCTCTGTGACAGAAGAAAGTCACCAGGACGAGGCAGAAGCGGAC
CACGCCACGATGAGCTGGACAGCTTGCTATGATGAATTCTGCGGAATCCATCGATCAGAT
AAAGAAGCAACCGGATGGTTCCCCAAGAAAAGGAAGACGAAGAACCATCAGAATAATG
TAACATGCACGGATTTAACTTCAAATATAACTTCGCGAAAAGTTCGCAAAGTTACCCAGC
AGTTGAATGCTACGGGACAAGCAGGACAGATATACTGCAAGGTTCAGATAAATGGACAC
ATACAATCAGCCATGATAGATTCAGGGGCTACAGGAAATTTTATTGCACCAGAAGCTGC
AAAGTACTTGGAAATACCACTTCAGACGAAACAATACCCCTATCGATTGCAGTTAGTTGA
CGGACAGCTAGCAGGGTCTGACGGAAAGATTTCGCAGGAGACAATCCCAGTACGAATGG
GCATAACCCAACATACAGAGGTTATACAGCTTGACGTTGTGCCATTGGGCCAACAACAG
ATCATCTTAGGAATGCCATGGTTGAAGGCACATAATCCGAAAATAGATTGGGCACAAGG
AATTGTGACATTTGATCAGTGCAAAAGCGGTCACAGGGACACGCTAGAGGCGTCCGCGA
GACGTAACACGCGCCAAGGAGAGTTGAACGCGAACAACACCGGCGACGTAGGACACCC
AGTCCAGGGTCCTCCATTAAGAGCGAAGGCCAGTACACCTCCTCTACAAATGCAGAAGC
CAACGACACGGCACGAAATCGCAATCGAGGCAAAAGAAAGGCCTACGATACCAGAACA
GTACAAGAAATATGAACATGTTTTCAAAGAACCAGGGATCCATGAGGCTTTACCGGAAC
 
ACAAGCCATGGGATCATGAGATAATATTGGAGGAAGGCAAGATGCCTGTGCACACCCCA
ATTTATTCAATGTCAGCCGATGAGTTAAAAAGGCTCAGAGAATACATCGACGACAATTTA
GCCAAGGGATGGATCAGGGAATCCGCGTCCCAAGTGGCCAGTCCAACTATGTGGGTACC
CAAGAAGGATGGACCCGATAGACTAGTTGTAGACTATAGAAAGCTTAACGCACTCACTA
AGAAGGATCGATATCCACTTCCATTAGCTACGGAATTAAGAGATCGATTAGGCGGAGCT
ACGATATTCACCAAGATGGACCTACGTAATGGTTACCACTTGATCAGAATGAAGGAAGG
CGAAGAATGGAAAACCGCTTTCAAAACAAGATACGGGCTATACGAGTACCAAGTTATGC
CGTTCGGGCTAACCAACGCACCAGCTACTTTCATGAGGCTTATGAACAATGTGTTGTCAC
AATATTTGGATACTTGCTGTATATGCTACTTGGACGACATCCTAGTATATTCAAACAACA
AGGTTCAACACATTAAGGACGTTAGCAGCATCCTCGAAAGTCTATCCAAAGCAGACTTG
CTGTGCAAACCAAGCAAATGCGAATTCCATGTCACAGAAACAGAATTCTTGGGATTCAC
CGTATCAAGCCAAGGGCTCAAGATGAGCAAAGACAAGGTTAAGGCAGTGCTCGAATGGA
AGCAGCCGACCACAATCAAGGAAGTACAATCCTTTCTAGGATTTGTCAACTTCTATAGAA
GATTTATCAAGGGTTATTCAGGGATTACTACACCCTTGACCACGTTAACCAGAAAAGATC
AAGGAAGCTTCGAATGGACTGCCAAAGCACAGGAGTCATTCGATACACTCAAACAAGCA
GTGGCAGAAGAACCAATACTGTTGACTTTTGACCCAGAGAAAGAAATCATAGTGGAAAC
GGATTCCTCAGATTTCGCTATAGGAGCAGTTCTGAGCCAACCGGGCCAGAATGGAAAAT
ACCAGCCAATCGCATTCTACTCCCGAAAACTATCACCAGCCGAGTTGAATTACGAGATAT
ATGACAAAGAATTACTGGCGATAGTCGATGCATTTAGAGAATGGCGAGTATATTTGGAA
GGATCGAAATACACAGTACAGGTGTATACAGATCATAAGAACTTGGTTTACTTCACCACA
ACGAAGCAGTTAAACAGACGACAGGTCAGATGGTCGGAGACCATGGCCAACTACAATTT
CAGAATTTCATATGTCAAAGGATCAGAAAACGCTAGAGCCGACGCTCTTAGCCGAAAAC
CAGAATATCAAGAAAACAAAACGTACGAGTCATACGCTATATTCAAGAAAGACGGCGAA
TCACTGGTCTACAATGCACCACAGCTTGCAGCAACACACCTGTTGGAAGACAACCACCTC
AGAAAACAGATTCAATCACACTACGACAAGGATGCTACTGCCACACGCATACGCAAGAC
AATAGAACCAGGATTCACTATAGAAAATGATACCATATACTTTCATGGAAAAGTATACA
TTCCGAGTCAAATGACCAAGGAATTTGTGACGGAACAACATGGGTTGCCGGCACATGG
ACACCAAGGAATTGCAAGGACATTTGCAAGAATACGGGAAATCAGTTACTTCCCACGAA
TGAGAACGATAGTTGAAGAAGTTGTTGGAAATTGTGACACCTGCATACGAAACAAGTCA
TCACGACATGCTCCGTATGGTCAGCTCCAGACCCCAGACATGCCTTCTCAGCCATGGAAG
TCCATCACATGGGACTTTGTGGTCAAACTACCACTCTCAAAGGATCCTACTACAGGAATT
GAGTACGACGCGATACTCAATATAGTAGACAGGCTAACGAAATTTGCATATATGATACC
ATTCAAGGAAACATGGGATGCTGAACAACTAGCATATGTGTTCCTAAGGATCATAGTAA
GCATACACGGAGTACCAGATGAGATAATCTCGGATCGAGACAAGCTCTTTACCTCAAAA
TTCTGGACTACCTTATTAGCACTTATGGGTATCAAGAGAAAGCTATCGACATCTTTCCAC
CCACAAACAGATGGTCAAACAGAGAGGACCAATCAGACAATGGAAGCATATCTTAGATG
CTATGTAAATTATCGACAAGACAATTGGGTAGAACTATTACCTATGGCACAATTCGCATA
TAATACATCGGAAACGGAAACCACGAAAATCACACCAGCACGAGCTAATTTTGGGTTTA
ATCCACAAGCGTATAAAATCCCGATACCACAAGAAGTTAATGCCGAATCAGCAATAGTA
CAAGTCGAACAGCTGAAAAATCTCCAAGAGCAACTGGCTCTTGATCTAAGATTCATATCT
TCCAGAACAGCAGCGTACTACAATACGAAACGTAGTATGGAACCTACGCTTAAAGAGGG
GGATAAAGTTTATTTGCTACGACGAAACATCGAAACCAAGAGACCAAGCAATAAACTCG
ACCACAGGAAACTAGGACCATTCAAGATTGATAAGGTAATAGGAACGGTTAATTATCGA
TTGAAATTACCAGACACAATGAATATCCACCCAGTATTCCACATATCCTTGCTCGAACCA
GCACCACCAGGAGCGCCAAATGCGCCATTTACAGAAATTGAACCAG
TCAACCCAAACGCCATATACGATGTCGAAACAATACTAGACTGCAAATACGTCAGAAAC
AAGGTCAAGTATTTGATCAAATGGTTAGACTACCCACATTCAGAAAACACATGGGAATT
CAAGGAGGATCTCAGCTGCCCTGAGAAGCTACGGGCATTCCACCTGAAGTACCCACACC
TGCCAGTAAAGCCTCAAGATCCG
CTTCGGACAACTCAGGCAAAGAAGGATCGAAGAAGTCGAAGGAAGAAGAATCAATAG
**SEQ ID NO:18 - LTR for siR5**
   >BC1G_07532 - retrotransposable element Tf2 1 protein type 1 
ATGGCATCCAGAGCTACCGCCACAGGTCAATCTGCCGGAGACACCAACGACATCGAGAT
GACCGACGCCCCAAAGGAGATCACTATCAACGAAACCCTTAAGATCGCCTTACCAGACA
 
AGTACCAAGGTAGTCGACAAGAGCTCGATACTTTCCTCTTACAACTTGAGATCTACTTTC
GATTCAATGAGGACAAGTTCACTACCAAGGAATCCAAGAGTATATGGGCCGCATCATAC
CTTCGAGGTGAAGCAACCAAATGGATCCAACCATATTTGCGCGACTATTTCGAGCATGAC
GATAAGGATCGTATGCAACCCACCCGAACAATCTTCAATAGTTTTGAAGGATTTAAGACA
GAGATTCGTAGAATCTTCGGAAATTCCAACGAGTTAGAGGTAGCGGAAGATAAGATCTT
CAACCTCAAGCAGACAGGATCAGCATTGAAATATGCTACGGAATTTCGAAGATATGCTG
GAACAACCAAGTGGGACGAAATCGCTATCATGAGTCACTACCGCAAGGGACTCAAACCA
GAAGTCAGACTGGAATTAGAAAGATCTGCCGAGAGTACAGATCTGAACGATCTAATTCA
GGACTCCATCGAATCAGATGATCGTCTCTACAGATATCGACAAAGCCAGAGATCATACA
AACCCCAAGGAAATCAGAAGCAAGGGCGTTACCGCAAAAATGAGGGTAGACCACGTTA
CAATCCACAGAGATACGGAGACCCCATGGAACTAGACGCCACGCACTACACAAACGGGA
ACGATGACTCGGAAAAGAGACGAAGACGAGAAAACAACTTATGCTTTGAATGTGGAAA
AGCAGGGCACCGAGCAGCAGACTGCCGAAGCAAGAAGACAGGAGGAAAAAGGGGCAA
CTTCAAACCTAAGTTCGGCAAAGGCCAACTTAACGCTACCTTTACAATCCCAGAAAATCC
AACTAAATCCGAAAATACTGAGACTTTCACCGTTGAGGAATTCCAGCAATTACTAAAGG
AATTACCACGAAATCAAGAGGGCATGAATGCAATAGACTTATGGGAGCAAGAGTATTAC
AGAACCCCAACACCCTCTGTGACAGAAGAAAGTCATCAGGACGAGGCAGAAGCAGACC
ACGCCACGATGAGCTGGACAGCTTGCTATGATGAATTCTGCGGAATCCATCGATCAGATA
AAGAAGCAACCGGATGGTCCCCTAAGAAGAGAAAGACGAAGAACCATCAGAATAATGT
AACATGCGAGGATTTAACTCCCAATATAACTTCGCAAGAAGTTCGCAAAGTTACCCAGC
AGTTAAATGCTACGGGACAGGCAGGACAGATATACTGCAAGGTTCAGATAAATGGACAC
ATACAATCAGCCATGATAGATTCAGGGGCTACAGGAAATTTTATTGCACCGGAAGCTGT
AAAGTACTTGGGAATACCACTTCAAACGAAACAACACCCCTATCGATTGCAGGACACGC
TAGAGGCGTCCGCGAGACGTAACACGCGCCAAGGAGAGTTGAACGCGAACAACACCGG
CGACGTAGGACACCCAGTCCAGGGTCCTCCATTAAGAGCGAAGGCCAGTACACCTCCTC
TACAAATGCAGAAGCCAACGACACGGCACGAAATCGCAATCGAGGCAAAAGAAAGGCC
TACGATACCAGAACAGTACAAGAAATATGAACATGTTTTCAAAGAACCAGGGATCCATG
AGGCTTTACCGGAACACAAGCCATGGGATCATGAGATAATATTGGAGGAAGGCAAGATG
CCTGTGCACACCCCAATTTATTCAATGTCAGCCGATGAGTTAAAAAGGCTCAGAGAATAC
ATCGACGACAATTTAGCCAAGGGATGGATCAGGGAATCCGCGTCCCAAGTGGCCAGTCC
AACTATGTGGGTACCCAAGAAGGATGGACCCGATAGACTAGTTGTAGACTATAGAAAGC
TTAACACACTCACTAAGAAGGATCGATATCCACTTCCATTAGCTACGGAATTAAGAGATC
GGTTAGGCGGAGCTACGATATTTACCAAGATGGACCTACGTAATGGTTACCACTTGATCA
GAATGAAGGAAGGCGAAGAATGGAAGACCGCTTTCAAAACAAGATACGGGCTATACGA
GTACCAAGTTATGCCGTTCGGGCTAACCAACGCACCAGCTACTTTCATGAGGCTTATGAA
CAATGTGTTGTCACAATACTTGGATACTTGCTGTATATGCTACTTGGACGACATCCTAGT
ATATTCAAACAACAAGGTTCAACACATTAAGGACGTTAGCAACATCCTCGAAAGCCTAT
CCAAGGCAGACTTGCTGTGCAAACCAAGCAAATGCGAATTCCATGTCACAGAGACAGAA
TTCTTGGGATTCACCGTATCAAGCCAAGGGCTCAAGATGAGCAAAGACAAGGTTAAGGC
AGTGCTCGAATGGAAGCAGCCAACCACAATCAAGGAAGTACAATCCTTTCTAGGGTTCG
TCAACTTCTACAGAAGATTTATCAAGGGTTATTCAGGGATTACTACACCCTTGACCACGT
TAACCAGAAAAGATCAAGAAAGCTTCGAATGGACTGCCATAGCACAGGAGTCATTCGAT
ACGCTCAAACAAGCAGTGGCAGAAGAGCCAATACTATTGACTTTTGACCCAGAGAAAGA
AATCATAGTGGAGACGGACTCCTCGGATTTCGCTATAGGAGCAGTTCTGAGCCAACCGG
GCCAGAATGGAAAATACCAGCCAATCGCATTCTACTCCCGAAAACTATCACCAGCCGAA
TTGAATTACGAGATATATGACAAAGAATTGCTGGCGATAGTCGATGCATTTAGAGAATG
GCGAGTATATTTGGAAGGATCGAAATACACAGTACAGGTGTATACAGATCATAAGAACT
TGGTTTACTTCACCACAACGAAGCAGTTAAACAGACGACAGGTCAGATGGTCGGAGACC
ATGGCCAACTACAATTTCAGAATTTCATATGTCAAAGGATCAGAAAACGCTAGAGCCGA
CGCTCTTAGCCGAAAACCAGAATATCAAGAAAACAAAACGTACGAGTCATACGCTATAT
TCAAGAAAGACGGCGAATCACTGGTTTACAATGCACCACAGCTTGCAGCAACACACCTG
TTGGAAGACAACTACCTTAGGAAACAGATCCAATCACACTACGACAAGGATGCTACTGC
CACACGCATACGCAAGACAATAGAACCAGGATTCACTATAGAAAATGATACCATATACT
TTCATGGAAAAGTATACATTCCGAGTCAAATGACCAAGGAATTTGTGACGGAACAACA
TGGGTTGCCGGCACATGGACACCAAGGAATTGCAAGGACATTTGCAAGAATACGGGAAA

64
 
TCAGTTACTTCCCACGAATGAGAACGATAGTTGAAGAAGTTGTTGGAAATTGTGACACCT
GCATACGAAACAAGTCATCACGGCATGCTCCGTATGGTCAGCTCCAGACCCCAGACATG
CCTTCTCAGCCATGGAAGTCCATCACATGGGACTTTGTGGTCAAACTACCACTCTCAAAG
GATCCTACTACAGGAATTGAGTACGACGCGATACTCAATATAGTAGACAGGCTAACGAA
ATTTGCATATATGATACCATTCAAGGAAACATGGGATGCTGAACAACTAGCATATGTGTT
CCTAAGGATCATAGTAAGCATACACGGAGTACCAGATGAGATAATCTCGGATCGAGACA
AGCTCTTTACCTCGAAATTCTGGACTACCTTATTAGCACTTATGGGTATCAAGAGAAAGC
TATCGACATCTTTCCACCCACAAACAGATGGTCAAACAGAGAGGACCAATCAGACAATG
GAAGCATATCTTAGATGCTATCGTATAAAATCCCGATACCACAAGAAGTTAATGCCGAAT
CAGCAATAG
**SEQ ID NO:19 - LTR for siR5**
   >BC1G 09712 - enzymatic polyprotein 
ATGGCATCCAGAGCTACCGCCACAGGTCAGTCTACCGAAGATACCAACGACATCGAGAT
GACCGATGCCCCAAAGGAGATCACTATCAACGAAACACTTAAGATCGCCTTACCAGACA
AGTACCAAGGTAGTCGACAAGAGCTCGATACTTTCCTCTTACAACTTGAGATCTACTTCC
GATTCAATGAAGACAAGTTCACTACCAAGGAATCCAAGAGCATATGGGCTGCATCATAC
CTCCGAGGTGAAGCAACCAAATGGATTCAACCATATTTGCGCGACTATTTCGAGCATGAC
GATAAGGATCGCATGCAACCCACCCGAACAATCTTCAATAGTTTTGAAGGATTTAAGAC
AGAGATTCGTAGAATCTTCGGAAATTCCAACGAGTTAGAGGTAGCGGAAGATAAGATCT
TCAACCTCAAGCAGACAGGATCAGCATTGAAATATGCTACGGAATTTCGAAGATATGCT
GGAACAACCAAGTGGGACGAAATCGCTATCATGAGTCACTACTGTAAGGGACTCAAACC
AGAAGTCAGACTAGAGTTAGAAAGATCTGCCGAGAGTACAGATCTGAACGATCTAATTC
AGGACTCCATCGAATCAGATGATCGTCTCTATAGATATCGACAAAGCCAAAGATCATAC
AAACCCCAAGGAAACCAAAAGCAAGGGCGTTACCGCAAGAATGAGGGTAGACCACGTT
ACAATCCACAGAGATACGGAGACCCCATGGAACTAGACGCCACGCACTACACAAACGGG
AACGATGACTCAGAAAAGAGACGAAGACGAGAAAACAACTTATGCTTTGAATGTGGAA
AAGCAGGGCACCGAGCAGTAGACTGCCGAAGCAAGAAGACAGGAGGAAAAAGGGGCA
ACTTCAAACCTAAGTTCGGCAAGGGCCAACTTAACGCCACCTTTGCCATCTCAGAAAACT
CAACTAAAACCGAAAATACTGAGACTTTCACCGTTGAGGAATTTCAGCAATTACTAAAG
GAATTACCACGAAATAAAGAGGGCATGAATGCAATAGACTTATGGGAACAAGAGTATTA
CAGAACCCCAACACCCTCTGTGACAGAAGAAAGTCACCAGGACGAGGCAGAAGCGGAC
CACGCCACGATGAGCTGGACAGCTTGCTATGATGAATTCTGCGGAATCCATCGATCAGAT
AAAGAAGCAACCGGATGGTTCCCCAAGAAAAGGAAGACGAAGAACCATCAGAATAATG
TAACATGCGAGGATTTAACTCCCAATATAACTTCGCAAGAAGTTCGCAAAGTTACCCAGC
AGTTGAATGCTACGGGACAGGCAGGACAAGTGTACTGCAAGGTCCAGATAAATGGACAC
ATACAATCAGCCATGATAGATTCAGGGGCTACAGGAAATTTTATTGCACCAGAAGCTGC
AAAGTACTTGGAAATACCACTTCAAACGAAACAACACCCCTATCGATTGCAGGACACGC
TAGAGGCGTCCGCGAGACGTAACACGCGCCAAGGGGAGTTGAACGCGAACAACACCGG
CGACGTAGGACACCCAGTCCAGGGTCCTCCATTAAGAGCGAAGGCCAGTACACCTCCTC
TACAAATGCAGAAGCCAACGACACGGCACGAAATCGCAATCGAGGCAAAAGAAAGGCC
TACGATACCAGAACAGTACAAGAAATATGAACATGTTTTCAAAGAACCAGGGATCCATG
AGGCTTTACCGGAACACAAGCCATGGGATCATGAGATAATATTGGAGGAAGGCAAGATG
CCTGTGCACACCCCAATTTATTCAATGTCAGCCGATGAGTTAAAAAGGCTCAGAGAATAC
ATCGACGACAATTTAGCCAAGGGATGGATCAGGGAATCCGCGTCCCAAGTGGCCAGTCC
AACTATGTGGGTACCCAAGAAGGATGGACCCGATAGACTAGTTGTAGACTATAGAAAGC
TTAACGCACTCACTAAGAAGGATCGATATCCACTTCCATTAGCTACGGAATTAAGAGATC
GATTAGGCGGAGCTACGATATTCACCAAGATGGACCTACGTAATGGTTACCACTTGATCA
GAATGAAGGAAGGCGAAGAATGGAAAACCGCTTTCAAAACAAGATACGGGCTATACGA
GTACCAAGTTATGCCATTCGGGCTAACCAACGCACCAGCTACTTTCATGAGGCTTATGAA
CAATGTGTTGTCACAATATTTGGATACTTGCTGTATATGCTACTTGGACGACATCCTAGTA
TATTCAAACAACAAGGTTCAACACATTAAGGACGTTAGCAACATCCTCGAAAGTCT
ATCCAAAGCAGACTTGCTGTGCAAACCAAGCAAATGCGAATTCCATGTCACAGAAACAG
AATTCTTGGGATTCACCGTATCAAGCCAAGGGCTCAAGATGAGCAAAGACAAGGTTAAG
GCAGTGCTCGAATGGAAGCAGCCAACCACAATCAAGGAGGTACAATCCTTTCTAGGGTT
 
CGTCAACTTCTACAGAAGATTTATCAAGGGTTATTCAGGGATTACTACACCCTTGACCAC
GTTAACCAGAAAAGATCAAGGAAGCTTCGAATGGACTGCCAAAGCACAGGAGTCATTCG
ATACACTCAAACAAGCAGTGGCAGAAGAACCAATACTGTTGACTTTTGACCCAGAGAAA
GAAATCATAGTGGAAACGGATTCCTCAGATTTCGCTATAGGAGCAGTTCTGAGCCAACC
GGGCCAGAATGGAAAATACCAGCCAATCGCATTCTACTCCCGAAAACTATCACCAGCTG
AGTTAAATTACGAGATATATGACAAAGAATTACTGGCAATAGTCGATGCATTTAGAGAA
TGGCGAGTATATTTGGAAGGATCGAAATACACAGTACAGGTGTATACAGATCATAAGAA
CTTGGTTTACTTCACCACAACGAAGCAGTTAAACAGACGACAGGTCAGATGGTCGGAGA
CCATGGCCAACTACAATTTCAGAATTTCATATGTCAAAGGATCAGAAAACGCTAGAGCC
GACGCTCTTAGCCGAAAACCAGAATATCAAGAAAACAAAACGTACGAGTCATACGCTAT
ATTCAAGAAAGACGGCGAATCACTGGTCTACAATGCACCACAGCTTGCAGCAACACACC
TGTTGGAAGACAACCACCTCAGGAAACAGATCCAATCACACTACAACAAGGATGCTACT
GCCACACGCATACGCAAGACAATAGAACCAGGATTCACTATAGAAGATGATACCATATA
CTTTCATGGAAAAGTATACATTCCGAGTCAAATGACCAAGGAATTTGTGACGGAACAA
CACGGATTGCCGGCACATGGACACCAAGGAATTGCAAGGACATTTGCAAGAATACGGGA
AATCAGTTACTTCCCACGAATGAGAACGATAGTTGAAGAAGTTGTTGGAAATTGTGACA
CCTGCATACGAAACAAGTCATCACGACATGCTCCGTATGGTCAGCTCCAGACCCCAGAC
ATGCCTTCTCAGCCATGGAAGTCCATCACATGGGACTTTGTGGTCAAACTACCACTCTCA
AAGGATCCTACTACAGGAATTGAGTACGACGCGATACTCAATATAGTAGACAGGCTAAC
GAAATTTGCATATATGATACCATTCAAGGAAACATGGGATGCTGAGCAACTAGCATATG
TGTTCCTAAGGGTCATAGTAAGCATACACGGAGTACCAGATGAGATAATCTCGGATCGA
GACAAGCTCTTTACCTCGAAATTCTGGACTACCTTATTAGCACTTATGGGTATCAAGAGA
AAGCTATCGACATCTTTCCACCCACAAACAGATGGTCAAACAGAGAGGACCAATCAGAC
AATGGAAGCATATCTTAGATGCTATCGTATAAAATCCCGATACCACAAGAAGTTAATGCC
GAAT CAGCAATAG
**SEQ ID NO:20 - LTR for siRS**
   >BC1G 15972 - enzymatic polyprotein 
ATGGCATCCAGAGCTACCGCCACAGGTCAATCTGCCGGAGACACCAACGACATCGAGAT
GACCGACGCTCCAAAGGAGATCACTATCAACGAAACCCTTAAGATCGCCTTACCAGACA
AGTACCAAGGTAGTCGACAAGAGCTCGATACTTTCCTCTTACAACTTGAGATCTACTTCC
GATTCAATGAGGACAAGTTCACTACCAAGGAATCCAAGAGCATATGGGCCGCGTCATAC
CTTCGAGGTGAAGCAACCAAATGGATTCAACCATATTTGCGCGACTATTTCGAGCATGAC
GATAAGGATCGCATGCAACCCACCCGAACAATCTTCAATAGTTTTGAAGGATTTAAGAC
AGAGGTTCGTAGAATCTTCGGAAATTCCAACGAGTTAGAGGTAGCGGAAGATAAGATCT
TCAACCTCAAGCAGACAGGATCAGCATTGAAATATGCTACGGAATTTCGAAGATATGCT
GGAACAACCAAGTGGGACGAAATCGCTATCATGAGTCACTACCGCAAGGGACTCAAACC
AGAAGTCAGACTAGAATTAGAAAGATCTGCCGAGAGTACAGATCTAAACGATCTAATTC
AGGACTCCATCGAATCAGATGATCGTCTCTACAGATATCGACAAAGCCAAAGATCATAC
AAACCCCAAGGAAATCAGAAGCAAGGGCGTTACCGCAAGAATGAGGGTAGACCACGTT
ACAATCCACAGAGATACGGAGACCCCATGGAACTAGACGCTACGCACTACACAAACGGG
AACGATGACTCAGAAAAGAGACGAAGACGAGATAACAACTTATGCTTTGAATGTGGAAA
AGCAGGGCACCGAGCAGCAGACTGCCGAAGCAAGAAGACAGGAGGAAAAAGGGGCAA
CTTCAAACCTAAGTTCGGCAAGGGCCAACTTAATGCCACCTTTGCAATCTCAGAAAACTC
AACTAAACCCGAAAATACTGAGACTTTCACCGTTGAGGAATTCCAGCAATTATTAGAGG
AATTACCACGAAACCAAGAGGGCATGAATGCAATAGACTTATGGGAACAAGAGTATTAC
AGAACTCCAACACCCTCTGTGACAGAAGAAAGTCACCAGGACGAGGCAGAAGCGGACC
ACGCCACGATAAGCTGGACAGCTTGCTATGACGAATTCTGCGGAATCCATCGATCAGAT
AAAGAAGCAACCGGATGGTTCCCCAAGAAGAGAAAGACGAAGAACCGACAGAATAATG
TAACATGCAAGGATTTAACTCCAAATGTAACTTCGCGAAAAGTTCGCAAAGTTACACAG
CAATTGAATGCTACGGGACAGGCAGGACAAATATACTGCACGGTTCAGATAAATGGACA
CATACAATCAGCCATGATAGATTCAGGGGCTACAGGGAATTTTATTGCACCAGAAGCTG
CAAAGTACTTGGAAATACCACTTCAAACGAAACAACACCCCTACCGATTGCAGTTAGTTG
ACGGACAGCTAGCAGGGTCTGACGGAAAGATTTCGCAGGAGACAATCCCAGTACGAATG
GGCATAACCCAACATACAGAGGTTATACAGCTTGACGTTGTGCCATTGGGCCAACAACA
 
GATCATCTTAGGAATGCCATGGTTAAAGGCACATAATCCGAAAATAGATTGGGCACAAG
GAATTGTGACATTTGATCAGTGCAAAAGCGGTCACAGGGACACGCTAGAGGCGTTCGCG
AGACGTAACACGCGCCAAGGAGAGTTGAACGCGAACAACACCGGCGACGTAGGACACC
CAGTCCAGGGTCCTCCATTAAGAGCGAAGGCCAGTACACCTCCTCTACAAATGCAGAAG
CCAACGACACGGCACGAAATCGCAATCGAGGCAAAAGAAAAGCCTACGATACCAGAAC
AGTACAAGAATTATGAACATGTTTTCAAAGAACCAGGGATCCATGAGGCTTTACCGGAA
CACAAGCCATGGGATCATGAGATAATATTGGAGGAAGGCAAGATGCCTGTGCACACCCC
AATTTATTCAATGTCAGCCGATGAGTTAAAGAGGCTCAGAGAGTACATCGACGACAATTT
AGCCAAGGGATGGATCAGGGAATCCGCGTCCCAAGTGGCCAGTCCAACTATGTGGGTAC
CCAAGAAGGATGGACCCGATAGACTAGTTGTAGACTATAGAAAGCTTAACGCACTCACT
AAGAAGGATCGATATCCACTTCCATTAGCTACGGAATTAAGAGATCGATTAGGCGGAGC
TACGATATTTACCAAGATGGACCTACGTAATGGTTACCACTTGATCAGAATGAAGGAAG
G
CGAAGAATGGAAAACCGCTTTCAAAACAAGATACGGGCTATACGAGTACCAAGTTATGC
CATTCGGGCTAACCAACGCACCAGCTACTTTCATGAGGCTTATGAACAATGTGTTGTCAC
AATATTTGGATACTTGCTGTATATGCTACTTGGACGACATCCTAGTATATTCAAACAACA
AGGTTCAACACATTAAGGACGTTAGCAACATCCTCGAAAGCCTATCCAAGGCAGACTTG
CTGTGCAAACCAAGCAAATGCGAATTCCATGTCACAGAGACAGAATTCTTGGGATTCAC
CGTATCAAGCCAAGGGCTCAAGATGAGCAAAGGCAAGGTTAAGGCAGTGCTCGAATGGA
AGCAGCCGACCACAATCAAGGAAGTACAATCCTTTCTAGGGTTCGTCAACTTCTACAGAA
GATTTATCAAAGGTTATTCAGGGATTACTACACCCTTGACCACGTTAACCAGAAAAGATC
AAGGAAGCTTCGAATGGACTGCCAAAGCACAGGAGTCATTCGATACGCTCAAACAAGCA
GTGGCAGAAGAGCCAATACTATTGACTTTTGACCCAGAGAAAGAAATCATAGTGGAGAC
GGACTCCTCGGATTTCGCTATAGGAGCAGTTCTGAGCCAACCGGGTCAGAATGGAAAAT
ACCAGCCAATCGCATTCTACTCCCGAAAACTATCACCAGCTGAGTTGAATTACGAGATAT
ATGACAAAGAATTACTGGCGATAGTCGATGCATTTAGAGAATGGCGAGTATATTTGGAA
GGATCGAAATACACAGTACAGGTGTACACAGATCATAAGAACTTGGTTTACTTCACCAC
AACGAAGCAGTTAAACAGACGACAGGTCAGATGGTCGGAGACCATGGCCAACTACAACT
TTAGAATTTCATATGTCAAAGGATCAGAAAATGCTAGAGCCGACGCTCTTAACCGAAAA
CCAGAATATCAAGAAAACAAAGCGTACGAGTCATACGCTATATTCAAGAAAGACAGCGA
ATCACTGGTTTACAATACACCACAGCTTGCAACAACACACCTGTTGGAAGACAACCACCT
CAGGAAACAGATCCAATCACACTACGACAAGGATACTACTGCCACACGCATACGCAAAA
CAATAGAACCAGGATTCACTATAGAAAATGATACCATATACTTTCATAGAAAAGTATAC
ATTCCGAGTCAAATGACCAAGGAATTTGTGACGGAACAACACGGGTTGCCGGCACATG
GACACCAAGGAATTGCAAGGACATTTGCAAGAATACGGGAAATCAGTTACTTCCCACGA
ATGAGAACGATAGTTGAAGAAGTTGTTGGAAATTGTGACACCTGCATACGAAACAAGTC
ATCACGACATGCTCCGTATGGTCAGCTCCAGACCCCAGACATGCCTTCTCAGCCATGGAA
GTCCATCACATGGGACTTTGTGGTCAAACTACCACTCTCAAAGGATCCTACTACAGGAAT
TGAGTACGACGCGATACTCAATATAGTAGACAGGCTAACGAAATTTGCATATATGATAC
CATTCAAGGAAACATGGGATGCTGAGCAACTAGCATATGTGTTCCTAAGGATCATAGTA
AGCATACACGGAGTACCAGATGAGATAATCTCGGATCGAGACAAGCTCTTTACCTCGAA
ATTCTGGACTACCTTATTAGCACTTATGGGTATCAAGAGAAAGCTATCGACATCTTTCCA
CCCACAAACAGATGGTCAAACAGAGAGGACCAATCAGACAATGGAAGCATATCTTAGAT
GCTATGTAAATTATCGACAAGACAATTGGGTAGAGCTATTACCCATGGCACAGTTCGCAT
ACAATACATCAGAAACGGAAACCACGAAAATCACACCAGCACGAGCTAATTTTGGGTTT
AATCCACAAGCGTATAAAATCCCGATACCACAAGAAGTTAATGCCGAATCAGCGATAGT
ACAAGTCGAACAGTTGAAAGATCTCCAAGAGCAACTGGCTCTTGATCTAAGATTCATATC
TTCCAGAACAGCAGCGTACTACAATACGAAACGTAGTATGGAACCTACGCTTAAAGAGG
GGGATAAAGTTTATTTGCTACGACGAAACATCGAAACCAAGAGACCAAGCAATAAACTC
GACCACAGGAAACTAGGACCATTCAAGATTGATAAGGTAATAGGAACGGTTAATTATCA
ATTGAAATTACCAGACACAATGAATATCCACCCAGTATTCCACATATCCTTGCTCGAACC
AGCACCACCAGGAGCGCCAAATGCGCCATTTACAGAAATTGAACCAG
TCAACCCAAACGCCATATACGATGTCGAAACAATACTAGACTGCAAATACGTCAGAAAC
AAGGTCAAGTATTTGATCAAATGGTTAGACTACCCACATTCAGAAAACACATGGGAACT
CAAGGAAGATCTCAGCTGCCCTGAGAAACTACGGGCATTCCACCTGAAGTACCCACATC
 
TGCCAACAAAGCCTCAAGCTCCG
CATCAGACAACAAAGGCAACGAGGGGTCGAAGAAACCAAAAGAAGAACCACTAG
**SEQ ID NO:21 - LTR for siRS**
   >BC1G 13999 retrotransposable element Tf2 1 protein type 1 
ATGTGGGTACCCAAGAAGGATGGACCCGATAGACTAGTTGTAGACTATAGAAAGCTTAA
CGCACTCACTAAGAAGGATCGATATCCACTTCCATTAGCTACGGAATTAAGAGATCGATT
AGGCGGAGCTACGATATTCACCAAGATGGACCTACTATATTCAAACAACAAGGTTCAAC
ACATTAAGGACGTTAGCAACATCCTCGAAAGCCTATCCAAGGCAGACTTGCTGTGCAAA
CCAAGCAAATGCGAATTCCATGTCACAGAGACAGAATTCTTGGGATTCACCGTATCAAG
CCAAGGGCTCAAGATGAGCAAAGGCAAGGTTAAGGCAGTGCTCGAATGGAAGCAGCCG
ACCACAATCAAGGAAGTACAATCCTTTCTAGGGTTCGTCAACTTCTACAGAAGATTTATC
AAAGGTTATTCAGGGATTACTACACCCTTGACCACGTTAACCAGAAAAGATCAAGGAAG
CTTCGAATGGACTGCCAAAGCACAGGAGTCATTCGATACGCTCAAACAAGCAGTGGCAG
AAGAGCCAATACTATTGACTTTTGACCCAGAGAAAGAAATCATAGTGGAGACGGACTCC
TCGGATTTCGCTATAGGAGCAGTTCTGAGCCAACCGGGTCAGAATGGAAAATACCAGCC
AATCGCATTCTACTCCCGAAAACTATCACCAGCTGAGTTGAATTACGAGATATATGACAA
AGAATTACTGGCGATAGTCGATGCATTTAGAGAATGGCGAGTATATTTGGAAGGATCGA
AATACACAGTACAGGTGTACACAGATCATAAGAACTTGGTTTACTTCACCACAACGAAG
CAGTTAAACAGACGACAGGTCAGATGGTCGGAGACCATGGCCAACTACAACTTTAGAAT
TTCATATGTCAAAGGATCAGAAAATGCTAGAGCCGACGCTCTTAGCCGAAAACCAGAAT
ATCAAGAAAACAAAACGTACGAGTCATACGCTATATTCAAGAAAGACGGCGAATCACTG
GTTTACAATGCACCACAGCTTGCAGCAACACACCTGTTGGAAGACAACCACCTCAGGAA
ACAGATCCAATCACACTACGACAAGGATGCTACTGCCACACGCATACGCAAGACAATAG
AACCAGGATTCACTATAGAAAATGATACCATATACTTTCATGGAAAAGTATACATTCCG
AGTCAAATGACCAAGGAATTTGTGACGGAACAACACGGGTTGCCGGCACATGGACACCA
AGGAATTGCAAGGACATTTGCAAGAATACGGGAAATCAGTTACTTCCCACGAATGAGAA
CGATAGTTGAAGAAGTTGTTGGAAATTGTGACACCTGCATACGAAACAAGTCATCACGA
CATGCTCCGTATGGTCAGCTCCAGACCCCAGACATGCCTTCTCAGCCATGGAAGTCCATC
ACATGGGACTTTGTGGTCAAACTACCACTCTCAAAGGATCCTACTACAGGAATTGAGTAC
GACGCGATACTCAATATAGTAGACAGGCTAACGAAATTTGCATATATGATACCATTCAA
GGAAACATGGGATGCTGAGCAACTAGCATATGTGTTCCTAAGGATCATAGTAAGCATAC
ACGGAGTACCAGATGAGATAATCTCGGATCGAGACAAGCTCTTTACCTCGAAATTCTGG
ACTACCTTATTAGCACTTATGGGTATCAAGAGAAAGCTATCGACATCTTTCCACCCACAA
ACAGATGGTCAAACAGAGAGGACCAATCAGACAATGGAAGCATATCTTAGATGCTATGT
AAATTATCGACAAGACAATTGGGTAGAGCTATTACCCATGGCACAGTTCGCATACAATA
CATCAGAAACGGAAACCACGAAAATCACACCAGCACGAGCTAATTTTGGGTTTAATCCA
CAAGCGTATAAAATCCCGATACCACAAGAAGTTAATGCCGAATCAGCGATATATGGAAC
CTACGCTTAA
**SEQ ID NO:22 - LTR for siRS**
   >BC1G_04888.1 retrotransposable element Tf2 1 protein type 1 (Transcript:BC1T_04888) 
ATGGCCAACTACAATTTTAGAATTTCATATGTCAAAGGATCAGAAAACGCTAGAGCCGA
CGCTCTTAGCCGAAAACCAGAATATCAAGAAAACAAAACGTACGAGTCATACGCTATAT
TCAAGAAAGACGGCGAATCACTGGTCTACAATGCACCACAGCTTGCAGCAACACACCTG
TTGGAAGACAACCACCTCAGGAAACAGATCCAATCACACTACAACAAGGATGCTACTGC
CACACGCATACGCAAGACAATAGAACCAGGATTCACTATAGAAGATGATACCATATACT
TTCATGGAAAAGTATACATTCCGAGTCAAATGACCAAGGAATTTGTGACGGAACAACA
TGGGTTGCCGGCACACGGACATCAAGGGATTGCAAGAACATTTGCAAGAATCCGGGAAA
TCAGTTACTTCCCACGAATGAGAACGATAGTTGAAGAAGTTGTTGGAAATTGTGACACCT
GCATACGAAACAAGTCATCACGACATGCGCCGTATGGTCAGCTCCAGACCCCAGACATG
CCTTCTCAGCCATGGAAGTCCATCACATGGGACTTTGTGGTCAAACTACCACTCTCAAAG
GATCCTACTACAGGAATTGAGTACGACGCGATACTCAATATAGTAGACAGGCTAACGAA
ATTCGCATATATGATACCATTCAAGGAAACATGGGATGCTGAGCAACTAGCATATGTGTT
 
CCTAAGGATCATAGTAAGCATACACGGAGTACCAGATGAGATAATCTCGGATCGAGACA
AGCTCTTTACCTCGAAATTCTGGACTACCTTATTAGCACTTATGGGTATCAAGAGAAAGC
TATCGACATCTTTCCACCCACAAACAGATGGTCAAACAGAGAGGACCAATCAGACAATG
GAAGCATATCTTAGATGCTATCGTATAAAATCCCGATACCACAAGAAGTTAATGCCGAAT
CAGCGATAG
**SEQ ID NO:23 - LTR for siRS**
   >BC1G_16375.1 hypothetical protein similar to truncated Pol (Transcript:BC1T_16375) 
ATCCAATCACACTACAACAAGGATGCTACTGCCACACGCATACGCAAGACAATAGAACCA
GGATTCACTATAGAAGATGATACCATATACTTTCATGGAAAAGTATACATTCCGAGTCAAA
TGACCAAGGAATTTGTGACGGAACAACATGGGTTGCCGGCACACGGACATCAAGGGATTG
CAAGAACATTTGCAAGAATCCGGGAAATCAGTTACTTCCCACGAATGAGAACGATAGTTG
AAGAAGTTGTTGGAAATTGTGACACCTGCATACGAAACAAGTCATCACGACATGCGCCGT
ATGGTCAGCTCCAGACCCCAGACATGCCTTCTCAGCCATGGAAGTCCATCACATGGGACTT
TGTGGTCAAACTACCACTCTCAAAGGATCCTACTACAGGAATTGACATACACGGAGTACCA
GATGAGATAATCTCGGATCGAGACAAGCTCTTTACCTCGAAATTCTGGACTACCTTATTAGC
ACTTATGGGTATCAAGAGAAAGCTATCGACATCTTTCCACCCACAAACAGATGGTCAAACA
GAGAGGACCAATCAGACAATGGAAGCATATCTTAGATGCTATGTAAATTATCGACAAGACA
ATTGGGTAGAGCTATTACCCATGGCACAGTTCGCATACAATACATCGGAAACGGAAACCAC
GAAAATCACCCCAGCACGAGCTAATTTTGGGTTTAATCCACAAGCGTATAAAATCCCGATA
CCACAAGAAGTTAATGCCGAATCAGCAATAGTACAAGTCGAACAGCTGAAAGATCTCCAA
GAGCAACTGGCTCTTGATCTAAGATTCATATCTTCCAGAACAGCAGCGTACTACAATACGA
AACGTAGTATGGAACCTACGCTTAAAGAGGGGGATAAAGTTTATTTGCTACAACGAAACAT
CGAAACCAAGAGACCAAGCAATAAACTCGACCACAGGAAACTAGGACCATTCAAGATTG
ATAAGGTAATAGGAACG
**SEQ ID NO:24 - LTR for siRS**
   >BC1G_06254.1 retrotransposable element Tf2 1 protein type 1 (Transcript:BC1T_06254) 
ATGGCATCCAGAGCCACCGCCACAGGTCAGTCTACCGGAGATACCAACGACATCGAGATG
ACCGATGCCCCAAAGGAGATCACTATCAACGAAACACTTAAGATCGCCTTACCAGACAAG
TACCAAGGTAGTCGACAAGAGCTCGATACTTTCCTCTTACAACTTGAGATCTACTTCCGAT
TCAATGAAGACAAGTTCACTACCAAGGAATCCAAGAGCATATGGGCTGCATCATACCTCCG
AGGTGAAGCAACCAAATGGATTCAACCATATTTGCGCGACTATTTCGAGCATGACGATAAG
GATCGCATGCAACCCACCCGAACAATCTTCAATAGTTTTGAAGGATTTAAGACAGAGATTC
GTAGAATCTTCGGAAATTCCAACGAGTTAGAGGTAGCGGAAGATAAGATCTTCAACCTCA
AGCAGACAGGATCAGCATTGAAATATGCTACGGAATTTCGAAGATATGCTGGAACAACCA
AGTGGGACGAAATCGCTATCATGAGTCACTACCGTAAGGGACTCAAACCAGAAGTCAGAC
TAGAGTTAGAAAGATCTGCCGAGAGTACAGATCTGAACGATCTAATTCAGGACTCCATCGA
ATCAGATGATCGTCTCTATAGATATCGACAAAGCCAAAGATCATACAAACCCCAAGGAAAC
CAAAAGCAAGGGCGTTACCGCAAGAATGAGGGTAGACCACGTTACAATCCACAGAGATAC
GGAGACCCCATGGAACTAGACGCCACGCACTACACAAACGGGAACGATGACTCAGAAAA
GAGACGAAGACGAGAAAACAACTTATGCTTTGAATGTGGAAAAGCAGGGCACCGAGCAG
TAGACTGCCGAAGCAAGAAGACAGGAGGAAAAAGGGGCAACTTCAAACCTAAGTTCGGC
AAGGGCCAACTTAACGCCACCTTTGCCATCTCAGAAAACTCAACTAAAACCGAAAATACT
GAGACTTTCACCGTTGAGGAATTTCAGCAATTACTAAAGGAATTACCACGAAATAAAGAG
GGCATGAATGCAATAGACTTATGGGAACAAGAGTATTACAGAACCCCAACACCCTCTGTG
ACAGAAGAAAGTCACCAGGACGAGGCAGAAGCGGACCACGCCACGATGAGCTGGACAG
CTTGCTATGATGAATTCTGCGGAATCCATCGATCAGATAAAGAAGCAACCGGATGGTTCCC
CAAGAAAAGGAAGACGAAGAACCATCAGAATAATGTAACATGCGAGGATTTAACTCCCAA
TATAACTTCGCAAGAAGTTCGCAAAGTTACCCAGCAGTTGAATGCTACGGGACAGGCAGG
ACAAGTGTACTGCAAGGTCCAGATAAATGGACACATACAATCAGCCATGATAGATTCAGGG
GCTACAGGAAATTTTATTGCACCAGAAGCTGCAAAGTACTTGGAAATACCACTTCAAACG
AAACAACATCCCTACCGATTGCAGGACACGCTAGAGGCGTCCGCGAGACGTAACACGCGC
 
CAAGGGGAGTTGAACGCGAACAACACCGGCGACGTAGGACACCCAGTCCAGGGTCCTCC
ATTAAGAGCGAAGGCCAGTACACCTCCTCTACAAATGCAGAAGCCAACGACACGGCACG
AAATCGCAATCGAGGCAAAAGAAAGGCCTACGATACCAGAACAGTACAAGAAATATGAAC
ATGTTTTCAAAGAACCAGGGATCCATGAGGCTTTACCGGAACACAAGCCATGGGATCATG
AGATAATATTGGAGGAAGGCAAGATGCCTGTGCACACCCCAATTTATTCAATGTCAGCCGA
TGAGTTAAAAAGGCTCAGAGAATACATCGACGACAATTTAGCCAAGGGATGGATCAGGGA
ATCCGCGTCCCAAGTGGCCAGTCCAACTATGTGGGTACCCAAGAAGGATGGACCCGATAG
ACTAGTTGTAGACTATAGAAAGCTTAACGCACTCACTAAGAAGGATCGATATCCACTTCCA
TTAGCTACGGAATTAAGAGATCGATTAGGCGGAGCTACGATATTCACCAAGATGGACCTAC
GTAATGGTTACCACTTGATCAGAATGAAGGAAGGCGAAGAATGGAAAACCGCTTTCAAAA
CAAGATACGGGCTATACGAGTACCAAGTTATGCCATTCGGGCTAACCAACGCACCAGCTAC
TTTCATGAGGCTTATGAACAATGTGTTGTCACAATATTTGGATACTTGCTATCAAGGAAGCT
TCGAATGGACTGCCAAAGCACAGGAGTCATTCGATACGCTCAAGCAAGCAGTGGCAGAA
GAACCAATACTGTT
GACTTTTGACCCAGAGAAAGAAATCATAGTGGAGACGGACTCCTCGGATTTCGCTATAGG
AGCAGTTCTGAGCCAACCGGGCCAGAATGGAAAATACCAGCCAATCGCATTCTACTCCCG
AAAACTATCACCAGCTGAGTTAAATTACGAGATATATGACAAAGAATTACTGGCAATAGTC
GATGCATTTAGAGAATGGCGAGCATATTTGGAAGGATCGAAATACACAGTACAGGTATATA
CAGATCATAAGAACTTGGTTTACTTCACCACAACGAAGCAGTTAAACAGACGACAGGTCA
GATGGTCGGAGACCATGGCCAACTACAACTTTAGAATTTCATATGTCAAAGGATCAGAAAA
CGCTAGAGCCGACGCTCTTAGCCGAAAACCAGAATATCAAGAAAACAAAACGTACGAGTC
ATACGCTATATTCAAGAAAGACGGCGAATCACTGGTCTACAATGCACCACAGCTTGCAGCA
ACACACCTGTTGGAAGACAACCACCTCAGGAAACAGATCCAATCACACTACAACAAGGAT
GCTACTGCCACACGCATACGCAAGACAATAGAACCAGGATTCACTATAGAAGATGATACCA
TATACTTTCATGGAAAAGTATACATTCCGAGTCAAATGACCAAGGAATTTGTGACGGAAC
AACATGGGTTGCCGGCACACGGACATCAAGGGATTGCAAGAACATTTGCAAGAATCCGGG
AAATCAGTTACTTCCCACGAATGAGAACGATAGTTGAAGAAGTTGTTGGAAATTGTGACA
CCTGCATACGAAACAAGTCATCACGACATGCGCCGTATGGTCAGCTCCAGACCCCAGACA
TGCCTTCTCAGCCATGGAAGTCCATCACATGGGACTTTGTGGTCAAACTACCACTCTCAAA
GGATCCTACTACAGGAATTGAGTACGACGCGATACTCAATATAGTAGACAGGCTAACGAAA
TTTGCATATATGATACCATTCAAGGAAACATGGGATGCTGAGCAACTAGCATATGTGTTCCT
AAGGGTCATAGTAAGCATACACGGAGTACCAGATGAGATAATCTCGGATCGAGACAAGCT
CTTTACCTCGAAATTCTGGACTACCTTATTAGCACTTATGGGTATCAAGAGAAAGCTATCGA
CATCTTTCCACCCACAAACAGATGGTCAAACAGAGAGGACCAATCAGAC
AATGGAAGCATATCTTAGATGCTATCGTATAAAATCCCGATACCACAAGAAGTTAATGCCGA
ATCAGCAATAG
**SEQ ID NO:25 - LTR for siR5**
   **>BC1G_08449.1 retrotransposable element Tf2 1 protein type 1 (Transcript:BC1T_08449)** 
ATGGCATCCAGAGATACCGCCACAGGTCAATCTGCCGGAGACACCAACGACATCGAGATG
ACCGATGCCCCAAAGGAGATCACTATCAACGAAACCCTTAAGATCGCCTTACCAGACAAG
TACCAAGGTAGTCGACAAGAGCTCGATACTTTCCTCTTACAACTTGAGATCTACTTCCGAT
TCAATGAGGACAAGTTCACTACCAAGGAATCCAAGAGTATATGGGCCGCATCATACCTCCG
AGGTGAAGCAACCAAATGGATTCAACCATATTTGCGCGACTATTTCGAACATGACGATAAG
AATCGCATGCAACCCACCCGAACAATCTTCAATAGTTTTGAAGGATTTAAGACAGAGATTC
GTAGAATCTTCGGAAATTCCAACGAGTTAGAGGTAGCGGAAGATAAGATCTTCAACCTCA
AGCAGACAGGATCAGCATTGAAATATGCTACGGAATTTCGAAGATATGCTGGAACAACCA
AGTGGGACGAAATCGCTATCATGAGTCACTACCGCAAGGGACTCAAACCAGAAGTCAGAC
TGGAATTAGAAAGATCTGCCGAGAGTACAGATCTGAACGATCTAATTCAGGACTCCATCGA
ATCAGATGATCGTCTCTACAGATATCGACAAAGCCAGAGATCATACAAACCCCAAGGAAAT
CAGAAGCAAGGGCGTTACCGCAAGAATGAGGGTAGACCACGTTACAATCCACAGAGGTA
CGGAGACCCAATGGAACTAGACGCTACGCACTACACAAACGGGAACGATGACTCAGAAA
AGAGACGAAGACGAGAAAACAACTTATGCTTTGAATGTGGAAAAGCAGGGCACCGAGCA
GCAGAGTGCCGAAGCAAGAAGACAGGAGGAAAAAGGGGCAACTTCAAACCTAAGTTCG
 
GCAAGGGCCAACTTAACGCCACCTTTGCAATCCCAGAAAACCCAACTAAATCCGAAAATA
CTGAGACTTTCACCATTGAAGAATTCCAGCAATTACTAGAGGAATTACCACGAAATCAAGA
GGGCATGAATGCAATAGACTTATGGGAACAAGAGTATTACAGAACCCCAACACCCTCTGTA
ACAGAAGAAAGTCACCAGGACGAGGCAGAAGCAGACCACGCCACAATGAGCTGGACAG
CCTGCTATGATGAATTCTGCGGAATTCATCGATCAGATAAAGAAGCAACCGGATGGTTCCC
CAAGAAAAGGAAGACGAAGAACCATCAGAATAATGTAACATGCGAGGATTTAACTCCCAA
TACAACTTCGCAAGAAGTTCGCAAAGTTACCCAGCAGTTGAATGCTACGGGACAGGCAGG
ACAGATATACTGCAAAGTTCAGATAAATGGACACATACAATCAGCCATGATAGATTCAGGG
GCTACAGGAAATTTTATTGCACCAGAAGCTGCAAAGTACTTGGAAATACCACTTCAGACG
AAACAACACCCCTACCGATTGCAGGACACGCTAGAGGCGTCCGCGAGACGTAACACGCG
CCAAGGAGAGTTGAACGCGAACGACACCGGCGACGTAGGACACCCAGTCCAGGGTCCTC
CATTAAGAGCGAAGGCCAGTACACCTCCTCTACAAATGCAGAAGCCAACGACACGGCACG
AAATCGCAATCGAGGCAAAAGAAAAGCCTACGATACCAGAACAGTACAAGAATTATGAAC
ATGTTTTCAAAGAACCAGGGATCCATGAGGCTTTACCGGAACACAAGCCATGGGATCATG
AGATAATATTGGAGGAAGGCAAGATGCCTGTGCACACCCCAATTTATTCAATGTCAGCCGA
TGAGTTAAAAAGGCTCAGAGAATACATCGACGACAATTTAGCCAAGGGATGGATCAGGGA
ATCCGCGTCCCAAGTGGCCAGTCCAACTATGTGGGTACCCAAGAAGGATGGACCCGATAG
ACTAGTTGTAGACTATAGAAAGCTTAACGCACTCACTAAGAAGGATCGATATCCACTTCCA
TTAGCTACGGAATTAAGAGATCGATTAGGCGGAGCTACGATATTTACCAAGATGGACCTAC
GTAATGGTTACCACTTGATCAGAATGAAGGAAGGCGAAGAATGGAAAACCGCTTTCAAAA
CAAGATACGGGCTATACGAGTACCAAGTTATGCCATTCGGGCTAACCAACGCACCAGCTAC
TTTCATGAGGCTTATGAACAATGTGTTGTCACAATATTTGGATACTTGCTGTATATGCTACTT
GGACGACATCCTAGTATATTCAAACAACAAGGTTCAACACATTAAGGACGTTAGCAACATC
CTCGAAAGCCT
ATCCAAGGCAGACTTGCTGTGCAAACCAAGCAAATGCGAATTCCATGTCACAGAGACAGA
ATTCTTGGGATTCACCGTATCAAGCCAAGGGCTCAAGATGAGCAAAGGCAAGGTTAAGGC
AGTGCTCGAATGGAAGCAGCCGACCACAATCAAGGAAGTACAATCCTTTCTAGGGTTCGT
CAACTTCTACAGAAGATTTATCAAAGGTTATTCAGGGATTACTACACCCTTGACCACGTTA
ACCAGAAAAGATCAAGGAAGCTTCGAATGGACTGCCAAAGCACAGGAGTCATTCGATAC
GCTCAAACAAGCAGTGGCAGAAGAGCCAATACTATTGACTTTTGACCCAGAGAAAGAAAT
CATAGTGGAGACGGACTCCTCGGATTTCGCTATAGGAGCAGTTCTGAGCCAACCGGGTCA
GAATGGAAAATACCAGCCAATCGCATTCTACTCCCGAAAACTATCACCAGCCGAATTAAAT
TATGAAATATACGACAAAGAATTACTGGCAATAGTCGATGCATTTAGAGAATGGCGAGTATA
TTTGGAAGGATCGAAATACACAGTACAGGTGTACACAGATCATAAGAACTTGGTTTACTTC
ACCACAACGAAGCAGTTAAACAGACGACAGGTCAGATGGTCGGAGACCATGGCCAACTA
CAATTTTAGAATTTCATATGTCAAAGGATCAGAAAACGCTAGAGCCGACGCTCTTAGCCGA
AAACCAGAATATCAAGAAAACAAAACGTACGAGTCATACGCTATATTCAAGAAAGACGGC
GAATCACTGGTTTACAATGCACCACAGCTTGCAGCAACACACCTGTTGGAAGACAACCAC
CTCAGGAAACAGATCCAATCACACTACGACAAGGATGCTACTGCCACACGCATACGCAAG
ACAATAGAACCAGGATTCACTATAGAAAATGATACCATATACTTTCATGGAAAAGTATACA
TTCCGAGTCAAATGACCAAGGAATTTGTGACGGAACAACATGGGTTGCCGGCACATGGA
CATCAAGGAATTGCAAGGACATTTGCAAGAATACGGGGAATCAGTTACTTCCCACGAATG
AGAACGATAGTTGAAGAAGTTGTTGGAAATTGTGACACCTGCATACGAAACAAGTCATCA
CGACATGCTCCGTATGGTCAGCTCCAGACCCCAGACATGCCTTCTCAGCCATGGAAGTCCA
TCACATGGGACTTTGTGATCAAACTACCACTCTCAAAGGATCCTACTACAGGAATTGAGTA
CGACGCGATACTCAATATAGTAGACAGGCTAACGAAATTTGCATATATGATACCATTCAAGG
AAACATGGGATGCTGAGCAACTAGCATATGTGTTCCTAAGGGTCATAGTAAGCATACACGG
AGTACCAGATGAGATAATCTCGGATCGAGACAAGCTCTTTACCTCGAAATTCTGGACTACC
TTATTAGCACTTATGGGTATCAAGAGAAAGCTATCGACATCTTTCCACCCACAAACAGATG
GTCAAACAGAGAGGACCAATCAGACAATGGAAGCATATCTTAGATGCTATCGTATAAAATC
CCGATACCACAAGAAGTTAATGCCGAAT CAGCGATAG
**SEQ ID NO:26 - LTR for siRS**
   >BC1G_16170.1 hypothetical protein similar to integrase (Transcript:BC1T_16170) 
ATGACCAAGGAATTTGTGACGGAACAACATGGGTTGCCGGCACACGGACATCAAGGGAT
TGCAAGAACATTTGCAAGAATCCGGGAAATCAGTTACTTCCCACGAATGAGAACGATAG
TTGAAGAAGTTGTTGGAAATTGTACACACCTGCATACGAAACAAGTCATCACGACATGC
GCCGTATGGTCAGCTCCAGACCCCAGACATGCCTTCTCAGCCATGGAAGTCCATCACATG
GGACTTTGTGGTCAAACTACCACTCTCAAAGGATCCTACTACAGGAATTGA
**SEQ ID NO:27 - Botrytis LTR genomic DNA sequence**
   >B. cinerea (B05.10) Botrytis cinerea supercontig 1.56 [DNA] 215700-227000 + 
CAAAGGGGGCATTACGCTTCCAACTGCCGAAACCCTGTTGTATGTCAACACTGTAAAGGA
AGTCACGGATCCAGAGAGTGCCCAGGAACTATGTCACAGCCTTCCCGACAGGGAAACGCT
TAGACCCAGCTGTTATTCTGAGCGTCCCACTGACGCTGGGTCCCCAAATAGAAGGACGTA
CTACCTTTACCATTATAGCAATGTTCCAACCAAAAGATAAGCCGATAGCGCTTCGATGCC
TTATCGACTCAGGAGCACAAGCCAACATCATCCAACAATCCAAGTGTATCGAATGGGACT
GGCTGCCTATTAAGAAAGGAACAGCTTTAGTATCTGCGAACGGTACCACGATGCCGTCGT
ATGGTAACCATCAGTTCCCCGTCGAAGTAAAAGATCAAAAGGGAGAGAAGAGAACCTTCA
CCCACGAGTTTACTGCTGCTGTACTAGACTTACCCAAAATCGATGCTATATTTGGATTAC
CCTGGCTACAAGCGGTAAACCCAGATATCGACTGGAAATCGACGTCTCTTCACTATCGCC
CCTCTCTTAGCGACCTCGAAATGATTTCTGCAAGCGAACTCTATAGCGAAGTGAAAAAGG
GCGTCCATGTATATGTTATACTACCAGAGATCCAGCCCCATTACCGTAGAGACAACGGGT
ACCGCCGGGTACTCACGCTCTCCACACTAAATATCCCCGAAGAATACCAAGAATACCAAC
AAGCCTTTTCCGAGGAAGAAAGCAGTACTCTACCAGAACACCACTCGATGGAGCATCGCA
TTGATCTCGAAGCCGATTCGAAACCTCCTTGGGGGCCAATCTATTCTTTATCTGAAGAGG
AATCAATAGTATTAAGGGAATACTTAGTAGAATATCAAAAAAAGGGATGGATAAGGAGGT
CCATTAGTTCGGCAGGAGCGCCAATCATGTTTGTTCCCAAGAAGGGGGGAGGCTATCGGC
TTTGTGTCGACTACCGGGGTCTAAATAGGATAACCAAAAAGGATCGAACCCCGCTACCCC
TAATCAGCGAGTCCTTAGACCGACTTCGACAAGGTGTCGTCTTCACTAAATTGGACCTGC
GAGATGCCTACCACCGTATTCGTATCAGGGAAGGCGACGAATGGAAGACGGCGTTCCGCA
CGCGGTACGGGCAATTCGAATACTTAGTTATGCCATTCGGCCTGACCAATGCTCCAGCAA
CGTTCCAAACATACATCAATCAAGCACTGTCAGGCTTGACAGACACCATATGCGTAGTGT
ACCTAGATGATATCCTGATTTACTCTGAGGATAGAGAAAGCCACACGCGGGATGTCCGCA
GGGTCCTCGAACGCCTTATAGAATACAAGCTGTTCGCAAAACTGAAAAAATGTGTCTTTT
ACACCCATGAGGTTGAATTCCTAGGATTCGTCGTCTCGGGAGCGGGAGTGACGATGGAAT
CCAGCCGCATTCAAACTATTATAGAATGGCCAACACCTACAAACCTTAGGGAGCTACAGG
TGTTCCTGGGCTTCGCGAACTTCTATCGACGGTTTATCAGGACCTATTCGACGGTAGCCC
ACGGGATGACCGCCCTTATGAAGGGAACAAAGAAAGGTAAAATGGTAGGGGAGTTTATAT
GGACAAAGGAGGCCCAAGATGCATTTGAGGCACTAAAGAAAGCATTCACCACGGCACCGA
TACTCAAGCACTTCGAACCATCGCTCCGCATCATGGTCGAAACCGACTCGTCGGTGTTTG
CTCTAGGATGCATCCTATCGCAACTATTCGAAGGAGGGACTGCAGAAGCACCGATACGAC
 
GGTGGCACCCCGTCGCGTTCTATTCGAGAAAGCTGAACCCTGCAGAACAACGATACTTCA
CTCACGATCAGGAATTATTAGCAATATACACTGCATTCATGCAATGGCGCCATTACTTGA
TAGGTAGTCGGCACACAATCGTGGTGAAATCGGACCATAACAGCTTACAACATTTTATGG
TGAAAAAGACCCTCAATGGCAGACAAGCTAGATGGGCGGAAGTACTAGCAGCCTACGACT
TCGAAATAGTGTACAGGGCAGGGAAACTGAATCCAGCCGACGGGCCATCGCGCCGCCCCG
ACTACGCTACCGACACGGAGGGTATCAATGATATGCTACCCACACTCCAGAATAAATTAA
AAAGTACCGCAGTTATCGCGAGTTTATTTTACGAATCCACCGTGAAAACGGAACCCCTGC
GTATTGCTATTAGTCGCTTGCAAAGGGAAGGGTATAGCTTGCCATTACGTGGACAGTTAG
TTTCACTGGTAAAAACTGGTTGCAAACAGTCGATACCACGTCGGATTGCCAGTGTTTTCG
CATCCGACGAAACGGCATTCGAACCTATATCGGAGTCGATGGGAAAAGCTTTATTGCGGC
TTCAGAAAGAAGACGATTTTATAAAGAATAAAGAGTACCTAAGACAAAGATTACGTTCCG
CCGGAGACGCCTCACCACGGCAGGTGGGCGCCGACGAGCTCCTTAGACACAAGGGGAGCG
CGTACGTACCGCCAGACAGCGCTCTCAGAGCAGAAATCTTAGAAACGCATCACGATGACC
CTATTGGAGGTCATTGGGGTGTCGCTAAAACATTGGAAATACTGAAGTCTAAATATTATT
GGCCTTCAATGAGAAAAGACGTCAAACAACATGTCAAAACATGTGCGGTATGCCAGCGAA
CCGCTATCAAAAGACATAAGCCACACGGCGAGTTACAGACCCTCCCTATTCCAAAAGGAC
CCTGGAAAGAGATAACTATGGATTTTATTACAGATTTACCTCCTTCGAAACACGGAAAAC
ACGTATACGATTCTATTCTAGTAGTAGTCGACAGGTTCACGAAGCTAGCCCGATATATCG
CCGTCAACAAGACGATATCGTCTCCTGAATTAGCTGACACTATGGTCAGCACAGTATTTA
AAGACTTTGGTGTGCCAGAGGGCATAGTCTCCGATAGGGGACCGCAATTCGTCAGTAAAT
TTTGGAGTAGCCTAATGTTTTACTTGCGAATCCGTCGTAAGCTGTCGACGGCGTTCCACC
CGCAGACCGACGGTCAAACCGAACGACAAAACCAAAATTTGATTCACTATATAAGTTGCT
ACACCAACTATAGGCAAGACGACTGGGCATCGCTATTGCCCCTTGCTGAATTCACATATA
ACGCGACATGGCACAGTACAACCAATACAAGCCCATTCCAGGCTATGTATGGGTTCCAAC
CCACATTCCATTATATCGGCGAGGACGCCGATTTAGAGGGAAGGGCGCCGGCAGCACGCG
AGCGCATCGACGCTTTAGAGAAAGAAAGAGAAAAGCTGAAAGAATTCTGGAAATCGGCAA
CCAATCAGAAAAACAAGAACACTACGAAGGGGTCACCACAGCGATATAGCATCGGGGACA
AGGTGATGCTAAGCACAAAGAACATTAAACAACTGCGACCTAAGAAAAAATTCTCCGATC
GATTTATAGGCCCCTTTGTCGTGACGGGTATAAAAACCAGCGGGCAAGCATACGAACTTA
GATTACCGCCCACCTACAAGATCCACAATGTATTCCACGTCTCTTTACTCGAACCATGGC
ACGAACGACAGGGTACCGCCGACCCGCCGCCGCCAGAAGAAATTGACGATCACATAGAGC
ATGAGGTGGAAAGGATTTTAGCACATAGAAAAAGAGGCAGAGGTGTGCAATACCTGGTGC
GATGGAAGGGCTACCAACCGGCGGAAGACACGTGGGAAGCACCCTACAACCTAGAAAATG
CGAAAGCAGCGATGGGAGAATATCATAAAGAAGAAGCATTACCAATACAGAAAAAGAAGA
 
GAACAAGAAAAAAGCTTAAAAATACTTGATACAAAAAGACCTCACGAGACCCACACCCAG
AACGCATGCATCACACCAAGCTACCCAAAAAGGACTATCCAACAAAGAAACCAGAAAGGA
CAACTCCTCCGAACCCACAGCATACCGACAACCAAACCCAAGTGACCCATCACGCAAAGA
GAACTCTGGAAGGTCGAAATCAGTTCCCTAATTCATCTGCCTGATCCAGGAGGTCAGCTG
CAATATCTCGATCGCCAAGAAGGACAGAACCTACATCGCTGGCATATCCCCCGGGACTCG
CGAGCCCGATCTGATCAACCTCACCCCCCCCACTGATCTCATCTTGATCACCGACTCCTT
CCTCCTCGTTCTCGCGAATCTCGCTGACAGGGCGAGCTCCTGTCGGAAAACCAGCGGCAA
CCCGTTGCCTCTTGGAGACCCGCTCCACATCAGAATTCTCAGAGCGAAGCCTTTGGGGCG
AATCTGCTTGACTATCAGACTCACCAATATAGACTTGTTGAACAGTGCTGGGAGCCCTCT
TTCGGGATTTAAGAGAGCCTACGGGATGAGAGCGGGGTGTCGGTGTGTTGCGAGAGGCAA
GCTGCGACGACTTAGAAGCGGAAAGGGGAATGGCGGATCTAGTCGCGAGCTTATCGGACC
GAGAACGACGAGGCGTAGCAGGAGGAGCGTTCTTTCTACCCAAGCTTCCAAACAATAAAG
CCGGTACACTCTCATCCAAGGGGTGACGCGGGGCACCGGTGGTCTGATGAGGTTGATCCG
ACACATCATCCTCTTCAGGAGCAGACTCTTCGGATTCATTGGGATCCACGATTACACTCC
TCTTCAACTTCGCAGAACCTTTCCGGAGGCCTTGCTGAGCCGTCGTGAAGACAGCGGGGC
CCGTCCGGCTCGATGAAGCAGAAGCATGACGGCGACCTAGGGAACCAGCTACCATATTCA
GGCCCTGGTTACCACCTGGGGGCCCCGGAGGAGCTTGAACACCCTGCAGTGCGTCGGCGA
CAGCGCGCACAGCGCGAGGGTGTATAACCGGAGCCATGAACTCCCCTTCTTCGTCCGAGT
TTAAGGCGGCGATTAATGGTGCAGTAGGAGCTGCGGCGGCGACGTCCAAGGCAGGCAGGT
TATTCTAAGAACCATCAAATCAGCTATCAACAGCACCAAGGGGCAAGAGCAGGGCAACCG
ACAACGAATCGATACGAATCCACAATGCGCTCCATAAGATCGCCAATTCTACTCAATTTG
GACACCAGAAGAAGAGTCAATTCCTCTGCGGACTGCGGTTTGTGAGTCCCCCCTTGTTTA
ACCTCTTTTCGAAGGAAAGCCTCCACTGCCTTGACGTATCGATTACACCGGCGAATCACC
ACCTCGGAGGCGTCCTCATCCTCTTGGCTATTATCCGGAGTAAGGCACCAAAAGGCATGA
GTCTGATATAAGAGGTTCACGTCGGGAACAAAGCGAGGAGGTATCGGGAGGCAGACTTTC
TTTTGTTTGCGACAATAACTACATTTAGCAGCAGGACCTTTATCAAAATGGCAAAATTCG
TCGTTAGAAGAGACGGCAATTCGCTTAGAACATCGAAGGCAAGTAGGGATAACTAACGCG
GAAGGGGCCGCTGCGGACCGATCAGCGATAGCAGCCGGAGCGATGGGTTGCAGTGCAGCC
ATCTTCGTATGAGTAAATAAGGGGGAATAATCTGATTGTGGGAGATATATCAGAGGCAAG
AAGACCCCCCTTATAGAACTATCGGTGATCTGCCGGTAAGGCGGTGAGGCGCGTAAGAAT
GCCGCCGTTTGCTTGTTTATTGTTTGTAATGCCTAAACAAGATTGGAATTGCTTTTGGAA
TGCGGCGCAGGGTCGGGCATGCAGCGACGCGACGACGCGACCCACATTCCGAGTAAACAA
TACGGAAGGAAGCAAACACTTCTCGGGACGCGAAGTGTAAAGAGAGGGGCTCTGTTACGG
GACAAAACGTGACCGGCTCAATTAGGCACGTGACAGTGGACCTCTCGGGTCTACTGCGTG
 
CCGAATGGGGCCCGCACACGTATAAATTGTATAATTTGCATAGTTATAGAAAAGCAATGA
AAAGTCTTGGTGCCACAATATACTAGTTGATTCATTTGTTACGGAGGTACCCGCACCGCA
ACATGGATTATAAGATAAACCTAAGGCCTTGGTGTTGGAACCTACGAAAACAGCACTGTA
GGGACAGTTGAATTAAAGGGTAACTAAAGATAGCAGTAACCGAATCAATAAGCAATGATT
AAAAGATAGGTACCTATCTTTTGTTGGCACCTACCCTACAGTAGGCACAGGAGGGATAGC
GGTTATAGGTTATCTAGTAAGCACAGGTTAGATAAGCAGTAGTATCATGTAGGTCACGGG
GCAAGTGTCACGTGATGGATAGACAGGATAGGCAGGCTATCCAGGCTATCCGTGGATAGA
CAGGATAGACAGTCTACCCAAGCTATCCAGACGAGAACGAAGGTCTATATAAGGGAATGG
GTTTCATTACAATGTAGAGCTTCGTGCTCAAGAACAATCATTAGTTTCATTACTATAGTT
ACGAGAATTGCAACCAGTTACAACCTTATTGAATTCCTACTTGAAGTCTAGTCTAAACCA
CCTCGAGAGATCTCTAGACACTTCCACGTGACCCTAGAGGCAGCTCCCGTAACACTTTGA
GCACCCTTTCTGCTTCAAGTACCGATTCGATAACCAACCGCTAATATGGCATCCAGAGCT
ACCGCCACAGGTCAGTCTACCGAAGATACCAACGACATCGAGATGACCGATGCCCCAAAG
GAGATCACTATCAACGAAACACTTAAGATCGCCTTACCAGACAAGTACCAAGGTAGTCGA
CAAGAGCTCGATACTTTCCTCTTACAACTTGAGATCTACTTCCGATTCAATGAAGACAAG
TTCACTACCAAGGAATCCAAGAGCATATGGGCTGCATCATACCTCCGAGGTGAAGCAACC
AAATGGATTCAACCATATTTGCGCGACTATTTCGAGCATGACGATAAGGATCGCATGCAA
CCCACCCGAACAATCTTCAATAGTTTTGAAGGATTTAAGACAGAGATTCGTAGAATCTTC
GGAAATTCCAACGAGTTAGAGGTAGCGGAAGATAAGATCTTCAACCTCAAGCAGACAGGA
TCAGCATTGAAATATGCTACGGAATTTCGAAGATATGCTGGAACAACCAAGTGGGACGAA
ATCGCTATCATGAGTCACTACTGTAAGGGACTCAAACCAGAAGTCAGACTAGAGTTAGAA
AGATCTGCCGAGAGTACAGATCTGAACGATCTAATTCAGGACTCCATCGAATCAGATGAT
CGTCTCTATAGATATCGACAAAGCCAAAGATCATACAAACCCCAAGGAAACCAAAAGCAA
GGGCGTTACCGCAAGAATGAGGGTAGACCACGTTACAATCCACAGAGATACGGAGACCCC
ATGGAACTAGACGCCACGCACTACACAAACGGGAACGATGACTCAGAAAAGAGACGAAGA
CGAGAAAACAACTTATGCTTTGAATGTGGAAAAGCAGGGCACCGAGCAGTAGACTGCCGA
AGCAAGAAGACAGGAGGAAAAAGGGGCAACTTCAAACCTAAGTTCGGCAAGGGCCAACTT
AACGCCACCTTTGCCATCTCAGAAAACTCAACTAAAACCGAAAATACTGAGACTTTCACC
GTTGAGGAATTTCAGCAATTACTAAAGGAATTACCACGAAATAAAGAGGGCATGAATGCA
ATAGACTTATGGGAACAAGAGTATTACAGAACCCCAACACCCTCTGTGACAGAAGAAAGT
CACCAGGACGAGGCAGAAGCGGACCACGCCACGATGAGCTGGACAGCTTGCTATGATGAA
TTCTGCGGAATCCATCGATCAGATAAAGAAGCAACCGGATGGTTCCCCAAGAAAAGGAAG
 
ACGAAGAACCATCAGAATAATGTAACATGCGAGGATTTAACTCCCAATATAACTTCGCAA
GAAGTTCGCAAAGTTACCCAGCAGTTGAATGCTACGGGACAGGCAGGACAAGTGTACTGC
AAGGTCCAGATAAATGGACACATACAATCAGCCATGATAGATTCAGGGGCTACAGGAAAT
TTTATTGCACCAGAAGCTGCAAAGTACTTGGAAATACCACTTCAAACGAAACAACACCCC
TATCGATTGCAGTTAGTTGATGGACAGCTAGCAGGGTCTGACGGAAAGATTTCGCAGGAG
ACAATCCCAGTACGAATGGGCATAACCCAACATACAGAGGTTATACAGCTTGACGTTGTG
CCATTGGGCCAACAACAGATCATCTTAGGAATGCCATGGTTGAAGGCACATAATCCGAAA
ATAGATTGGGCACAAGGAATTGTGACATTTGATCAGTGCAAAAGCGGTCACAGGGACACG
CTAGAGGCGTCCGCGAGACGTAACACGCGCCAAGGGGAGTTGAACGCGAACAACACCGGC
GACGTAGGACACCCAGTCCAGGGTCCTCCATTAAGAGCGAAGGCCAGTACACCTCCTCTA
CAAATGCAGAAGCCAACGACACGGCACGAAATCGCAATCGAGGCAAAAGAAAGGCCTACG
ATACCAGAACAGTACAAGAAATATGAACATGTTTTCAAAGAACCAGGGATCCATGAGGCT
TTACCGGAACACAAGCCATGGGATCATGAGATAATATTGGAGGAAGGCAAGATGCCTGTG
CACACCCCAATTTATTCAATGTCAGCCGATGAGTTAAAAAGGCTCAGAGAATACATCGAC
GACAATTTAGCCAAGGGATGGATCAGGGAATCCGCGTCCCAAGTGGCCAGTCCAACTATG
TGGGTACCCAAGAAGGATGGACCCGATAGACTAGTTGTAGACTATAGAAAGCTTAACGCA
CTCACTAAGAAGGATCGATATCCACTTCCATTAGCTACGGAATTAAGAGATCGATTAGGC
GGAGCTACGATATTCACCAAGATGGACCTACGTAATGGTTACCACTTGATCAGAATGAAG
GAAGGCGAAGAATGGAAAACCGCTTTCAAAACAAGATACGGGCTATACGAGTACCAAGTT
ATGCCATTCGGGCTAACCAACGCACCAGCTACTTTCATGAGGCTTATGAACAATGTGTTG
TCACAATATTTGGATACTTGCTGTATATGCTACTTGGACGACATCCTAGTATATTCAAAC
AACAAGGTTCAACACATTAAGGACGTTAGCAACATCCTCGAAAGTCTATCCAAAGCAGAC
TTGCTGTGCAAACCAAGCAAATGCGAATTCCATGTCACAGAAACAGAATTCTTGGGATTC
ACCGTATCAAGCCAAGGGCTCAAGATGAGCAAAGACAAGGTTAAGGCAGTGCTCGAATGG
AAGCAGCCAACCACAATCAAGGAGGTACAATCCTTTCTAGGGTTCGTCAACTTCTACAGA
AGATTTATCAAGGGTTATTCAGGGATTACTACACCCTTGACCACGTTAACCAGAAAAGAT
CAAGGAAGCTTCGAATGGACTGCCAAAGCACAGGAGTCATTCGATACACTCAAACAAGCA
GTGGCAGAAGAACCAATACTGTTGACTTTTGACCCAGAGAAAGAAATCATAGTGGAAACG
GATTCCTCAGATTTCGCTATAGGAGCAGTTCTGAGCCAACCGGGCCAGAATGGAAAATAC
CAGCCAATCGCATTCTACTCCCGAAAACTATCACCAGCTGAGTTAAATTACGAGATATAT
GACAAAGAATTACTGGCAATAGTCGATGCATTTAGAGAATGGCGAGTATATTTGGAAGGA
 
TCGAAATACACAGTACAGGTGTATACAGATCATAAGAACTTGGTTTACTTCACCACAACG
AAGCAGTTAAACAGACGACAGGTCAGATGGTCGGAGACCATGGCCAACTACAATTTCAGA
ATTTCATATGTCAAAGGATCAGAAAACGCTAGAGCCGACGCTCTTAGCCGAAAACCAGAA
TATCAAGAAAACAAAACGTACGAGTCATACGCTATATTCAAGAAAGACGGCGAATCACTG
GTCTACAATGCACCACAGCTTGCAGCAACACACCTGTTGGAAGACAACCACCTCAGGAAA
CAGATCCAATCACACTACAACAAGGATGCTACTGCCACACGCATACGCAAGACAATAGAA
CCAGGATTCACTATAGAAGATGATACCATATACTTTCATGGAAAAGTATACATTCCGAGT
CAAATGACCAAGGAATTTGTGACGGAACAACACGGATTGCCGGCACATGGACACCAAGGA
ATTGCAAGGACATTTGCAAGAATACGGGAAATCAGTTACTTCCCACGAATGAGAACGATA
GTTGAAGAAGTTGTTGGAAATTGTGACACCTGCATACGAAACAAGTCATCACGACATGCT
CCGTATGGTCAGCTCCAGACCCCAGACATGCCTTCTCAGCCATGGAAGTCCATCACATGG
GACTTTGTGGTCAAACTACCACTCTCAAAGGATCCTACTACAGGAATTGAGTACGACGCG
ATACTCAATATAGTAGACAGGCTAACGAAATTTGCATATATGATACCATTCAAGGAAACA
TGGGATGCTGAGCAACTAGCATATGTGTTCCTAAGGGTCATAGTAAGCATACACGGAGTA
CCAGATGAGATAATCTCGGATCGAGACAAGCTCTTTACCTCGAAATTCTGGACTACCTTA
TTAGCACTTATGGGTATCAAGAGAAAGCTATCGACATCTTTCCACCCACAAACAGATGGT
CAAACAGAGAGGACCAATCAGACAATGGAAGCATATCTTAGATGCTATGTAAATTATCGA
CAAGACAATTGGGTAGAGCTATTACCCATGGCACAGTTCGCATACAATACATCGGAAACG
GAAACCACGAAAATCACCCCAGCACGAGCTAATTTTGGGTTTAATCCACAAGCGTATAAA
ATCCCGATACCACAAGAAGTTAATGCCGAATCAGCAATAGTACAAGTCGAACAGCTGAAA
GATCTCCAAGAGCAACTGGCTCTTGATCTAAGATTCATATCTTCCAGAACAGCAGCGTAC
TACAATACGAAACGTAGTATGGAACCTACGCTTAAAGAGGGGGATAAAGTTTATTTGCTA
CAACGAAACATCGAAACCAAGAGACCAAGCAATAAACTCGACCACAGGAAAATAGGACCA
TTCAAGATTGATAAGGTAATAGGAACGGTTAATTATCGATTGAAATTACCAGACACAATG
AATATCCACCCAGTATTCCACATATCCTTGCTCGAACCAGCACCACCAGGAGCGCCAAAT
GCGCCATTTACAGAAATCGAACCAGTCAACCCAAACGCCATATACGACGTTGAAACAATA
CTAGATTGTAAATATGTCAGGGGCAAAATCAAGTATTTGATCAAATGGTTAGACTACCCA
CATTCGGAAAACACATGGGAA
**SEQ ID NO:28 - Botrytis DCL1 promoter sequence**
   >B. cinerea (B05.10) Botrytis cinerea supercontig 1.69 [DNA] 45790-46725 - 
GAAGAGGTTGTTGGCAATATTTTGAAGAAAGCTGAGGCTGATTTGAATGGAGATTAAAA
GGGGAATGAAGCTGCGGGGCCACCGATAGCACAAAAACTACTGAAGATTTGAAGCACGT
TAAAATTACACTCAGGAATAAACGGATGGCAAGCTTTTCGATCGCCCAAACACGGATCT
ACGACTACGAGTTACGCACGACATGATTTAGCCTTTTGTGTGCAATGATGATTAGATAGC
ATTGCATTTCTCGAAATTGACGGCACGACTTTTACGGGCAGATAATATCAAAGATTCCTA
 
GTGAGCAAGCGGTGATGATACGATGTCATTCCAAAAGTTTTTTCCTCGCGAATTTTATTTC
ATTTCGAAGGCATCTTTGCTTAGCAGCATATTCACCTTTGATGTCCTCTGTAGGGGATGG
AGTCTCTAATCTCGCGGTCACAATGAGACGTGATGCGCTGCGAAGTGGTGACAATTTCCC
TTTACTTAGAATAGATCATGCACACATGCATGATGCATAGCTAGCTAGTTTTTTATTCAAT
GATAGTTTAATGACAAACACGTATCTAGATATCCTCATTCATGTATCTGTGGGAGGTTGA
CTTAAGTTATGGCTGACTTGATAGTTTCATTATATATGTATATGTGATATCTAAGTAAAGA
TTAAAGTGAAATCGAAATGCAACGCCGAAATTCTATTAATTCCATGAAATGATGTGATAT
GGCATGACATGATATCCAAACTCCGATTTGAAATGCTCCAGCTTCGCTTTCTAAAATTGG
TAAAAGGGACATTATTTCGTCTGGTTGTGGGTTTTCATTTCTGTGCTCCTACTAGGTGTGA
ATGATAGAGTATGCTGTGGTGTGGTGTGATCTCGGAATTTGGAAATTTGAGGGCTGTATA
TCACCTCATTTCGTGTGTCCGAATTTCTACAGACT
**SEQ ID NO:29 - Botrytis DCL2 promoter sequence**
   >B. cinerea (B05.10) Botrytis cinerea supercontig 1.78 [DNA] 26792-27461 - 
AGAGCATTTGTAGGGGAAGGAGGAAAAATTGAGGAGGAGGATAAGATGAATTTTGATA
AATTTATTTCCTAACATCAGGTCACAATCTATGAATTACATTTGATAGTATTACGTATGCC
GGTCTGTACACAACACAACCATATAGTAAGGTATCAATCAAATGCGATGGATAGTCATTT
CAATTTCTTAGTGAATAATTACAACGAACCAGTAAAATAGCAATAACTCTGAAAAGCTTC
CGGACTGCCAAAAGGTCTCCAGGACGAGATTATTACGAAGAACCCAAGAATTCGCCTAG
GAACCAAGATAAACAAATCATCGACGTGTTGCACTTCCATCTATGCGACAATTATGCCAA
GCGAGCCGCCAGTTCTTGGGGGTGGAGCGCTAGGAATAGGGGGCCGGATTGCCATATCC
TTATCTAGATCTAGATGGTATCGATATGATAAATCAATGCAATGGAGAGTTAAAAAGTTA
TATGCCATATGATTGATAATTATTGACAATGCAGGCTATCGCGGGACAATGGTAAATGGT
TGTAAAATATGGAGTCTATTTCCTTAGCTAGCGATAAGATGGGTGGTTTAAACACATCCC
GCCTTCTCTTTATCATTCTCCTTCTCGTATTCATATATCATAATTGCAAAGTAAGGTTGTA
TTTTGGACTGTG
**SEQ ID NO:30 - Verticillium DCL1 promoter sequence**
   >V. dahliae VdLs.17 supercont1.1 of Verticillium dahliae (VdLs.17) [DNA] 1574620-1574964 - 
AAGCTGTCAATTGATGCGGAGGGTGAGTGAACGTCTCGTCGGCGGGGCCCCTTGAGGCG
AGCGCCCGTTGGGGGGTGTTGTGGCACTAGGTTCTCTAGGCCGGCGGTGACTTTCATTAC
TATATTAGAAGCAAATACGGCGCCTTCATCACAATAATAAATATCGATCTCGAGTCGATT
CCAGACCCGTTATAAACCTATGTCTGTGCAACCAGTTGGGTGCTAATTTCTTGCATTATCA
TCATGGATGTTGTCTATTTGAGTCTCAGGTCCAGCTGGTGCTTATAGGTCATCTCCAGTAT
GCGACTACCTCTCTCCCTCTTTGCCATTCCTAACTGATTCTAAC
**SEQ ID NO:31 - Verticillium DCL2 promoter sequence**
   >V. dahliae VdLs.17 supercont1.15 of Verticillium dahliae (VdLs.17) [DNA] 194566-195565 +

CTTCATCTTCCAACCGCCATTACCTCCCCCATACGCGTCCTGCCAAAGAATCATAACTGG
CTAAAACATAAGACGGGACTGGTCATCCGCTGAACCATTCCGAGCTATGTGTCCTGATTG
ACCCATCTCGGCTTATTCGCTCTCAAATACGACTGCAATCGCGTGTGGCTTGGAAACCGT
GGAATACCATCCTCATATTGTCAGCACCTGTAGCGATACAGCACAATGCTTGACGATTCG
GAATCATTTTCCGCTTCTTTGCGGAGCAGCGGATGTCCAATTGACGATGACTTGACTCCA
GAACCAACGTCCGAATCACGCGACTCAACCTCCCTACCGTATGGCCTTCAGGACGACATC
GGCCCCCTTGCTGCCACCCCGAGCCAGTCGAGTAACGTCACAATCAATGCACGGGCATA
CCAGTTGGAGATGCTGGCGGAAAGTAGGAAGAGGAATATCATTCTAGCTGTGCGAACCT
TCCCTCTTGCGCCAGTCAACCTCGATTGACACCTCCATAGATGGACACAGGCAGTGGCAA
GACCCAAGTGTACGTTCCCTGCAAGCCGAACCTGATTATTGATACTGATTTTCCCAGTGC
CGTCCTCCGAATTCGAGCAGAGCTAGAAGAAGGGGCTTCAGACAAGGTTTGACAAACTC
CACTTGGTAGCTTCGCAATCACTTACAGGGTTTTAGCTTGTATGGTTCGTGGCTCACAATG
TTGAGCTTTGCGCTCAGCAGCATTCTGTACTGCAGTCTCAGATTCCTGCAGTTCAGACCA
AGCTGCTTCTTGGCAGCGATAATGTTGATTCATGGTCCAACCAAGAGACTTGGAACGCTG
TGCTTCTCAACGTCAAAATTGTGGTGTCAACCCCTCAAGTTCTCTGCGATGCCTTGAGCC

ACGGCTTTGTCCAGATGGGTTCATTATCCTTGCTTGTCTTTGATGAAGGTATTCAATCAGC
GCAGTTTATCAAGTGTTCTTGCCCTAACAACGGTGTAGCGC

## Claims

1. A method of increasing pathogen resistance in a plant or a part of a plant, the method comprising:
contacting the plant or the part of the plant with a first double-stranded RNA, small RNAs, or small RNA duplexes that target a first fungal pathogen dicer-like (DCL) gene or promoter, and contacting the plants or part of the plant with a second double-stranded RNA, small RNAs, or small RNA duplexes that target a second fungal pathogen DCL gene or promoter, wherein
(a) the first double-stranded RNA, small RNAs, or small RNA duplexes target *Botrytis* DCL1 and the second double-stranded RNA, small RNAs, or small RNA duplexes target *Botrytis* DCL2; wherein the first and second DCL gene or promoter comprise a sequence of SEQ ID NO:1, SEQ ID NO:3, SEQ ID N0:9, SEQ ID NO:10, SEQ ID NO:28, or SEQ ID NO:29, or a fragment thereof comprising at least 50 contiguous nucleotides, or
(b) the first double-stranded RNA, small RNAs, or small RNA duplexes target *Verticillium* DCL1 and the second double-stranded RNA, small RNAs, or small RNA duplexes target *Verticillium* DCL2; wherein the first and second DCL gene or promoter comprise a sequence of SEQ ID NO:5, SEQ ID NO:7, SEQ ID NO:11, SEQ ID NO:12, SEQ ID NO:30, or SEQ ID NO:31 or a fragment thereof comprising at least 50 contiguous nucleotides,
wherein the plant is a species of the genera *Asparagus, Atropa, Avena, Brassica, Citrus, Citrullus, Capsicum, Cucumis, Cucurbita, Daucus, Fragaria, Glycine, Gossypium, Helianthus, Heterocallis, Hordeum, Hyoscyamus, Lactuca, Linum, Lolium, Lycopersicon, Malus, Manihot, Majorana, Medicago, Nicotiana, Oryza, Panieum, Pannesetum, Persea, Pisum, Pyrus, Prunus, Raphanus, Rosa, Secale, Senecio, Sinapis, Solanum, Sorghum, Trigonella, Triticum, Vitis, Vigna,* or *Zea,* and wherein the plant or the part of the plant has increased resistance to the fungal pathogen compared to a control plant or control plant part that has not been contacted with the double-stranded RNA, small RNAs, or small RNA duplexes.

2. The method of claim 1, wherein
the double-stranded RNAs, small RNAs, or small RNA duplexes are sprayed onto the plant or the part of the plant.

3. The method of claim 1 or 2, wherein the plant is an ornamental plant.

4. The method of claim 1 or 2, wherein
(i) the plant is a fruit- or vegetable-producing plant; and/or
(ii) the part of the plant is a fruit, a vegetable, or a flower.

5. A method of increasing pathogen resistance in a fruit, a vegetable, or a flower, the method comprising:
contacting the fruit, vegetable, or flower with a first double-stranded RNA, small RNAs, or small RNA duplexes that target a first fungal pathogen dicer-like (DCL) gene or promoter, and contacting the fruit, vegetable, or flower with a second double-stranded RNA, small RNAs, or small RNA duplexes that target a second fungal pathogen DCL gene or promoter, wherein
(a) the first double-stranded RNA, small RNAs, or small RNA duplexes target *Botrytis* DCL1 and the second double-stranded RNA, small RNAs, or small RNA duplexes target *Botrytis* DCL2; wherein the first and second DCL gene or promoter comprise the sequence of SEQ ID NO:1, SEQ ID NO:3, SEQ ID NO:9, SEQ ID NO:10, SEQ ID NO:28, or SEQ ID NO:29, or a fragment thereof comprising at least 50 contiguous nucleotides; or
(b) the first double-stranded RNA, small RNAs or small RNA duplexes target *Verticillium* DCL1 and the second double-stranded RNA, small RNAs, or small RNA duplexes target *Verticillium* DCL2; wherein the first and second DCL gene or promoter comprises the sequence of SEQ ID NO:5, SEQ ID NO:7, SEQ ID NO:11, SEQ ID NO:12, SEQ ID NO:30, or SEQ ID NO:31 or a fragment thereof comprising at least 50 contiguous nucleotides, and
wherein the fruit, vegetable, or flower has increased resistance to the fungal pathogen compared to a control fruit, vegetable, or flower that has not been contacted with the double-stranded RNA, small RNAs, or small RNA duplexes.

6. The method of claim 4(ii) or 5, wherein the fruit, vegetable, or flower is a post-harvest fruit, vegetable, or flower.

7. The method of claim 5 or 6, wherein the double-stranded RNAs, small RNAs, or small RNA duplexes are sprayed onto the fruit, vegetable, or flower.

8. The method of any of claims 1 to 7, wherein
the double-stranded RNA, small RNAs, or small RNA duplexes target any of SEQ ID NO:9, SEQ ID NO:10, SEQ ID NO:11, or SEQ ID NO:12.

9. The method of any of claims 1 to 7, wherein the double-stranded RNA, small RNAs, or small RNA duplexes comprises an inverted repeat of a sequence that is at least 70% identical to any of SEQ ID NO:1, SEQ ID NO:3, SEQ ID NO:5, SEQ ID NO:7, SEQ ID NO:9, SEQ ID NO:10, SEQ ID NO:11, or SEQ ID NO:12, or a fragment thereof that comprises at least 15 contiguous nucleotides, or a complement thereof, optionally wherein the double-stranded RNA is siRNA.

10. The method of any of claims 1 to 9, comprising the use of two or more double-stranded RNAs or small RNA duplexes.

11. The method of claim 1, comprising increasing pathogen resistance to a first and a second species of fungal pathogens, wherein the method comprises:
contacting the plant or the part of the plant with
(i) a first double-stranded RNA, small RNAs, or small RNA duplexes that target a *Botrytis* DCL1 gene or promoter and a second double-stranded RNA, small RNAs, or small RNA duplexes that target a *Botrytis* DCL2 gene or promoter; wherein the DCL1 and DCL2 gene or promoter comprises the sequence of SEQ ID NO: 1, SEQ ID NO:3, SEQ ID NO:9, SEQ ID NO:10, SEQ ID NO:28, or SEQ ID NO:29, or a fragment thereof comprising at least 50 contiguous nucleotides; and
(ii) a first double-stranded RNA, small RNAs, or small RNA duplexes that target *Verticillium* DCL1 gene or promoter and a second double-stranded RNA, small RNAs, or small RNA duplexes that target a *Verticillium* DCL2 gene or promoter; wherein the DCL1 and DCL2 gene or promoter comprises the sequence of SEQ ID NO:5, SEQ ID NO:7, SEQ ID NO:11, SEQ ID NO:12, SEQ ID NO:30, or SEQ ID NO:31 or a fragment thereof comprising at least 50 contiguous nucleotides;
wherein the plant or the part of the plant has increased resistance to the first species of pathogen and the second species of pathogen compared to a control plant or control plant part that has not been contacted with the first and second double-stranded RNAs, small RNAs, or small RNA duplexes of (i) and (ii).

12. The method of claim 11, wherein:
(i) the first and second double-stranded RNAs, small RNAs, or small RNA duplexes of (i) and (ii) are sprayed onto the plant or the part of the plant; and/or
(ii) the part of the plant is a fruit, a vegetable, or a flower.

13. A method of making a plant having increased pathogen resistance to a first species and a second species of pathogens, wherein the first species of pathogen is a species of *Botrytis* and the second species of pathogen is a species of *Verticillium,* the method comprising:
introducing into the plant by transformation
(1) a heterologous expression cassette comprising a promoter operably linked to a first polynucleotide that inhibits expression of a *Botrytis* DCL1 gene or promoter and a heterologous expression cassette comprising a promoter operably linked to a second polynucleotide that inhibits expression of *Botrytis* DCL2 gene or promoter; wherein the DCL1 and DCL2 gene or promoter comprises the sequence of SEQ ID NO:1, SEQ ID NO:3, SEQ ID NO:9, SEQ ID NO:10, SEQ ID NO:28, or SEQ ID NO:29, or a fragment thereof comprising at least 50 contiguous nucleotides;
(2) a heterologous expression cassette comprising a promoter operably linked to a first polynucleotide that inhibits expression of a *Verticillium* DCL1 gene or promoter and a heterologous expression cassette comprising a promoter operably linked to a second polynucleotide that inhibits expression of *Verticillium* DCL2 gene or promoter; wherein the DCL1 and DCL2 gene or promoter comprises the sequence of SEQ ID NO:5, SEQ ID NO:7, SEQ ID NO:11, SEQ ID NO:12, SEQ ID NO:30, or SEQ ID NO:31 or a fragment thereof comprising at least 50 contiguous nucleotides; and
selecting a plant comprising the expression cassettes.

14. The method of claim 13, wherein the first and second polynucleotides of (1) and (2) encode antisense nucleic acids or inhibitory RNA (RNAi) that target the DCL gene or promoter or a fragment thereof.

15. The method of any of claims 11 to 14, wherein:
(i) the plant is a species of the genera *Asparagus, Atropa, Avena, Brassica, Citrus, Citrullus, Capsicum, Cucumis, Cucurbita, Daucus, Fragaria, Glycine, Gossypium, Helianthus, Heterocallis, Hordeum, Hyoscyamus, Lactuca, Linum, Lolium, Lycopersicon, Malus, Manihot, Majorana, Medicago, Nicotiana, Oryza, Panieum, Pannesetum, Persea, Pisum, Pyrus, Prunus, Raphanus, Rosa, Secale, Senecio, Sinapis, Solanum, Sorghum, Trigonella, Triticum, Vitis, Vigna,* or *Zea;* and/or
(ii) the plant is an ornamental plant or a vegetable- or fruit-producing plant.

## Patentansprüche

1. Verfahren zum Erhöhen der Pathogenresistenz in einer Pflanze oder einem Teil einer Pflanze, wobei das Verfahren umfasst:
Inberührungbringen der Pflanze oder des Teils der Pflanze mit einer ersten doppelsträngigen RNA, kleinen RNAs oder kleinen RNA-Duplexen, die auf ein erstes pilzpathogenes Dicer-ähnliches (DCL) Gen oder einen ersten pilzpathogenen Dicer-ähnlichen (DCL) Promotor abzielen, und Inberührungbringen der Pflanzen oder des Teils der Pflanze mit einer zweiten doppelsträngigen RNA, kleinen RNAs oder kleinen RNA-Duplexen, die auf ein zweites pilzpathogenes DCL-Gen oder einen pilzpathogenen DLC-Promotor abzielen, wobei
(a) die erste doppelsträngige RNA, kleinen RNAs oder kleinen RNA-Duplexe auf *Botrytis* DCL1 abzielen und die zweite doppelsträngige RNA, kleinen RNAs oder kleinen RNA-Duplexe auf *Botrytis* DCL2 abzielen; wobei das erste und zweite DCL-Gen oder der erste und der zweite DCL-Promotor eine Sequenz von SEQ ID NO: 1, SEQ ID NO: 3, SEQ ID NO: 9, SEQ ID NO: 10, SEQ ID NO: 28 oder SEQ ID NO: 29 oder ein Fragment davon umfassen, das wenigstens 50 zusammenhängende Nukleotide umfasst, oder
(b) die erste doppelsträngige RNA, kleinen RNAs oder kleinen RNA-Duplexe auf *Verticillium* DCL1 abzielen und die zweite doppelsträngige RNA, kleinen RNAs oder kleinen RNA-Duplexe auf *Verticillium* DCL2 abzielen; wobei das erste und das zweite DCL-Gen oder der erste und der zweite DCL-Promotor eine Sequenz von SEQ ID NO: 5, SEQ ID NO: 7, SEQ ID NO: 11, SEQ ID NO: 12, SEQ ID NO: 30 oder SEQ ID NO: 31 oder ein Fragment davon umfassen, das wenigstens 50 zusammenhängende Nukleotide umfasst,
wobei die Pflanze eine Art der Gattungen *Asparagus, Atropa, Avena, Brassica, Citrus, Citrullus, Capsicum, Cucumis, Cucurbita, Daucus, Fragaria, Glycine, Gossypium, Helianthus, Heterocallis, Hordeum, Hyoscyamus, Lactuca, Linum, Lolium, Lycopersicon, Malus, Manihot, Majorana, Medicago, Nicotiana, Oryza, Panieum, Pannesetum, Persea, Pisum, Pyrus, Prunus, Raphanus, Rosa, Secale, Senecio, Sinapis, Solanum, Sorghum, Trigonella, Triticum, Vitis, Vigna* oder *Zea* ist, und wobei die Pflanze oder der Teil der Pflanze im Vergleich zu einer Kontrollpflanze oder einem Kontrollpflanzenteil, die/der nicht mit der doppelsträngigen RNA, kleinen RNAs oder kleinen RNA-Duplexen in Berührung gebracht wurde, eine erhöhte Resistenz gegenüber dem Pilzpathogen aufweist.

2. Verfahren nach Anspruch 1, wobei
die doppelsträngigen RNAs, kleinen RNAs oder kleinen RNA-Duplexe auf die Pflanze oder den Teil der Pflanze gesprüht werden.

3. Verfahren nach Anspruch 1 oder 2, wobei die Pflanze eine Zierpflanze ist.

4. Verfahren nach Anspruch 1 oder 2, wobei
(i) die Pflanze eine frucht- oder gemüseproduzierende Pflanze ist; und/oder
(ii) der Teil der Pflanze eine Frucht, ein Gemüse oder eine Blüte ist.

5. Verfahren zum Erhöhen der Pathogenresistenz in einer Frucht, einem Gemüse oder einer Blüte, wobei das Verfahren umfasst:
Inberührungbringen der Frucht, des Gemüses oder der Blüte mit einer ersten doppelsträngigen RNA, kleinen RNAs oder kleinen RNA-Duplexen, die auf ein erstes pilzpathogenes Dicer-ähnliches (DCL) Gen oder einen ersten pilzpathogenen Dicer-ähnlichen (DCL) Promotor abzielen, und Inberührungbringen der Frucht, des Gemüses oder der Blüte mit einer zweiten doppelsträngigen RNA, kleinen RNAs oder kleinen RNA-Duplexen, die auf ein zweites pilzpathogenes DCL-Gen oder einen pilzpathogenen DLC-Promotor abzielen, wobei
(a) die erste doppelsträngige RNA, kleinen RNAs oder kleinen RNA-Duplexe auf *Botrytis* DCL1 abzielen und die zweite doppelsträngige RNA, kleinen RNAs oder kleinen RNA-Duplexe auf *Botrytis* DCL2 abzielen; wobei das erste und das zweite DCL-Gen oder der erste und der zweite DCL-Promotor eine Sequenz von SEQ ID NO: 1, SEQ ID NO: 3, SEQ ID NO: 9, SEQ ID NO: 10, SEQ ID NO: 28 oder SEQ ID NO: 29 oder ein Fragment davon umfassen, das wenigstens 50 zusammenhängende Nukleotide umfasst, oder
(b) die erste doppelsträngige RNA, kleinen RNAs oder kleinen RNA-Duplexe auf *Verticillium* DCL1 abzielen und die zweite doppelsträngige RNA, kleinen RNAs oder kleinen RNA-Duplexe auf *Verticillium* DCL2 abzielen; wobei das erste und das zweite DCL-Gen oder der erste und der zweite DCL-Promotor eine Sequenz von SEQ ID NO: 5, SEQ ID NO: 7, SEQ ID NO: 11, SEQ ID NO: 12, SEQ ID NO: 30 oder SEQ ID NO: 31 oder ein Fragment davon umfassen, das wenigstens 50 zusammenhängende Nukleotide umfasst,
wobei die Frucht, das Gemüse oder die Blüte im Vergleich zu einer Kontrollfrucht, einem Kontrollgemüse oder einer Kontrollblüte, die/das nicht mit der doppelsträngigen RNA, den kleinen RNAs oder den kleinen RNA-Duplexen in Berührung gebracht wurde, eine erhöhte Resistenz gegenüber dem Pilzpathogen aufweist.

6. Verfahren nach Anspruch 4(ii) oder 5, wobei die Frucht, das Gemüse oder die Blüte eine Nacherntefrucht, ein Nacherntegemüse oder eine Nachernteblüte ist.

7. Verfahren nach Anspruch 5 oder 6, wobei die doppelsträngigen RNAs, kleinen RNAs oder kleinen RNA-Duplexe auf die Frucht, das Gemüse oder die Blüte gesprüht werden.

8. Verfahren nach einem der Ansprüche 1 bis 7, wobei
die doppelsträngige RNA, kleinen RNAs oder kleinen RNA-Duplexe auf eine beliebige der SEQ ID NO: 9, SEQ ID NO: 10, SEQ ID NO: 11 oder SEQ ID NO: 12 abzielen.

9. Verfahren nach einem der Ansprüche 1 bis 7, wobei die doppelsträngige RNA, die kleinen RNAs oder die kleinen RNA-Duplexe eine invertierte Wiederholung einer Sequenz aufweisen, die wenigstens zu 70 % mit einer der SEQ ID NO: 1, SEQ ID NO: 3, SEQ ID NO: 5, SEQ ID NO: 7, SEQ ID NO: 9, SEQ ID NO: 10, SEQ ID NO: 11 oder SEQ ID NO: 12 oder einem Fragment davon, das wenigstens 15 zusammenhängende Nukleotide umfasst, oder einem Komplement davon identisch ist, optional wobei die doppelsträngige RNA siRNA ist.

10. Verfahren nach einem der Ansprüche 1 bis 9, umfassend die Verwendung von zwei oder mehr doppelsträngigen RNAs oder kleinen RNA-Duplexen.

11. Verfahren nach Anspruch 1, umfassend das Erhöhen der Pathogenresistenz gegenüber einer ersten und einer zweiten Art von Pilzpathogenen, wobei das Verfahren umfasst:
Inberührungbringen der Pflanze oder des Teils der Pflanze mit:
(i) einer ersten doppelsträngigen RNA, kleinen RNAs oder kleinen RNA-Duplexen, die auf ein/einen *Botrytis-*DCL1*-*Gen oder -Promoter abzielen, und einer zweiten doppelsträngigen RNA, kleinen RNAs oder kleinen RNA-Duplexen, die auf ein/einen *Botrytis-DCL2-Gen* oder -Promoter abzielen; wobei das DCL1- und das DCL2-Gen oder der DCL1- und DCL2-Promotor eine Sequenz von SEQ ID NO: 1, SEQ ID NO: 3, SEQ ID NO: 9, SEQ ID NO: 10, SEQ ID NO: 28 oder SEQ ID NO: 29 oder ein Fragment davon umfassen, das wenigstens 50 zusammenhängende Nukleotide umfasst, und
(ii) einer ersten doppelsträngigen RNA, kleinen RNAs oder kleinen RNA-Duplexen, die auf ein/einen *Verticillium-*DCL1*-*Gen oder -Promoter abzielen, und einer zweiten doppelsträngigen RNA, kleinen RNAs oder kleinen RNA-Duplexen, die auf ein/einen *Verticillium-*DCL2*-*Gen oder -Promoter abzielen; wobei das DCL1- und DCL2-Gen oder der DCL1- und DCL2-Promotor eine Sequenz von SEQ ID NO: 5, SEQ ID NO: 7, SEQ ID NO: 11, SEQ ID NO: 12, SEQ ID NO: 30 oder SEQ ID NO: 31 oder ein Fragment davon umfassen, das wenigstens 50 zusammenhängende Nukleotide umfasst,
wobei die Pflanze oder der Teil der Pflanze eine erhöhte Resistenz gegenüber der ersten Art des Pathogens und der zweiten Art des Pathogens im Vergleich zu einer Kontrollpflanze oder einem Kontrollpflanzenteil aufweist, die/der nicht mit der ersten und der zweiten doppelsträngigen RNA, kleinen RNAs oder kleinen RNA-Duplexen von (i) und (ii) in Berührung gebracht wurde.

12. Verfahren nach Anspruch 11, wobei:
(i) die erste und die zweite doppelsträngige RNA, kleinen RNAs oder kleinen RNA-Duplexe von (i) und (ii) auf die Pflanze oder den Teil der Pflanze gesprüht werden; und/oder
(ii) der Teil der Pflanze eine Frucht, ein Gemüse oder eine Blüte ist.

13. Verfahren zum Herstellen einer Pflanze mit einer erhöhten Resistenz gegenüber einer ersten Art und einer zweiten Art von Pathogenen, wobei die erste Art von Pathogen eine Art von *Botrytis* ist und die zweite Art von Pathogen eine Art von *Verticillium* ist, wobei das Verfahren umfasst:
Einführen in die Pflanze durch Transformation
(1) einer heterologen Expressionskassette, die einen Promoter umfasst, der mit einem ersten Polynukleotid wirksam verknüpft ist, das die Expression eines *Botrytis-*DCL1-Gens oder -Promoters hemmt, und einer heterologen Expressionskassette, die einen Promoter umfasst, der mit einem zweiten Polynukleotid wirksam verknüpft ist, das die Expression eines *Botrytis-*DCL2*-*Gens oder eines Botrytis-DCL2-Promoters hemmt; wobei das DCL1- und DCL2-Gene oder der DCL1- und DCL2-Promoter die Sequenz von SEQ ID NO: 1, SEQ ID NO: 3, SEQ ID NO: 9, SEQ ID NO: 10, SEQ ID NO: 28 oder SEQ ID NO: 29 oder ein Fragment davon umfassen, das wenigstens 50 zusammenhängende Nukleotide umfasst;
(2) einer heterologen Expressionskassette, die einen Promoter umfasst, der mit einem ersten Polynukleotid wirksam verknüpft ist, das die Expression eines *Verticillium-*DCL1-Gens oder -Promoters hemmt, und einer heterologen Expressionskassette, die einen Promoter umfasst, der mit einem zweiten Polynukleotid wirksam verknüpft ist, das die Expression eines *Verticillium* -DCL2-Gens oder -Promoters hemmt; wobei das DCL1- und DCL2-Gene oder der DCL1- und DCL2-Promoter die Sequenz von SEQ ID NO: 5, SEQ ID NO: 7, SEQ ID NO: 11, SEQ ID NO: 12, SEQ ID NO: 30 oder SEQ ID NO: 31 oder ein Fragment davon umfassen, das wenigstens 50 zusammenhängende Nukleotide umfasst; und
Auswählen einer Pflanze, die die Expressionskassetten umfasst.

14. Verfahren nach Anspruch 13, wobei das erste und das zweite Polynukleotid von (1) und (2) für Antisense-Nukleinsäuren oder hemmende RNA (RNAi) kodieren, die auf das DCL-Gen oder den DCL-Promoter oder ein Fragment davon abzielen.

15. Verfahren nach einem der Ansprüche 11 bis 14, wobei:
(i) die Pflanze eine Art der Gattungen *Asparagus, Atropa, Avena, Brassica, Citrus, Citrullus, Capsicum, Cucumis, Cucurbita, Daucus, Fragaria, Glycine, Gossypium, Helianthus, Heterocallis, Hordeum, Hyoscyamus, Lactuca, Linum, Lolium, Lycopersicon, Malus, Manihot, Majorana, Medicago, Nicotiana, Oryza, Panieum, Pannesetum, Persea, Pisum, Pyrus, Prunus, Raphanus, Rosa, Secale, Senecio, Sinapis, Solanum, Sorghum, Trigonella, Triticum, Vitis, Vigna* oder *Zea* ist; und/oder
(ii) die Pflanze eine Zierpflanze oder eine gemüse- oder fruchtproduzierende Pflanze ist.

## Revendications

1. Procédé d'augmentation de la résistance aux pathogènes dans une plante ou une partie de plante, le procédé comprenant :
la mise en contact de la plante ou de la partie de la plante avec un premier ARN double brin, des petits ARN ou des duplex de petits ARN qui ciblent un premier gène ou promoteur de type Dicer (DCL) de pathogène fongique, et la mise en contact de la plante ou de la partie de la plante avec un deuxième ARN double brin, des petits ARN, ou des duplex de petits ARN qui ciblent un second gène ou promoteur DCL de pathogène fongique, dans lequel
(a) le premier ARN double brin, les petits ARN ou les duplex de petits ARN ciblent *Botrytis* DCL1 et le second ARN double brin, les petits ARN ou les duplex de petits ARN ciblent *Botrytis* DCL2 ; dans lequel le premier et le second gène ou promoteur DCL comprennent une séquence de SEQ ID NO : 1, SEQ ID NO : 3, SEQ ID NO : 9, SEQ ID NO : 10, SEQ ID NO : 28, ou SEQ ID NO : 29, ou un fragment de celle-ci comprenant au moins 50 nucléotides contigus, ou
(b) le premier ARN double brin, les petits ARN ou les duplex de petits ARN ciblent *Verticillium* DCL1 et le second ARN double brin, les petits ARN ou les duplex de petits ARN ciblent *Verticillium* DCL2 ; dans lequel le premier et le second gène ou promoteur DCL comprennent une séquence de SEQ ID NO : 5, SEQ ID NO : 7, SEQ ID NO : 11, SEQ ID NO : 12, SEQ ID NO : 30, ou SEQ ID NO : 31 ou un fragment de celle-ci comprenant au moins 50 nucléotides contigus, dans lequel la plante est une espèce des genres *Asparagus, Atropa, Avena, Brassica, Citrus, Citrullus, Capsicum, Cucumis, Cucurbita, Daucus, Fragaria, Glycine, Gossypium, Helianthus, Heterocallis, Hordeum, Hyoscyamus, Lactuca, Linum, Lolium, Lycopersicon, Malus, Manihot, Majorana, Medicago, Nicotiana, Oryza, Panieum, Pannesetum, Persea, Pisum, Pyrus, Prunus, Raphanus, Rosa, Secale, Senecio, Sinapis, Solanum, Sorghum, Trigonella, Triticum, Vitis, Vigna* ou *Zea,* et dans lequel la plante ou la partie de la plante présente une résistance accrue au pathogène fongique par rapport à une plante témoin ou une partie de plante témoin qui n'a pas été mise en contact avec l'ARN double brin, les petits ARN ou les duplex de petits ARN.

2. Procédé selon la revendication 1, dans lequel
les ARN double brin, les petits ARN ou les duplex de petits ARN sont pulvérisés sur la plante ou la partie de la plante.

3. Procédé selon la revendication 1 ou 2, dans lequel la plante est une plante ornementale.

4. Procédé selon la revendication 1 ou 2, dans lequel
(i) la plante est une plante productrice de fruits ou de légumes ; et/ou
(ii) la partie de la plante est un fruit, un légume ou une fleur.

5. Procédé d'augmentation de la résistance aux pathogènes dans un fruit, un légume, ou une fleur, le procédé comprenant :
la mise en contact du fruit, du légume ou de la fleur avec un premier ARN double brin, des petits ARN ou des duplex de petits ARN qui ciblent un premier gène ou promoteur de type Dicer (DCL) de pathogène fongique, et la mise en contact du fruit, du légume ou de la fleur avec un second ARN double brin, des petits ARN, ou des duplex de petits ARN qui ciblent un second gène ou promoteur DCL de pathogène fongique, dans lequel
(a) le premier ARN double brin, les petits ARN ou les duplex de petits ARN ciblent *Botrytis* DCL1 et le second ARN double brin, les petits ARN ou les duplex de petits ARN ciblent *Botrytis* DCL2 ; dans lequel le premier et le second gène ou promoteur DCL comprennent la séquence de SEQ ID NO : 1, SEQ ID NO : 3, SEQ ID NO : 9, SEQ ID NO : 10, SEQ ID NO : 28, ou SEQ ID NO : 29, ou un fragment de celle-ci comprenant au moins 50 nucléotides contigus ; ou
(b) le premier ARN double brin, les petits ARN ou les duplex de petits ARN ciblent *Verticillium* DCL1 et le second ARN double brin, les petits ARN ou les duplex de petits ARN ciblent *Verticillium* DCL2 ; dans lequel le premier et le second gène ou promoteur DCL comprennent la séquence de SEQ ID NO : 5, SEQ ID NO : 7, SEQ ID NO : 11, SEQ ID NO : 12, SEQ ID NO : 30, ou SEQ ID NO : 31 ou un fragment de celle-ci comprenant au moins 50 nucléotides contigus, et
dans lequel le fruit, le légume ou la fleur présente une résistance accrue au pathogène fongique par rapport à un fruit, un légume ou une fleur témoin qui n'a pas été mis en contact avec l'ARN double brin, les petits ARN ou les duplex de petits ARN.

6. Procédé selon la revendication 4(ii) ou 5, dans lequel le fruit, le légume ou la fleur est un fruit, un légume ou une fleur après récolte.

7. Procédé selon la revendication 5 ou 6, dans lequel les ARN double brin, les petits ARN ou les duplex de petits ARN sont pulvérisés sur le fruit, le légume ou la fleur.

8. Procédé selon l'une quelconque des revendications 1 à 7, dans lequel l'ARN double brin, les petits ARN, ou les duplex de petits ARN ciblent l'une quelconque des SEQ ID NO : 9, SEQ ID NO : 10, SEQ ID NO : 11, ou SEQ ID NO : 12.

9. Procédé selon l'une quelconque des revendications 1 à 7, dans lequel l'ARN double brin, les petits ARN ou les duplex de petits ARN comprennent une répétition inversée d'une séquence qui est identique à au moins 70 % à l'une quelconque des SEQ ID NO : 1, SEQ ID NO : 3, SEQ ID NO : 5, SEQ ID NO : 7, SEQ ID NO : 9, SEQ ID NO : 10, SEQ ID NO : 11, ou SEQ ID NO : 12, ou un fragment de celle-ci qui comprend au moins 15 nucléotides contigus, ou un complément de celle-ci, éventuellement dans lequel l'ARN double brin est un siARN.

10. Procédé selon l'une quelconque des revendications 1 à 9, comprenant l'utilisation de deux ou plusieurs ARN double brin ou duplex de petits ARN.

11. Procédé selon la revendication 1, comprenant l'augmentation de la résistance aux pathogènes d'une première et d'une seconde espèce d'agents pathogènes fongiques, dans lequel le procédé comprend :
la mise en contact de la plante ou de la partie de la plante avec
(i) un premier ARN double brin, des petits ARN ou des duplex de petits ARN qui ciblent un gène ou un promoteur *Botrytis* DCL1 et un second ARN double brin, des petits ARN ou des duplex de petits ARN qui ciblent un gène ou un promoteur *Botrytis* DCL2 ; dans lequel le gène ou promoteur DCL1 et DCL2 comprennent la séquence de SEQ ID NO : 1, SEQ ID NO : 3, SEQ ID NO : 9, SEQ ID NO : 10, SEQ ID NO : 28, ou SEQ ID NO : 29, ou un fragment de celle-ci comprenant au moins 50 nucléotides contigus ; et
(ii) un premier ARN double brin, des petits ARN, ou des duplex de petits ARN qui ciblent le gène ou le promoteur *Verticillium* DCL1 et un second ARN double brin, des petits ARN, ou des duplex de petits ARN qui ciblent un gène ou un promoteur *Verticillium* DCL2 ; dans lequel le gène ou promoteur DCL1 et DCL2 comprennent la séquence de SEQ ID NO : 5, SEQ ID NO : 7, SEQ ID NO : 11, SEQ ID NO : 12, SEQ ID NO : 30, ou SEQ ID NO : 31 ou un fragment de celle-ci comprenant au moins 50 nucléotides contigus ;
dans lequel la plante ou la partie de la plante a une résistance accrue à la première espèce de pathogène et à la seconde espèce de pathogène par rapport à une plante témoin ou une partie de plante témoin qui n'a pas été mise en contact avec les premier et second ARN double brin, petits ARN, ou duplex de petits ARN de (i) et (ii).

12. Procédé selon la revendication 11, dans lequel :
(i) les premier et second ARN double brin, petits ARN, ou duplex de petits ARN de (i) et (ii) sont pulvérisés sur la plante ou la partie de la plante ; et/ou
(ii) la partie de la plante est un fruit, un légume, ou une fleur.

13. Procédé de fabrication d'une plante ayant une résistance accrue aux pathogènes d'une première espèce et d'une seconde espèce de pathogènes, dans lequel la première espèce de pathogène est une espèce de *Botrytis* et la seconde espèce de pathogène est une espèce de *Verticillium,* le procédé comprenant :
l'introduction dans la plante par transformation
(1) d'une cassette d'expression hétérologue comprenant un promoteur lié de manière opérationnelle à un premier polynucléotide qui inhibe l'expression d'un gène ou promoteur *Botrytis* DCL1 et d'une cassette d'expression hétérologue comprenant un promoteur lié de manière opérationnelle à un second polynucléotide qui inhibe l'expression du gène ou promoteur *Botrytis* DCL2 ; dans lequel le gène ou promoteur DCL1 et DCL2 comprennent la séquence de SEQ ID NO : 1, SEQ ID NO : 3, SEQ ID NO : 9, SEQ ID NO : 10, SEQ ID NO : 28, ou SEQ ID NO : 29, ou un fragment de celle-ci comprenant au moins 50 nucléotides contigus ;
(2) d'une cassette d'expression hétérologue comprenant un promoteur lié de manière opérationnelle à un premier polynucléotide qui inhibe l'expression d'un gène ou promoteur *Verticillium* DCL1 et d'une cassette d'expression hétérologue comprenant un promoteur lié de manière opérationnelle à un second polynucléotide qui inhibe l'expression du gène ou promoteur *Verticillium* DCL2 ; dans lequel le gène ou promoteur DCL1 et DCL2 comprennent la séquence de SEQ ID NO : 5, SEQ ID NO : 7, SEQ ID NO : 11, SEQ ID NO : 12, SEQ ID NO : 30, ou SEQ ID NO : 31 ou un fragment de celle-ci comprenant au moins 50 nucléotides contigus ; et
la sélection d'une plante comprenant les cassettes d'expression.

14. Procédé selon la revendication 13, dans lequel les premier et second polynucléotides de (1) et (2) codent pour des acides nucléiques antisens ou un ARN inhibiteur (ARNi) qui ciblent le gène ou promoteur DCL ou un fragment de celui-ci.

15. Procédé selon l'une quelconque des revendications 11 à 14, dans lequel :
(i) la plante est une espèce des genres Asparagus, Atropa, Avena, Brassica, Citrus, Citrullus, Capsicum, Cucumis, Cucurbita, Daucus, Fragaria, Glycine, Gossypium, Helianthus, Heterocallis, Hordeum, Hyoscyamus, Lactuca, Linum, Lolium, Lycopersicon, Malus, Manihot, Majorana, Medicago, Nicotiana, Oryza, Panieum, Pannesetum, Persea, Pisum, Pyrus, Prunus, Raphanus, Rosa, Secale, Senecio, Sinapis, Solanum, Sorghum, Trigonella, Triticum, Vitis, Vigna, ou Zea, et/ou
(ii) la plante est une plante ornementale ou une plante productrice de légumes ou de fruits.
